# EUROPÄISCHE PATENTANMELDUNG

(11) **EP 1 123 925 A1**
(43) Veröffentlichungstag der Anmeldung: **16.08.2001**
(21) Anmeldenummer: 01109310.1
(22) Anmeldetag: 09.01.1989
(51) Int. Cl.: C07D 213/55, A61K 31/44, C07D 213/80, C07D 213/85, C07D 401/06, C07D 405/04, C07D 405/06, C07F 7/18, C07F 7/08, C07D 213/82, C07D 213/89, C07D 401/12, C07D 405/12

(54) **Verfahren zur Herstellung von substituierten Pyridinen**

(30) Priorität: 20.01.1988 DE 3801406; 11.07.1988 IT 2131788
(62) Teilanmeldung aus: 89100250.3
(71) Anmelder: BAYER AG, 51368 Leverkusen (DE)
(72) Erfinder: Angerbauer, Rolf, Dr., 42113 Wuppertal (DE); Fey, Peter, Dr., 42111 Wuppertal (DE); Hübsch, Walter, Dr., 42113 Wuppertal (DE); Philipps, Thomas, Dr., deceased (DE); Bischoff, Hilmar, Dr., 42113 Wuppertal (DE); Petzinna, Dieter, Dr. Dr., 40627 Düsseldorf (DE); Schmidt, Delf, Dr., 42113 Wuppertal (DE); Thomas, Günter, Dr., 42115 Wuppertal (DE)

(57) **Zusammenfassung**

Neue substituierte Pyridine können durch Reduktion von Pyridinen, die durch einen Ketonrest substituiert sind, und nachfolgender Verseifung, Cyclisietrung oder Hydrierung hergestellt werden. Die neuen Verbindungen können als Wirkstoffe in Arzneimittel verwendet werden.

## Beschreibung

Die Erfindung betrifft substituierte Pyridine, Zwischenverbindungen zu ihrer Herstellung, ihre Herstellung und ihre Verwendung in Arzneimitteln.

Es ist bekannt, daß aus Pilzkulturen isolierte Lacton-derivate Inhibitoren der 3-Hydroxy-3-methyl-glutaryl Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden nun substituierte Pyridine der allgemeinen Formel (I), 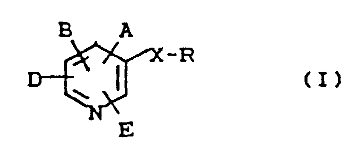 in welcher
A
   - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein kann,
      worin

   - R¹, R²: - gleich oder verschieden sind und Alkyl, Aryl, Aralkyl, Acyl, Alkylsulfonyl oder Arylsulfonyl bedeuten,
   oder
   - für Aryl steht, das bis zu 5-fach gleich oder verschieden substituiert sein kann durch Alkyl, das gegebenenfalls durch Hydroxy oder Alkoxy substituiert sein kann, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
      worin

   - R¹ und R²: die oben angegebene Bedeutung haben,
- für geradkettiges oder verzweigtes Alkyl steht,
B
   - für Cycloalkyl steht, oder
   - für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
      worin

   - R¹, R²: - gleich oder verschieden sind und Alkyl, Aryl, Aralkyl, Acyl, Alkylsulfonyl oder Arylsulfonyl bedeuten,
   oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
D,E gleich oder verschieden sind und
   - für Wasserstoff stehen, oder
   - für CN oder NO₂ stehen, oder
   - für Cycloalkyl stehen, oder
   - für geradkettiges oder verzweigtes Alkyl stehen, das substituiert sein kann durch Azido, Halogen, Hydroxy, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
   worin
   - R¹, R²: die oben angegebene Bedeutung haben,
   oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
   oder
- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein können,
   worin
   - R¹ und R²: die oben angegebene Bedeutung haben,
   oder
- für Aryl stehen, das bis zu 5-fach gleich oder verschieden substituiert sein kann durch Alkyl, das gegebenenfalls durch Hydroxy oder Alkoxy substituiert sein kann, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
   worin
   - R¹ und R²: die oben angegebene Bedeutung haben, oder
   - für eine Gruppe der Formel -NR³R⁴, -COR⁵ oder -CR¹¹R¹²-Y stehen,
      worin
   - R³ und R⁴: gleich oder verschieden sind und
   - Wasserstoff oder
   - Alkyl, Aryl, Aralkyl, oder
   - eine Gruppe der Formel -COR⁶ oder -SO₂R⁷ bedeuten, oder
   - R³ und R⁴: gemeinsam eine Alkylidenkette bilden, die durch N, O, S und/oder N-Alkyl, N-Aryl, N-Carbamoyl oder N-Alkoxycarbonyl unterbrochen sein kann,
   R⁶
   - für Wasserstoff steht, oder
   - für eine Gruppe -NHR⁸ steht, oder
   - für Alkoxy steht, oder
   - für Alkyl, Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

   R⁷
   - für Cycloalkyl steht, oder
   - für Alkyl steht, das durch Cyano, Halogen, Trifluormethyl, Trifluormethoxy oder Alkoxycarbonyl substituiert sein kann, oder
   - für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
   und
   R⁸
   - für Wasserstoff steht, oder
   - für Cycloalkyl steht, oder
   - für gegebenenfalls durch Cyano, Halogen, Trifluormethyl oder Trifluormethoxy substituiertes Alkyl steht, oder
   - für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
   - R⁵: - Wasserstoff, Cycloalkyl, Hydroxy, Alkoxy, Trimethylsilylalkoxy, Aryloxy, Aralkoxy bedeutet, oder
   - für eine Gruppe der Formel -NR⁹R¹⁰ steht,
   worin
   R⁹ und R¹⁰ gleich oder verschieden sind und Wasserrstoff, Alkyl, Aryl oder Aralkyl bedeuten, oder
   - ein über ein Stickstoffatom gebundenen, gegebenenfalls substituierten heterocyclischen Rest der Reihe Pyrrolidin, Piperidin, Morpholin, Thiomorpholin oder Piperazin bedeutet,
   und
   R¹¹ und R¹² gleich oder verschieden sein können und
   - für Wasserstoff, oder
   - für Alkyl stehen, das gegebenenfalls durch Hydroxy, Halogen, Alkoxy oder Alkoxycarbonyl substituiert sein kann, oder
   - für Cycloalkyl stehen, oder

   R¹¹ und R¹² gemeinsam einen gesättigten oder ungesättigten carbocyclischen oder heterocyclischen Ring mit bis zu 6 Kohlenstoffatomen bilden, und
   - Y: - eine Gruppe der Formel -NR¹³R¹⁴, -COR¹⁵, -S-R¹⁶, -SO-R¹⁶, -SO₂-R¹⁶, -OR¹⁷ oder -N₃ bedeutet,
   worin
   R¹³ und R¹⁴ gleich oder verschieden sind und
   - für Wasserstoff, Alkyl, Aryl oder Aralkyl stehen, wobei die Arylreste durch Halogen, Cyano, Alkyl, Alkoxy oder Trifluormethyl substituiert sein können, oder
   - für eine Gruppe der Formel -COR¹⁵, -SO₂R¹⁶ stehen,
   oder R¹³ und R¹⁴ gemeinsam eine Alkylenkette bilden, die durch N, O, S und/oder N-Alkyl, N-Aryl, N-Aralkyl, N-Carbamoyl oder N-Alkoxycarbonyl unterbrochen sein kann,
   R¹⁵
   - Wasserstoff bedeutet, oder
   - eine Gruppe -NR¹⁸R¹⁹ bedeutet, oder
   - Alkyl oder Alkoxy bedeutet, oder
   - Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,

   R¹⁶
   - Cycloalkyl bedeutet, oder
   - geradkettiges oder verzweigtes Alkyl bedeutet, das durch Cyano, Halogen, Trifluormethyl, Trifluormethoxy oder Alkoxycarbonyl substituiert sein kann, oder
   - Aryl, Aralkyl oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
   - Trimethylsilyl oder Dimethylethylsilyl bedeutet, oder
   - eine Gruppe -NR⁹R¹⁰ bedeutet,
   wobei
   R⁹ und R¹⁰ die oben angegebene Bedeutung haben,
   R¹⁷
   - für Wasserstoff steht, oder
   - für Cycloalkyl steht, oder
   - für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
   worin
   R¹, R² die oben angegebene Bedeutung haben, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
   oder
   - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein kann,
   worin
   R¹ und R² die oben angegebene Bedeutung haben, oder
   - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
   worin
   R¹ und R² die oben angegebene Bedeutung haben, oder
   - für 2,5-Dioxo-tetrahydropyrryl,
   - für Tetrahydropyranyl, oder
   - für Trialkylsilyl steht, oder eine Gruppe COR¹⁶ bedeutet,
   wobei
   R¹⁶ die oben angegebene Bedeutung hat,
   und R¹⁸ und R¹⁹ gleich oder verschieden sind und
   - Wasserstoff bedeuten, oder
   - Cycloalkyl bedeuten, oder
   - gegebenenfalls durch Cyano, Halogen, Trifluormethyl oder Trifluormethoxy substituiertes Alkyl bedeuten, oder
   - Aryl, Aralkyl oder Heteroaryl bedeuten, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
   oder,
D und E gemeinsam für den Rest der Formel 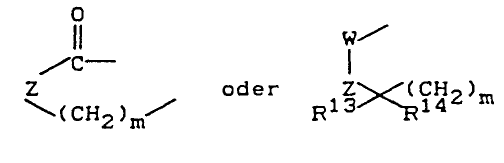 stehen, und einen Ring bilden, wobei
W - für eine Gruppe der Formel 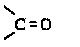 oder -CHOH steht,
m - für eine Zahl 1, 2 oder 3 steht,
Z - für O, S, CH₂ oder für N-R²⁰ steht,
R¹³ und R¹⁴ die oben angegebene Bedeutung haben,
   und
R²⁰ - für Wasserstoff, Alkyl, Aryl, Aralkyl, Carbamoyl oder Alkoxycarbonyl steht,
   und wobei in diesem Fall D und E benachbart sind,
X - für eine Gruppe der Formel -CH₂-CH₂- oder -CH=CH-steht,
   und
R - für eine Gruppe der Formel 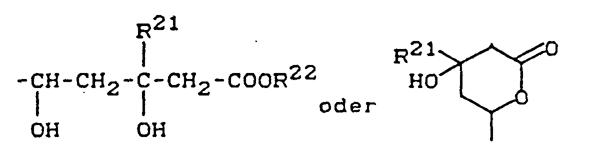 steht, worin
   - R²¹: - Wasserstoff oder Alkyl bedeutet
   und
   - R²²: - Wasserstoff,
   - Alkyl, Aryl oder Aralkyl, oder
   - ein Kation bedeutet,
   und deren Oxidationsprodukte gefunden.

Unter den Oxidationsprodukten der erfindungsgemäßen Verbindungen der allgemeinen Formel (I) versteht man die entsprechenden Verbindungen des Pyridin-N-oxids.

Überraschenderweise zeigen die erfindungsgemäßen substituierten Pyridine eine überlegene inhibitorische Wirkung auf die HMG-CoA Reduktase (3-Hydroxy-3-methyl-glutaryl Coenzym A Reduktase).

Cycloalkyl steht im allgeinen für einen cyclischen Kohlenwasserstoffrest mit 3 bis 8 Kohlenstoffatomen, Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine SO₂-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propyleulfonyl, Isopropylsulfonyl, Butylsulfonyl, Isobutylsulfonyl, Pentylsulfonyl, Isopentylsulfonyl, Hexylsulfonyl, Isohexylsulfonyl.

Sulfamoyl (Aminosulfonyl) steht für die Gruppe -SO₂-NH₂.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine SO₂-Gruppe gebunden ist. Beispielsweise seien genannt: Phenylsulfonyl, Naphthylsulfonyl und Biphenylsulfonyl,

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine SO₂-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethlysulfonyl und Phenylpropylsulfonyl.

Alkoxycarbonyl kann beispielsweise durch die Formel 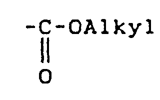 dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl,

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Steht R²² für einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester (C₁ bis C₄) und Aralkylester (C₇ bis C₁₀), bevorzugt Niederalkylester sowie Benzylester. Darüber hinaus seien die folgenden Esterreste genannt: Methylester, Ethylester, Propylester, Benzylester.

Steht R²² für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniederalkylamine, Triniederalkylamine, Prokain, Dibenzylamin, N,N'-Dibenzylethylendiamin, N-Benzyl-β-phenylethylamin, N-Methylmorpholin oder N-Ethylmorpholin, 1-Ephenamin, Dihydroabiethylamin, N,N'-Bis-dihydroabiethylethylendiamin, N-Niederalkylpiperidin und andere Amine, die zur Bildung von Salzen verwendet werden können.

Im Rahmen der vorliegenden Erfindung entsprechen die substituierten Pyridine (Ia) der allgemeinen Formel 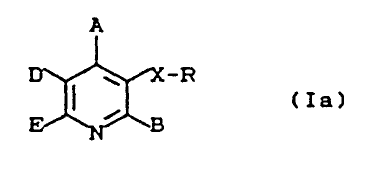 in welcher
A, B, D, E, X und R die oben angegebene Bedeutung haben.

Im Rahmen der vorliegenden Erfindung entsprechen die substituierten Pyridine (Ib) der allgemeinen Formel 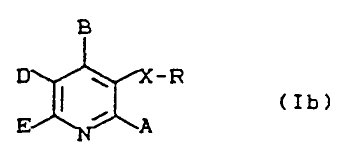 in welcher
A, B, D, E, X und R die oben angegebene Bedeutung haben.

Im Rahmen der allgemeinen Formel (I) sind Verbindungen der allgemeinen Formeln (Ia) und (Ib) bevorzugt.

Bevorzugt sind solche Verbindungen der allgemeinen Formeln (Ia) und (Ib) 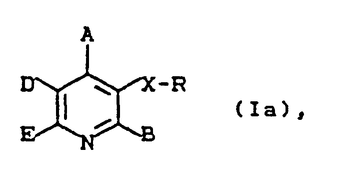 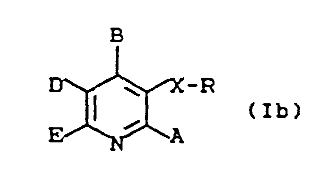 in welchen
A
   - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
   - für Phenyl oder Naphthyl steht, das bis zu 4-fach gleich oder verschieden substituiert sein kann durch Niederalkyl, das gegebenenfalls durch Hydroxy oder Niederalkoxy substituiert sein kann, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom oder Cyano,
      - R¹ und R²: gleich oder verschieden sind und Niederalkyl, Phenyl, Benzyl, Acetyl, Benzoyl, Phenylsulfonyl oder Niederalkylsulfonyl bedeuten,
   - für geradkettiges oder verzweigtes Niederalkyl steht,
B
   - für Cyclopropyl, Cyclopentyl oder Cyclohexyl steht oder
   - für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel -NR¹R²,
      worin
      - R¹ und R²: die oben angegebene Bedeutung haben, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
D,E
   - gleich oder verschieden sind und
   - für Wasserstoff stehen, oder
   - für CN oder NO₂ stehen, oder
   - für Cyclopropyl, Cyclopentyl oder Cyclohexyl stehen, oder
   - für geradkettiges oder verzweigtes Niederalkyl stehen, das substituiert sein kann durch Azido, Fluor, Chlor, Brom, Cyano, Hydroxy, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel -NR¹R²,
      worin
      - R¹ und R²: die oben angegebene Bedeutung haben,
      oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,
   - für Thienyl, Furyl, Thiazolyl, Tetrazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
   - für Phenyl oder Naphthyl stehen, die bis zu 4-fach gleich oder verschieden substituiert sein können durch Niederalkyl, das gegebenenfalls durch Hydroxy oder Niederalkoxy substituiert sein kann, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel -NR¹R²,
      wobei
      - R¹ und R²: die oben angegebene Bedeutung haben,
      oder
   - für eine Gruppe der Formel -NR³R⁴, -COR⁵ oder -CR¹¹R¹²-Y stehen,
      worin
      - R³ und R⁴: gleich oder verschieden sind und
      - Wasserstoff oder
      - Niederalkyl, Phenyl oder Benzyl, oder
      - eine Gruppe der Formel -COR⁶ oder -SO₂R⁷ bedeuten,
      wobei
      R⁶
      - für Wasserstoff steht, oder
      - für eine Gruppe -NHR⁸ steht, oder
      - für Niederalkoxy steht, oder
      - für gegebenenfalls durch Niederalkyl, Niederalkoxy, Fluor, Chlor, Brom, Trifluormethyl, Dimethylamino oder Diethylamino substituiertes Niederalkyl, Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,

      R⁷
      - für Cyclopropyl, Cyclopentyl oder Cyclohexyl steht, oder
      - für gegebenenfalls durch Cyano, Fluor, Chlor, Brom, Trifluormethyl oder Niederalkoxycarbonyl substituiertes Niederalkyl steht, oder
      - für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl steht,
      und
      R⁸
      - für Wasserstoff steht, oder
      - für gegebenenfalls durch Cyano, Fluor, Chlor oder Brom substituiertes Niederalkyl steht, oder
      - für gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl steht,
      - R⁵: - Wasserstoff, Cyclohexyl, Hydroxy, Niederalkoxy, Trimethylsilylniederalkoxy, Benzyloxy bedeutet, oder
      - für eine Gruppe der Formel -NR⁹R¹⁰ steht,
      worin
      R⁹ und R¹⁰ gleich oder verschieden sind und
      - Wasserstoff, Niederalkyl oder Phenyl bedeuten, oder
      - einen heterocyclischen Ring der Reihe Pyrrolidin, Piperidin, Piperazin, N-Alkylpiperazin, N-Arylpiperazin, N-Benzylpiperazin, N-Carbamoylpiperazin oder N-Alkoxycarbonylpiperazin bedeutet,
      und
      R¹¹ und R¹² gleich oder verschieden sein können und
      - für Wasserstoff, oder
      - für Niedrigalkyl stehen, das gegebenenfalls durch Hydroxy, Fluor, Chlor, Niederalkoxy oder Niederalkoxycarbonyl substituiert sein kann,
      oder
      - für Cyclopropyl, Cyclopentyl oder Cyclohexyl stehen, oder

   - R¹¹ und R¹²: gemeinsam einen gesättigten oder ungesättigten carbocyclischen oder heterocyclischen Ring mit bis zu 6 Kohlenstoffatomen bilden,
   - Y: - eine Gruppe der Formel -NR¹³R¹⁴, -COR¹⁵, -S-R¹⁶, SO-R¹⁶, -SO₂R¹⁶, -OR¹⁷ oder -N₃ bedeutet,
   wobei
   - R¹³ und R¹⁴: gleich oder verschieden sind und
   - für Wasserstoff, Niederalkyl, Phenyl oder Benzyl stehen, wobei die genannten Reste durch Fluor, Chlor, Brom, Niederalkyl, Niederalkoxy oder Trifluormethyl substituiert sein können, oder
   - für eine Gruppe der Formel -COR¹⁵, -SO₂R¹⁶ stehen,
   oder R¹³ und R¹⁴ gemeinsam eine Alkylenkette bilden, die durch O, N, S, N-Niederalkyl, N-Benzyl, N-Phenyl, N-Carbamoyl oder N-Niederalkoxycarbonyl unterbrochen sein kann,
   R¹⁵
   - eine Gruppe -NR¹⁸R¹⁹ bedeutet, oder
   - Niederalkyl oder Niederalkoxy bedeutet,
   oder
   - gegebenenfalls durch Niederalkyl, Niederalkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

   R¹⁶
   - Cyclopropyl, Cyclopentyl, Cyclohexyl, oder
   - gegebenenfalls durch Cyano, Fluor, Chlor, Brom, Trifluormethyl oder Niederalkoxycarbonyl substituiertes geradkettiges oder verzweigtes Niederalkyl bedeutet, oder
   - gegebenenfalls ein- oder mehrfach gleich oder verschieden durch Niederalkyl, Niederalkoxy, Fluor, Chlor, Brom, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
   - Trimethylsilyl oder Dimethylethylsilyl bedeutet, oder
   - eine Gruppe -NR⁹R¹⁰ bedeutet,
      wobei
      R⁹ und R¹⁰ die oben angegebene Bedeutung haben,

   R¹⁷
   - für Wasserstoff, oder
   - für Cyclopropyl, Cyclopentyl oder Cyclohexyl steht, oder
   - für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel -NR¹R²,
      worin
      R¹ und R² die oben angegebene Bedeutung haben, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein kann,
   - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
   - für Benzyl, Phenyl oder Naphthyl steht, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel -NR¹R²,
      wobei
      R¹ und R² die oben angegebene Bedeutung haben, oder
   - für 2,5-Dioxo-tetrahydropyrryl oder
   - für Tetrahydropyranyl, oder
   - für Dimethyl-tert.butylsilyl, Tripropylsilyl oder Tributylsilyl steht, oder
   - eine Gruppe der Formel COR¹⁶ bedeutet,
      wobei
      R¹⁶ die oben angegebene Bedeutung hat,
   und
   R¹⁸ und R¹⁹ gleich oder verschieden sind und
   - Wasserstoff bedeuten, oder
   - gegebenenfalls durch Cyano, Fluor, Chlor oder Brom substituiertes Niederalkyl bedeuten, oder
   - gegebenenfalls durch Niederalkyl, Niederalkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeuten,
   oder
D, E gemeinsam einen Ring der Formel 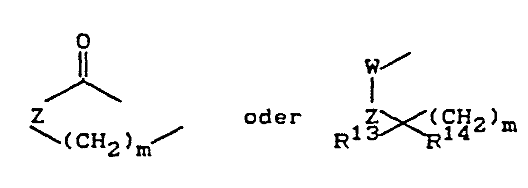 bilden, worin
   W - für eine Gruppe der Formel C=O oder für CH-OH steht,
   m - für eine Zahl 1 oder 2 steht,
   Z - für O, CH₂ oder NHR²⁰ steht,
   R¹³ und R¹⁴ die oben angegebene Bedeutung haben,
      und
   R²⁰ - für Wasserstoff, Niederalkyl, Phenyl, Benzyl, Carbamoyl oder Niederalkoxycarbonyl steht,
      und wobei in diesem Fall D und E benachbart sind,
X - für eine Gruppe der Formel -CH=CH- steht,
R - für eine Gruppe der Formel 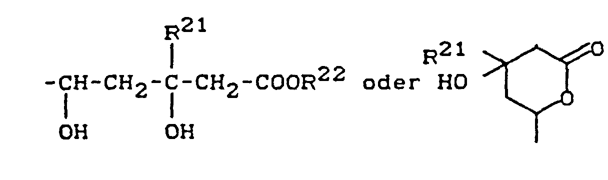 steht, worin
   - R²¹: - Wasserstoff oder Niederalkyl bedeutet,
   und
   - R²²: - Niederalkyl, Phenyl oder Benzyl bedeutet, oder
   - ein Kation bedeutet,
und deren Oxidationsprodukte.

Besonders bevorzugt sind Verbindungen der allgemeinen Formeln (Ia) und (Ib) ,
in welchen
A
   - für Thienyl, Furyl, Pyridyl, Pyrimidyl, Chinolyl oder Isochinolyl steht, das durch Fluor, Chlor, Methyl, Methoxy oder Trifluormethyl substituiert sein kann, oder
   - für Phenyl steht, das bis zu 3-fach gleich oder verschieden substituiert sein kann durch Methyl, Hydroxymethyl, Ethyl, Propyl, Isopropyl, Hydroxyethyl, Hydroxypropyl, Butyl, Isobutyl, Methoxymethyl, Ethoxymethyl, Propoxymethyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
   - für Methyl, Ethyl, Propyl, Isopropyl, Butyl oder tert. Butyl steht,
B
   - für Cyclopropyl, Cyclopentyl oder Cyclohexyl steht, oder
   - für Methyl, Ethyl, Propyl, Isopropyl, Butyl, sec.-Butyl oder tert.Butyl steht, das substituiert sein kann durch Fluor, Chlor, Brom, Cyano, Methoxy, Ethoxy, Propoxy, Isopropoxy, Butoxy, sec.Butoxy, tert.-Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
D,E
   - gleich oder verschieden sind und
   - für CN, NO₂ stehen, oder
   - für Wasserstoff, Cyclopropyl, Cyclopentyl oder Cyclohexyl stehen, oder
   - für Methyl, Ethyl, Propyl, Isopropyl, Butyl, Isobutyl, tert.Butyl, Pentyl, Isopentyl, Hexyl oder Isohexyl stehen, das substituiert sein kann durch Azido, Fluor, Chlor, Brom, Jod, Cyano, Hydroxy, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
      wobei
      - R¹ und R²: gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
      oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
      - für Thienyl, Furyl, Pyridyl, Pyrimidyl, Pyrazinyl, Tetrazolyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
      - für Phenyl stehen, das bis zu 3-fach gleich oder verschieden durch Methyl, Ethyl, Propyl, Isopropyl, Butyl, Isobutyl, tert.Butyl, Pentyl, Isopentyl, Hexyl, Methylhydroxy, Ethylhydroxy, Propylhydroxy, Isohexyl, Methoxy, Ethoxy, Propoxy, Isopropoxy, Butoxy, Isobutoxy, Methoxymethyl, Ethoxymethyl, Propoxymethyl, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR¹R² substituiert sein kann,
         wobei
         - R¹ und R²: die oben angegebene Bedeutung haben,
         oder
      - für eine Gruppe der Formel -NR³R⁴, -COR⁵ oder -CR¹¹R¹²-Y stehen,
         worin
         - R³ und R⁴: gleich oder verschieden sind und
         - Wasserstoff oder
         - Methyl, Ethyl, Propyl, Isopropyl, tert.Butyl, Pentyl, Isopentyl, Hexyl, Isohexyl, Phenyl, Benzyl, oder
         - eine Gruppe der Formel -COR6 oder -SO₂R⁷ bedeuten,
         worin
         R⁶
         - für Wasserstoff steht, oder
         - für eine Gruppe -NHR⁸ steht, oder
         - für Methoxy, Ethoxy, Propoxy oder Isopropoxy steht, oder
         - für Methyl, Ethyl, Propyl, Isopropyl oder Butyl steht, oder
         - für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,

         R⁷
         - für gegebenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Ethyl, Propyl, Isopropyl, Butyl oder Isobutyl steht, oder
         - für gegebenenfalls durch Methyl, Ethyl, Propyl, Isopropyl, Methoxy, Ethoxy, Propoxy oder Isopropoxy, Fluor oder Chlor substituiertes Phenyl, Thienyl, Furyl, Pyridyl, Pyrimidyl, Chinolyl oder Isochinolyl steht,
         und
         R⁸
         - für Wasserstoff steht, oder
         - für gegebenenfalls durch Fluor oder Chlor substituiertes Methyl, Ethyl, Propyl, Isopropyl, Butyl, Isobutyl, Pentyl, Isopentyl, Hexyl oder Isohexyl steht, oder
         - für Phenyl steht, das durch Fluor, Chlor, Methyl oder Methoxy substituiert sein kann,

         R⁵
         - Wasserstoff, Cyclohexyl, Hydroxy, Methoxy, Ethoxy, Propoxy, Butoxy, Isopropoxy, Isobutoxy oder Trimethylsilylethoxy bedeutet, oder
         - für NR⁹R¹⁰ steht, worin

         R⁹ und R¹⁰ gleich oder verschieden sind und Wasserstoff, Methyl, Ethyl, Propyl, Isopropyl, Butyl, Isobutyl oder Phenyl bedeutet, oder
         - einen heterocylischen Ring der Reihe Piperidin, N-Methylpiperazin, N-Ethylpiperazin, N-Benzylpiperazin oder Morpholin bedeutet,
         und
         - R¹¹ und R¹²: gleich oder verschieden sind und
         - für Wasserstoff, oder
         - für Methyl, Ethyl, Propyl, Isopropyl stehen, das gegebenenfalls durch Hydroxy, Fluor, Chlor, Methoxy, Ethoxy, Methoxycarbonyl, Ethoxycarbonyl substituiert sein kann, oder
         - für Cyclopropyl, Cyclopentyl oder Cyclohexyl stehen,
         oder R¹¹ und R¹² gemeinsam für Cyclopropyl, Cyclopentyl oder Cyclohexyl stehen,
         und
         - Y: - eine Gruppe der Formel -NR¹³R¹⁴, -COR¹⁵, -SR¹⁶, -SO-R¹⁶, -SO₂R¹⁶, -OR¹⁷ oder -N₃ bedeutet,
         wobei
         R¹³ und R¹⁴ gleich oder verschieden sind, und
         - für Wasserstoff, Methyl, Ethyl, Propyl, Isopropyl, Butyl, Isobutyl, oder
         - für gegebenenfalls durch Fluor, Chlor, Methyl oder Methoxy subsituiertes Phenyl, oder
         - für eine Gruppe -COR¹⁵ oder -SO₂R¹⁶ stehen, oder

         R¹³ und R¹⁴ gemeinsam mit dem Stickstoffatom einen Ring der Reihe Piperidin, Piperazin, Morpholin, Morpholin-N-oxid, N-Niederalkylpiperazin, Benzylpiperazin oder Phenylpiperazin bilden,
         R¹⁵
         - Wasserstoff bedeutet, oder
         - eine Gruppe -NR¹⁸R¹⁹ bedeutet, oder
         - Methyl, Ethyl, Propyl, Isopropyl, Methoxy, Ethoxy, Propoxy, Isopropoxy, oder
         - gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,

         R¹⁶
         - gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl, Isobutyl oder Isopentyl bedeutet, oder
         - gegebenenfalls ein- oder mehrfach gleich oder verschieden durch Methyl, Ethyl, Propyl, Isopropyl, Methoxy, Fluor oder Chlor substituiertes Benzyl, Phenyl, Naphthyl, Thienyl, Furyl, Pyridyl, Pyrimidyl, Chinolyl oder Isochinolyl bedeutet, oder
         - Trimethylsilyl oder Dimethylethylsilyl bedeutet,
         - eine Gruppe -NR⁹R¹⁰ bedeutet,
            wobei
            R⁹ und R¹⁰ die oben angegebene Bedeutung haben,

         R¹⁷
         - für Wasserstoff, Cyclopropyl, Cyclopentyl oder Cyclohexyl steht, oder
         - für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.-Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.-Butoxycarbonyl, Benzoyl, Acetyl, Ethylcarbonyl, oder durch eine Gruppe -NR¹R²,
            wobei
            R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten, oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein kann, oder
         - für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzooxazolyl, Benzimidazolyl oder Benzothiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
         - für Benzyl oder Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR¹R² substituiert sein kann,
            wobei
            R¹ und R² die oben angegebene Bedeutung haben, oder
         - für 2,5-Dioxo-tetrahydropyrryl oder
         - für Tetrahydropyranyl steht, oder
         - für Dimethyl-tert.butylsilyl oder Trimethylsilyl steht, oder
         - eine Gruppe -COR¹⁶ bedeutet,
            wobei
            R¹⁶ die oben angegebene Bedeutung hat,
         und
         R¹⁸ und R¹⁹ gleich oder verschieden sind und
         - Wasserstoff bedeuten, oder
         - gegebenenfalls durch Fluor oder Chlor substituiertes Methyl, Ethyl, Propyl, Isopropyl, Butyl, Isobutyl, Pentyl, Isopentyl, Hexyl oder Isohexyl bedeuten, oder
         - Phenyl bedeuten, das durch Fluor, Chlor, Methyl oder Methoxy substituiert sein kann,
   oder
D und E gemeinsam einen Ring der Formel 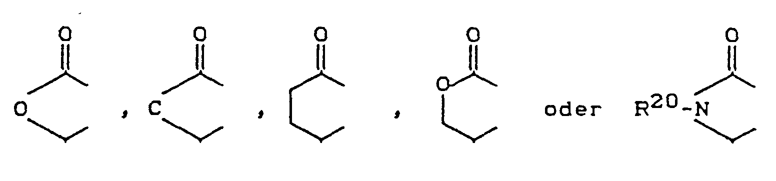 bilden,
   worin
   - R²⁰: - für Wasserstoff, Methyl, Ethyl, Propyl, Isopropyl, Carbamoyl, Methoxycarbonyl oder Ethoxycarbonyl steht,
X - für eine Gruppe der Formel -CH=CH- steht,
R - für eine Gruppe der Formel 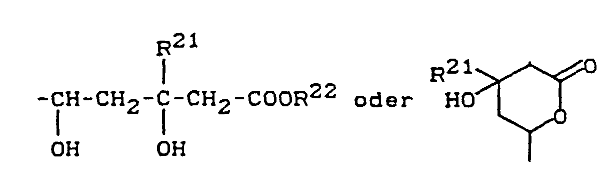 steht, worin
   - R²¹: - Wasserstoff, Methyl, Ethyl, Propyl, Isopropyl, Butyl, Isobutyl oder tert.Butyl bedeutet,
   und
   - R²²: - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet.
und deren Oxidationsprodukte.

Ganz besonders bevorzugt sind Verbindungen der allgemeinen Formeln (Ia) und (Ib)
in welchen
A
   - für Thienyl oder Furyl steht,
   - für Phenyl steht, das bis zu 2-fach gleich oder verschieden durch Methyl, Hydroxymethyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Trifluormethyl substituiert sein kann,
   - für Methyl, Ethyl, Propyl oder Isopropyl steht,
B
   - für Cyclopropyl, Methyl, Ethyl, Propyl, Isopropyl, Butyl, Isobutyl oder tert.Butyl steht, das durch Fluor, Chlor, Methoxy, Phenyl oder Phenoxy substituiert sein kann,
D,E gleich oder verschieden sind und
   - für Wasserstoff, CN, NO₂, Cyclopropyl, Cyclopentyl oder Cyclohexyl stehen, oder
   - für Methyl, Ethyl, Propyl, Isopropyl, Butyl, Isobutyl, tert.Butyl, Pentyl, Isopentyl, Hexyl oder Isohexyl stehen, das substituiert sein kann durch Azido, Fluor, Chlor, Iod, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
      wobei
      - R¹ und R²: gleich oder verschieden sind und
      - für Wasserstoff, Methyl, Ethyl, Propyl, Isopropyl, Phenyl oder Benzyl stehen,
      oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
   - für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Tetrazolyl, Benzthiazolyl oder Benzimidazolyl stehen, oder
   - für Phenyl stehen, das bis zu 2-fach gleich oder verschieden durch Methyl, Ethyl, Propyl, Isopropyl, Butyl, Isobutyl, tert.Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel -NR¹R² substituiert sein kann,
      wobei
      - R¹ und R²: die oben angegebene Bedeutung haben.
      oder
   - für eine Gruppe der Formel -NR³R⁴, -COR⁵ oder -CR¹¹R¹²-Y stehen
      worin
      - R³: - Wasserstoff bedeutet,
      - R⁴: - Wasserstoff, Methyl, Ethyl oder Propyl, oder
      - eine Gruppe der Formel -COR⁶ oder -SO₂R⁷ bedeutet,
      worin
      R⁶
      - für Wasserstoff steht, oder
      - für eine Gruppe -NHR⁸ steht, oder
      - für Methoxy oder Ethoxy steht, oder
      - für Methyl, Ethyl, Propyl oder Isopropyl steht,

      R⁷ - für Trifluormethyl, Phenyl, Tolyl steht,
      R⁸
      - für Wasserstoff steht, oder
      - für Methyl, Ethyl, Propyl, Isopropyl oder Butyl, oder
      - für Phenyl steht,
      - R⁵: - Wasserstoff, Cyclohexyl, Hydroxy, Amino, Methylamino, Dimethylamino, Methoxy, Ethoxy oder Trimethylsilylethoxy bedeutet, oder
      - für die Gruppe NR⁹R¹⁰ steht
         worin

      R⁹ und R¹⁰ gleich oder verschieden sind und
      - Wasserstoff, Methyl, Ethyl, Propyl oder Phenyl bedeuten, oder

      R⁹ und R¹⁰ gemeinsam einen Morpholinring bilden, und
      R¹¹ und R¹² Wasserstoff, Methyl oder Ethyl bedeuten,
      - Y: - eine Gruppe der Formel -NR¹³R¹⁴, -COR¹⁵, -S-R¹⁶, -SO-R¹⁶, -SO₂R¹⁶ oder -OR¹⁷ bedeutet,
      wobei
      R¹³ und R¹⁴ gleich oder verschieden sind, und
      - für Wasserstoff, Methyl, Ethyl, Propyl, oder
      - für eine Gruppe -COR¹⁵ oder -SO₂R¹⁶ stehen
      oder R¹³ und R¹⁴ gemeinsam mit dem Stickstoffatom einen Ring der Reihe Morpholin oder Morpholin-N-oxid bilden,
      und
      R¹⁵
      - Wasserstoff oder Methyl bedeutet, oder
      - eine Gruppe -NR¹⁸R¹⁹ bedeutet, oder
      - Methyl, Ethyl, Propyl, Methoxy oder Ethoxy bedeutet,

      R¹⁶
      - Trifluormethyl, Methyl, Ethyl, Propyl, Isopropyl, Butyl, Isobutyl, Isopentyl oder Benzyl bedeutet, oder
      - gegebenenfalls durch ein- oder mehrere Methyl oder Chlor substituiertes Phenyl, oder Naphthyl bedeutet,
      - Trimethylsilyl oder Dimethylethylsilyl, oder
      - eine Gruppe -NR⁹R¹⁰ bedeutet,
      wobei
      R⁹ und R¹⁰ die oben angegebene Bedeutung haben,
      R¹⁷
      - für Wasserstoff, Cyclopropyl, Cyclopentyl oder Cyclohexyl steht, oder
      - für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
      wobei
      R¹ und R² gleich oder verschieden sind und
      - für Wasserstoff, Methyl, Ethyl, Propyl, Isopropyl, Phenyl oder Benzyl stehen,
      oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
      - für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Benzthiazolyl oder Benzimidazolyl steht, oder
      - für Benzyl oder Phenyl steht, das bis zu 2-fach gleich oder verschieden durch Methyl, Ethyl, Propyl, Isopropyl, Butyl, Isobutyl, tert.Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel -NR¹R² substituiert sein kann,
         wobei

      R¹ und R² die oben angegebene Bedeutung haben,
      und
   - für 2,5-Dioxo-tetrahydropyrryl oder
   - für Tetrahydropyranyl, oder
   - für Dimethyl-tert.butylsilyl oder Trimethylsilyl steht, oder
   - eine Gruppe -COR¹⁶ bedeutet,
      wobei
      - R¹⁶: die oben angegebene Bedeutung hat,
   und
   - R¹⁸ und R¹⁹: gleich oder verschieden sind und
   - Wasserstoff bedeuten, oder
   - Methyl, Ethyl, Propyl, Isopropyl, Butyl oder Isobutyl bedeuten, oder
   - Phenyl bedeuten,
   oder
D und E gemeinsam einen Ring der Formel 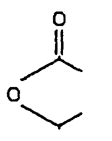 bilden,
X - für eine Gruppe der Formel 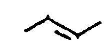 (E-konfiguriert) steht ,
   und
R - für eine Gruppe der Formel 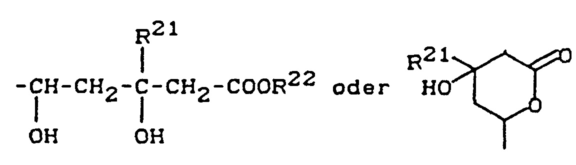 steht,
   worin
   - R²¹: - Wasserstoff bedeutet
   und
   - R²²: - Wasserstoff, Methyl oder Ethyl bedeutet, oder ein Natrium- oder Kaliumion bedeutet
und deren Oxidationsprodukte.

Die erfindungsgemäßen substituierten Pyridine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw, des Restes R ergeben sich unterschiedliche Stereoisomere, die im folgenden näher erläutert werden sollten:
a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können: 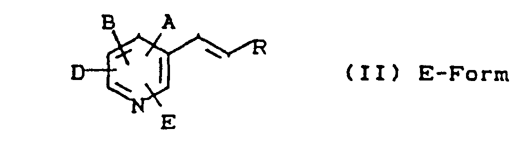 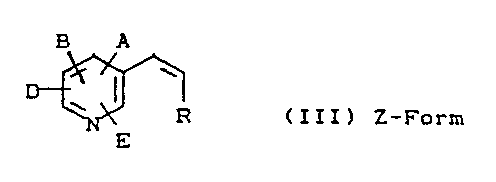 (A, B, D, E und R haben die oben angegebene Bedeutung).
   Bevorzugt sind diejenigen Verbindungen der allgemeinen Formel (I) die E-konfiguriert sind (II).
b) Steht der Rest -R- für eine Gruppe der Formel 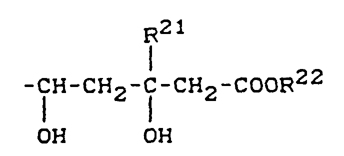 so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen. 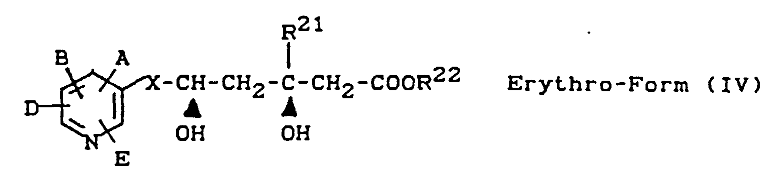 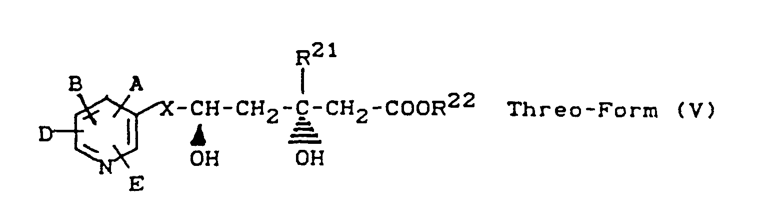
   Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).
   Bevorzugt sind hierbei die Erythro-konfigurierten Isomeren, besonders bevorzugt das 3R,5S-Isomere sowie das 3R,5S-3S,5R-Racemat.
c) Steht der Rest -R- für eine Gruppe der Formel 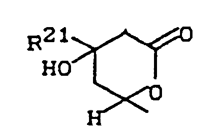 so besitzen die substituierten Pyridine mindestens zwei asymmetrische Kohlenstoffatome, nämlich das Kohlenstoffatom an dem die Hydroxygruppe gebunden ist, und das Kohlenstoffatom an welchem der Rest der Formel 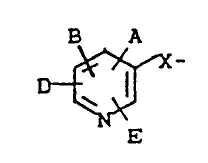 gebunden ist. Je nach der Stellung der Hydroxygruppe zur freien Valenz am Lactonring können die substituierten Pyridine als cis-Lactone (VI) oder als trans-Lactone (VII) vorliegen.

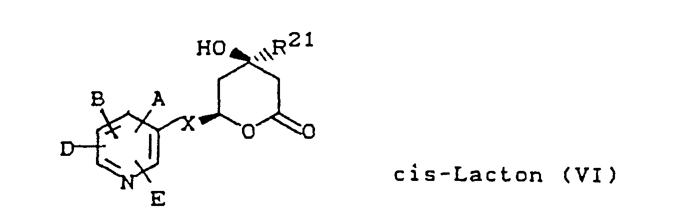

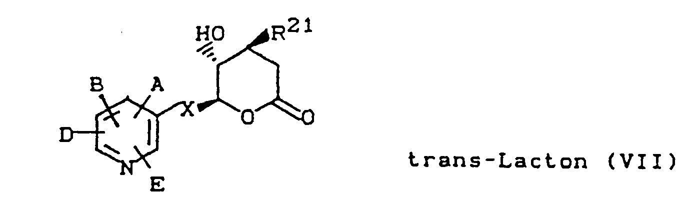

Sowohl vom cis-Lacton aus als auch vom trans-Lacton existieren wiederum jeweils zwei Isomeren nämlich das 4R,6R-Isomere bzw. das 45,6S-Isomere (cis-Lacton), sowie das 4R,6S-Isomere bzw. 4S,6R-Isomere (trans-Lacton). Bevorzugte Isomeren sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyridine genannt:

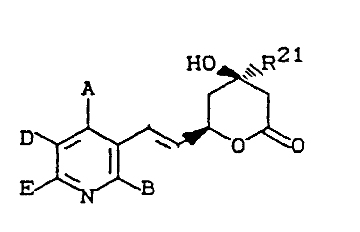

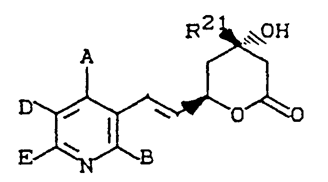

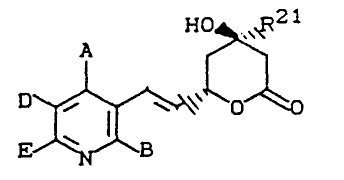

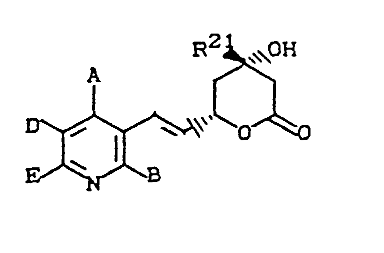

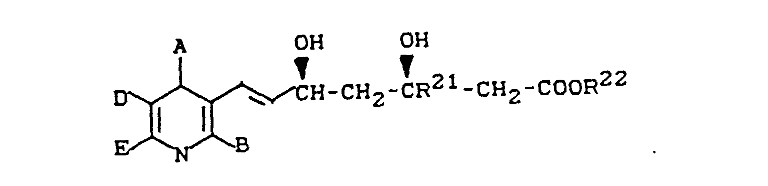

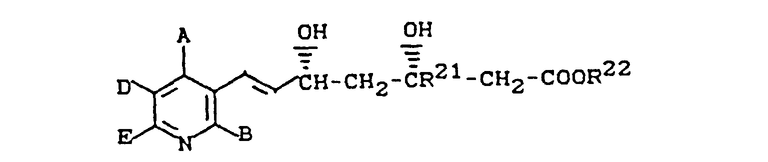

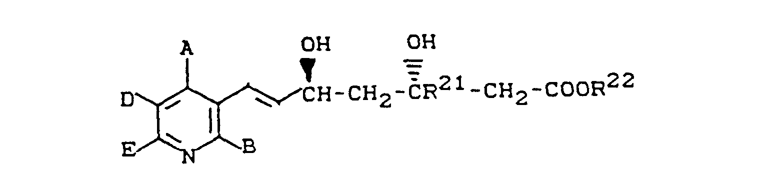

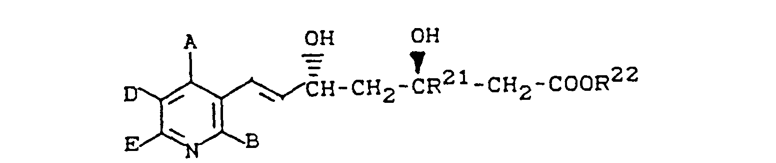

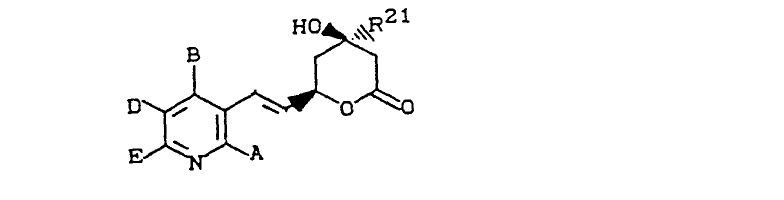

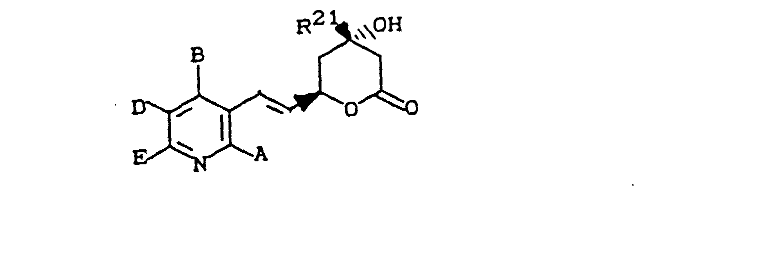

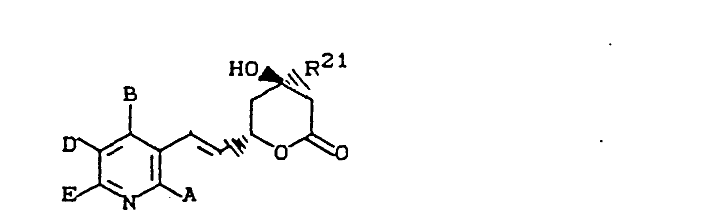

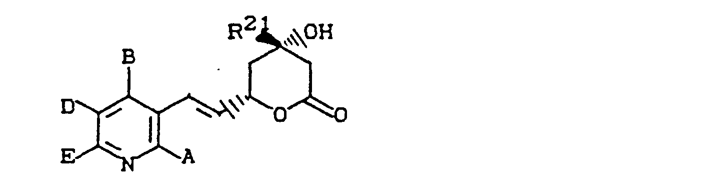

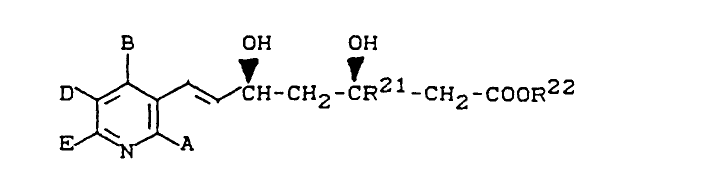

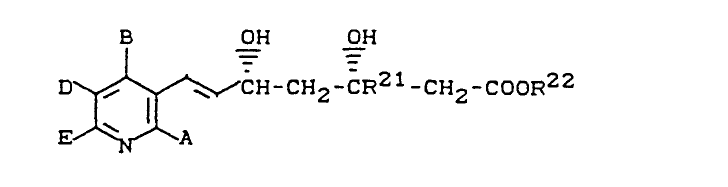

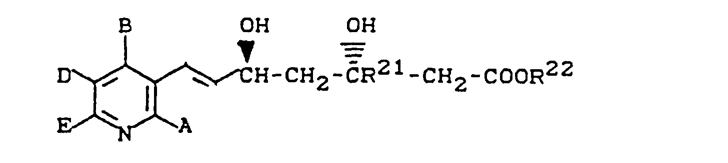

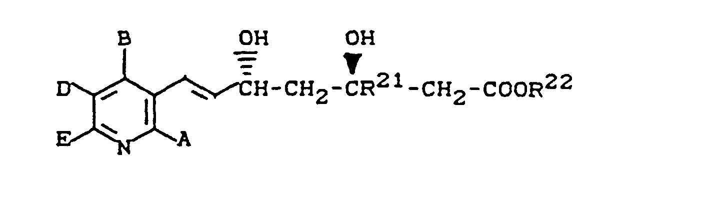

Außerdem wurde ein Verfahren zur Herstellung der substituierten Pyridine der allgemeinen Formel (I) 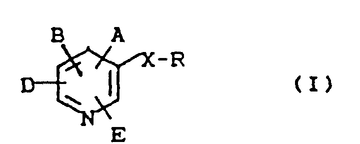 in welcher
A, B, D, E, X und R die oben angegebene Bedeutung haben, gefunden,
das dadurch gekennzeichnet ist, daß man Ketone der allgemeinen Formel (VIII) 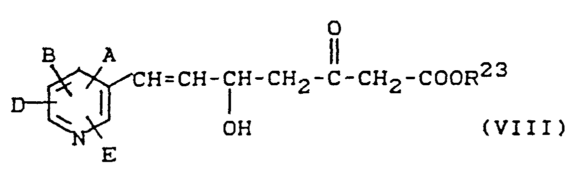 in welcher
A, B, D und E die oben angegebene Bedeutung haben,
und
- R²³: - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = -CH₂-CH₂-) die Ethenverbindungen (X = -CH=CH-) nach üblichen Methoden hydriert,
und gegebenenfalls Isomeren trennt.

Das erfindungsgemäße Verfahren kann durch das folgendes Formelschema erläutert werden:

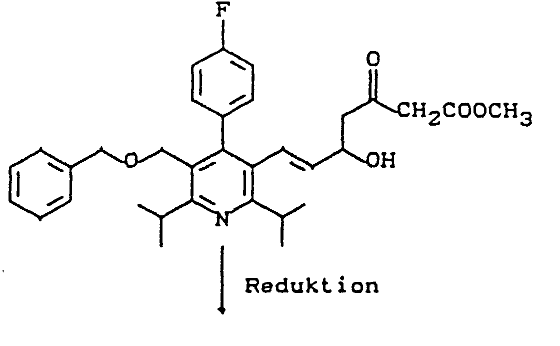

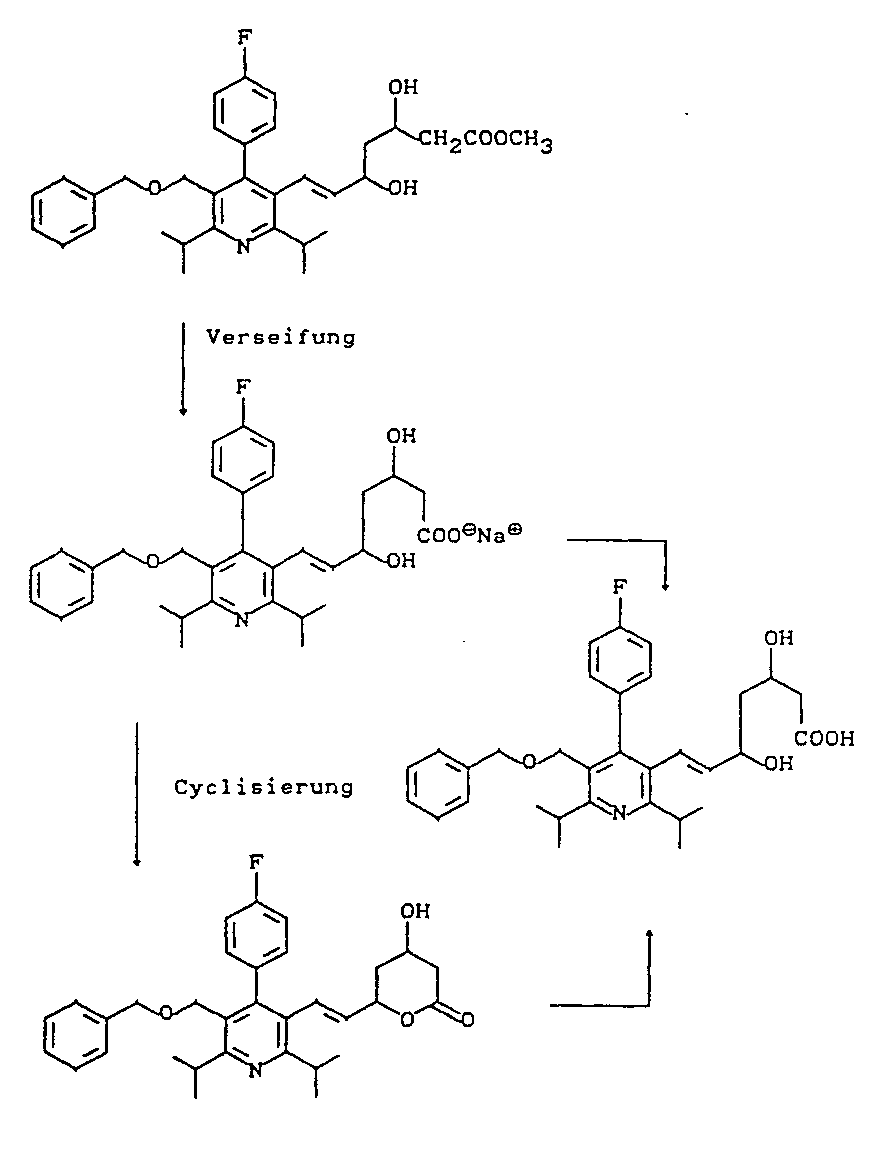

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kahumboranat, Zinkboranat, Lithium-trialkylhydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels-Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic) 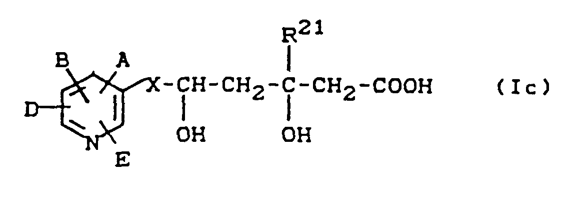 in welcher
A, B, D, E und R²⁰ die oben angegebene Bedeutung haben.

Die Carbonsäureester im Rahmen der allgemeinen Formel (I) entsprechen der Formel (Id) 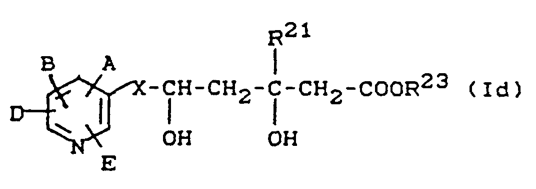 in welcher
A, B, D, E, und R²¹ die oben angegebene Bedeutung haben,
und
R²³ - für Alkyl steht.

Die Salze der erfindungsgemäßen Verbindungen im Rahmen der allgemeinen Formel (I) entsprechen der Formel (Ie) 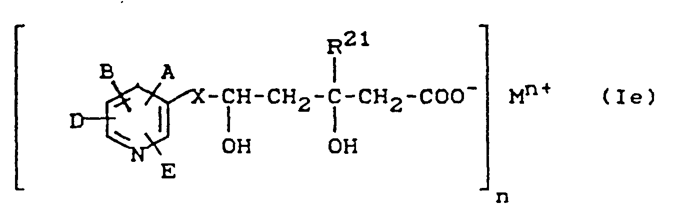 in welcher
A, B, D, E und R²¹ die oben angegebene Bedeutung haben, und
Mⁿ⁺ für ein Kation steht, wobei n die Wertigkeit angibt.

Die Lactone im Rahmen der allgemeinen Formel (I) entsprechen der Formel (If) 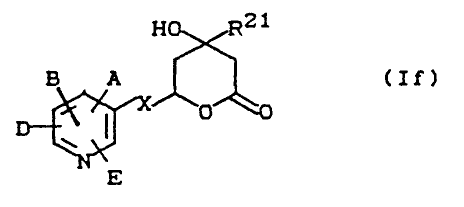 in welcher
A, B, D, E und R²¹ die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kahummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäueren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cylisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid-Paratoluolsulfonat, N-Cyclohexyl-N-[2-(N''-methylmorpholinium)ethyl]carbodiimid oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0° C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben werden. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig) 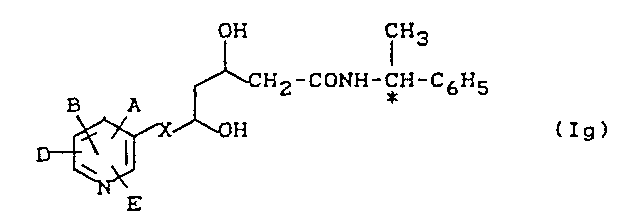 überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

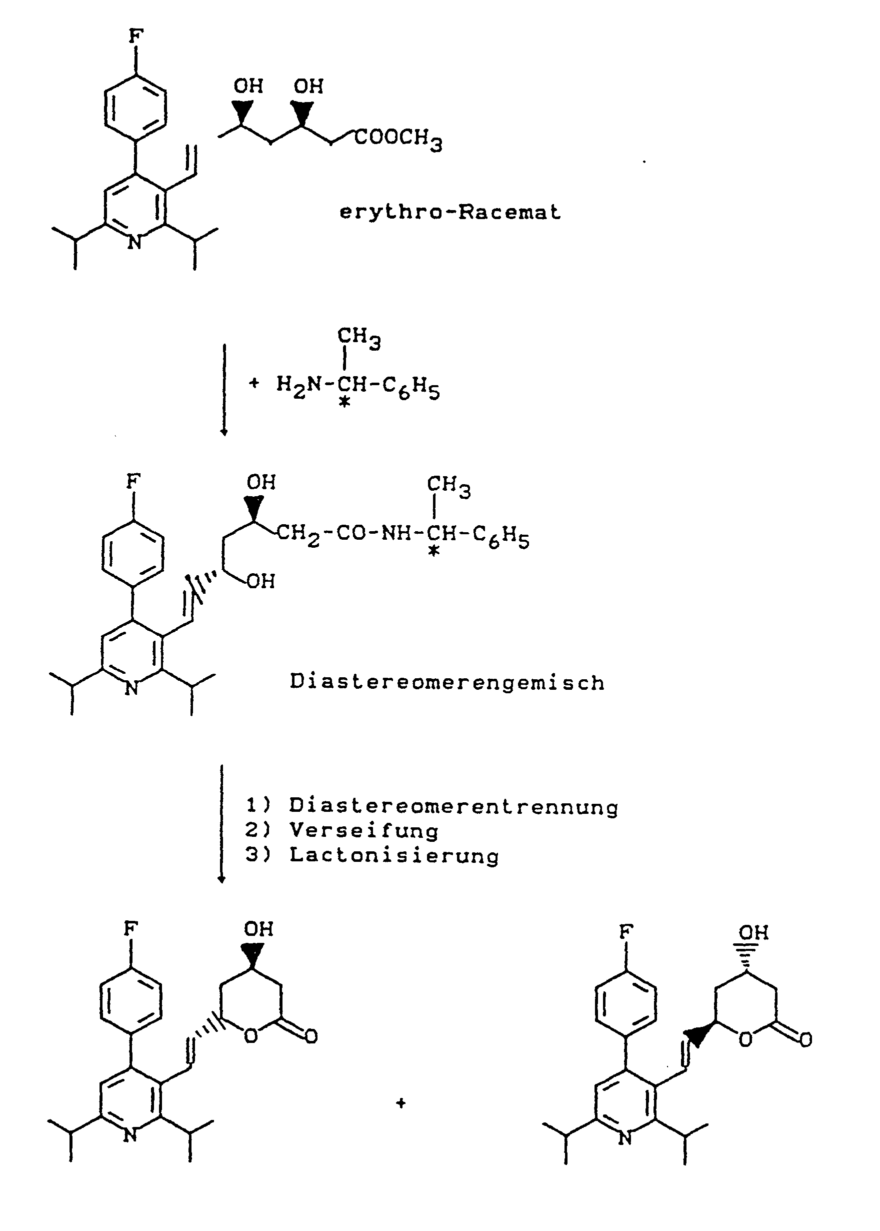

Die als Ausgangsstoffe eingesetzten Ketone (VIII) sind neu.

Es wurde ein Verfahren zur Herstellung der erfindungsgemäßen Ketone der allgemeinen Formel (VIII) 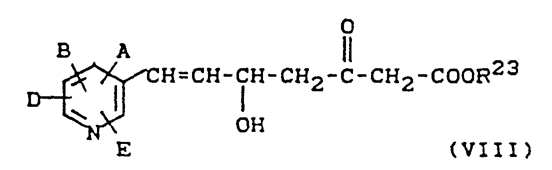 in welcher
A, B, D, E und R²³ die oben angegebene Bedeutung haben, gefunden, das dadurch gekennzeichnet ist, daß man Aldehyde der allgemeinen Formel (IX) 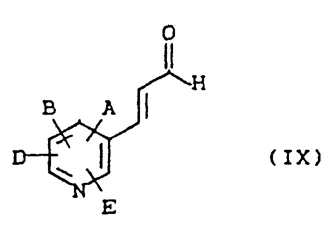 in welcher
A, B, D und E die oben angegebene Bedeutung haben,
in inerten Lösemitteln mit Acetessigester der allgemeinen Formel (X) 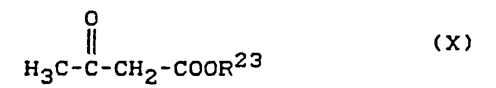 in welcher
R²³ die oben angegebene Bedeutung hat,
in Anwesenheit von Basen umsetzt.

Das erfindungsgemäße Verfahren kann beispielsweise durch folgendes Formelschema erläutert werden:

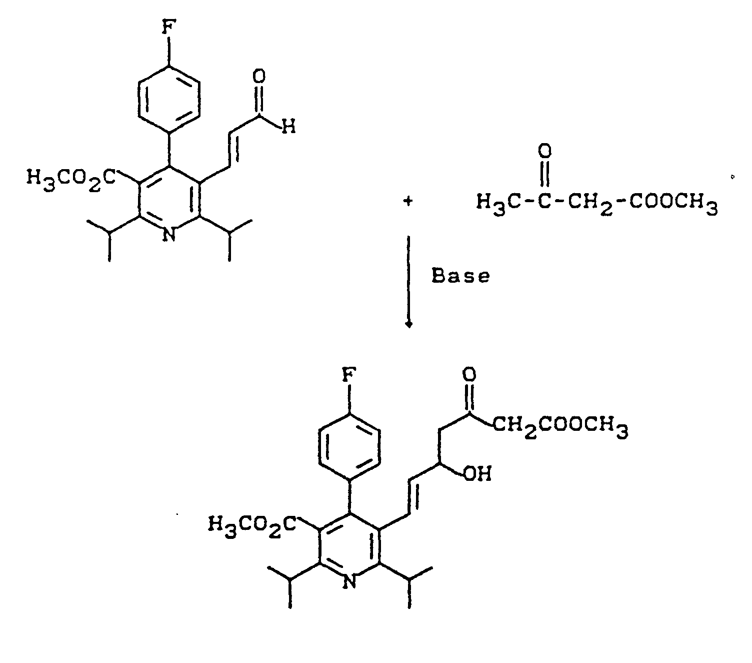

Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder'Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die Verbindungen des Typs (Ia) erläutert werden.

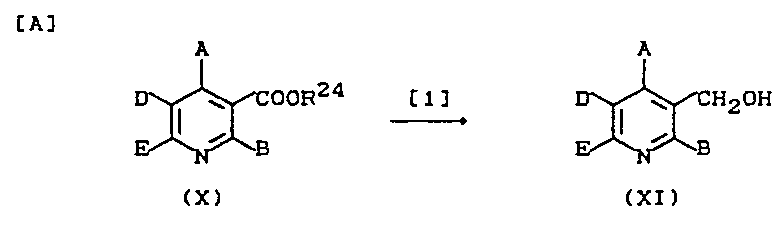

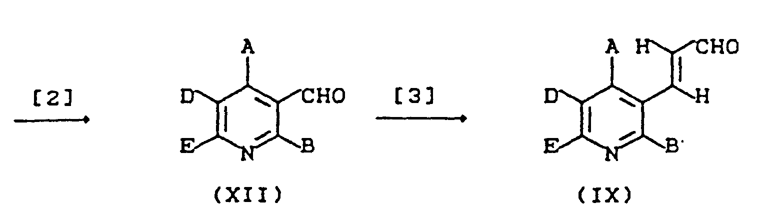

Hierbei werden gemäß Schema A Pyridine der Formel (X),in welchen R²⁴ für einen Alkylrest mit bis zu 4 Kohlenstoffatomen steht, im ersten Schritt [1] in inerten Lösemitteln wie Ethern, beispielsweise Diethylether, Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70°C bis +100°C, vorzugsweise von -70°C bis Raumtemperatur, bzw. von Raumtemperatur bis 70°C je nach verwendetem Reduktionsmittel zu den Hydroxymethylverbindungen (XI) reduziert. Vorzugsweise wird die Reduktion mit Lithiumaluminiumhydrid in Tetrahydrofuran in einem Temperaturbereich von Raumtemperatur bis 80°C durchgeführt. Die Hydroxymethylverbindungen (XI) werden im zweiten Schritt [2] nach üblichen Methoden zu den Aldehyden (VII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XII) werden im dritten Schritt [3] mit

Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten Pyridine der Formel (X) erhält man hierbei im allgemeinen gemäß Schema B durch Oxidation von Dihydropyridinen (XIII), die wiederum je nach der Bedeutung des Restes D durch Variation der entsprechenden funktionellen Gruppen erhalten wurden. Die hierbei als Ausgangsstoffe eingesetzten Dihydropyridine sind bekannt oder können nach bekannten Methoden hergestellt werden [EP-A 88 276, DE-A 28 47 236]. Die Oxidation der Dihydropyridine (XIII) zu den Pyridinen (X) kann beispielsweise mit Chromoxid in Eisessig in einem Temperaturbereich von -20°C bis +150°C, vorzugsweise bei Rückflußtemperatur, oder mit 2,3-Dichlor-5,6-dicyan-p-benzochinon als Oxidationsmittel in inerten Lösemitteln wie Chlorkohlenwasserstoffe, vorzugsweise Methylenchlorid in einem Temperaturbereich von 0°C bis +100°C, vorzugsweise bei Raumtemperatur durchgeführt werden.

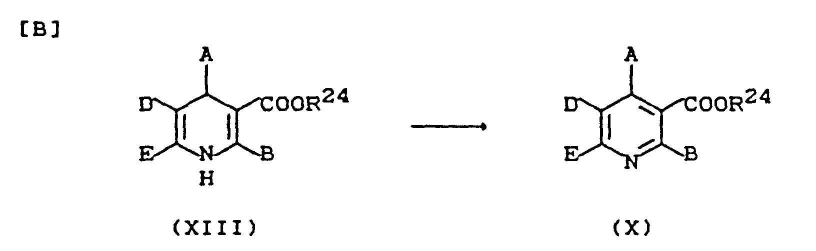

Die Variation des Restes D soll in den folgenden Reaktionsgleichungen an einigen Beispielen erläutert werden:

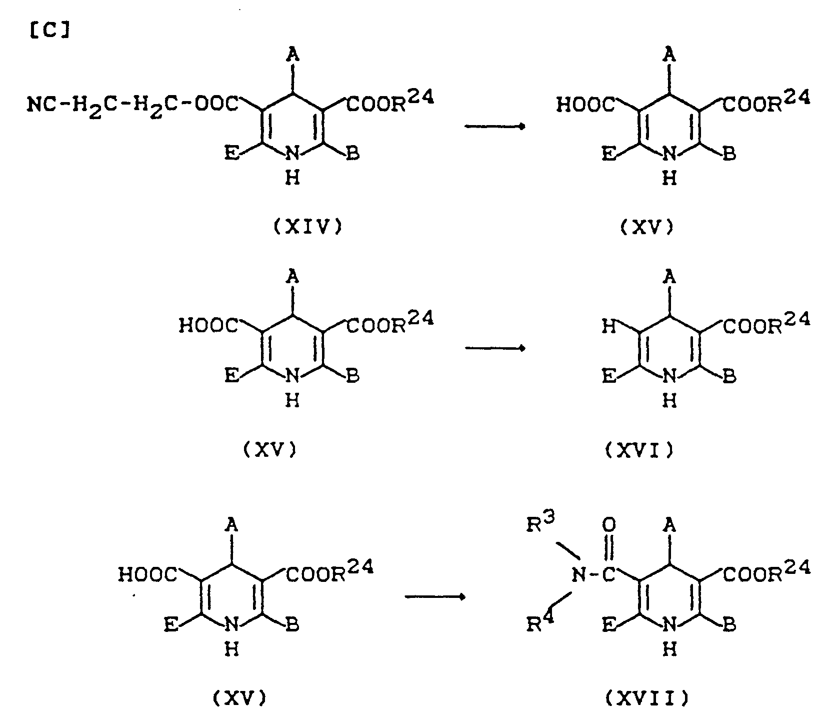

Die Dihydropyridine (XIV) können zu den Dihydropyridincarbonsäuren (XV) verseift werden, beispielsweise durch Umsetzung mit einem Alkalihydroxyd in Dimethoxyethan bei Raumtemperatur. Die Dihydropyridincarbonsäuren (XV) können beispielsweise durch Erhitzen auf 200°C in Diethylenglykol zu den Dihydropyridinen (XVI) decarboxyliert werden. Außerdem können die Dihydropyridincarbonsäuren (XV) nach bekannten Methoden zu den Dihydropyridincarbonsäureamiden (XVII) umgesetzt werden, beispielsweise durch Reaktion mit Dicyclohexylcarbodiimid.

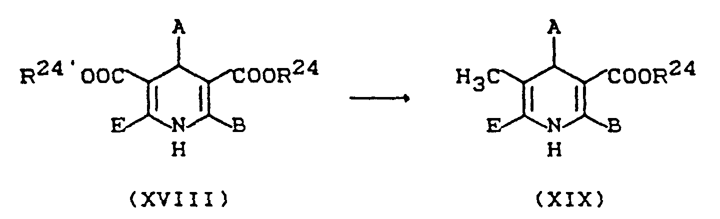

Die Dihydropyridine (XVIII) können mit üblichen Reduktionsmitteln zu den Dihydropyridinen (XIX) reduziert werden, beispielsweise durch Umsetzung Lithiumaluminiumhydrid in Tetrahydrofuran, bei Raumtemperatur oder in der Siedehitze.

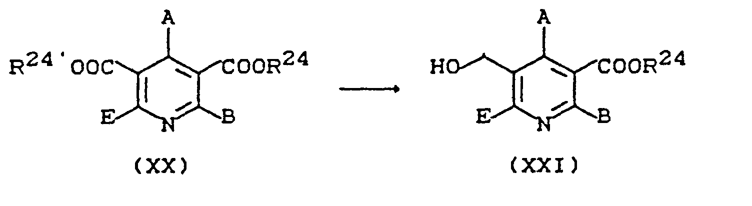

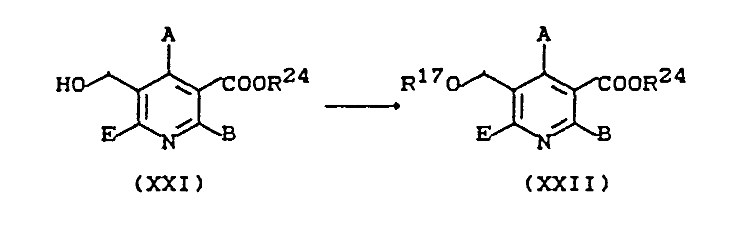

Die Pyridine (XX), die wie oben beschrieben, aus den Dihydropyridinen (XVIII) durch Oxidation hergestellt werden, können durch geeignete Reduktionsmittel, wie beispielsweise Lithiumaluminiumhydrid, Diisobutylaluminiumhydrid oder Natrium-bis-(2-methoxyethoxy)-dihydroaluminat in inerten Lösemitteln, wie beispielsweise Tetrahydrofuran, zu den Pyridinen (XXI) reduziert werden.

Die Pyridine (XXI) können nach bekannten Methoden zu den Pyridinen (XXII) umgesetzt werden, beispielsweise durch Reaktion mit einem Alkyl- oder Benzylhalogenat in Gegenwart einer Base wie beispielsweise Natriumhydrid oder beispielsweise durch Umsetzung mit einem Trialkylsilylhalogenid oder einem Säurehalogenid in Gegenwart einer Base wie Imidazol, Pyridin oder Triethylamin. Die Hydroxygruppe der Pyridine (XXI) kann nach bekannten Methoden in eine Abgangsgruppe überführt werden, z.B. durch Umsetzung mit Trifluormethansulfonsäureanhydrid, Thionylchlorid oder Methansulfonsäurechlorid in Gegenwart einer Base. Die Abgangsgruppe kann dann nach bekannten Methoden gegen Nucleophile ausgetauscht werden.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutarylCoenzym A (HGM-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden.
Die erfindungsgemäßen Wirkstoffe bewirken außerdem eine Senkung des Cholesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen,

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Clycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstvergtändlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw, der Art des Applikationsweges, vom individuellen Verhalten gegenüber dem Medikament , der Art von dessen Formulierung und dem Zeitpunkt bzw. Intervall, zu welchen die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

(E/Z)-4-Carboxyethyl-5-(4-fluorphenyl)-2-methyl-pent-4-en-3-on 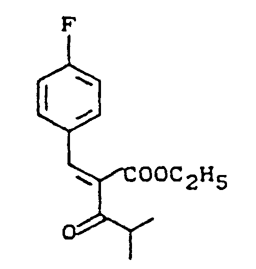 62 g (0,5 Mol) 4-Fluorbenzaldehyd und 79 g (0,5 mol) Isobutyrylessigsäureethylester werden in 300 ml Isopropanol vorgelegt und mit einem Gemisch aus 2,81 ml (28 mmol) Piperidin und 1,66 ml (29 mmol) Essigsäure in 40 ml Isopropanol versetzt. Man läßt 48 Stunden bei Raumtemperatur rühren, engt im Vakuum ein und destilliert den Rückstand im Hochvakuum.
- Kp 0,5 mm:: 127°C
- Ausbeute:: 108,7 g (82,3% der Theorie)

### Beispiel 2

1,4-Dihydro-2,6-diisopropyl-4-(4-fluorphenyl)-pyridin-3,5-dicarbonsäure-diethylester 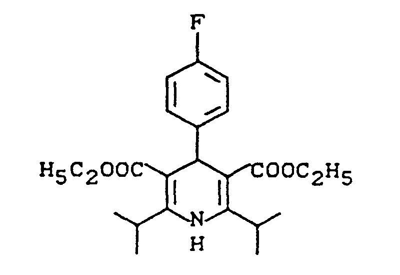 98 g (0,371 mol) der Verbindung aus Beispiel 1 werden mit 58,3 g (0,371 mol) 3-Amino-4-methyl-pent-2-en-säure-ethylester in 300 ml Ethanol 18 h am Rückfluß gekocht. Die Mischung wird auf Raumtemperatur abgekühlt, das Lösungsmittel im Vakuum abgedampft und die nicht umgesetzten Ausgangsmaterialien im Hochvakuum bei 130°C abdestilliert. Den zurückbleibenden Sirup verrührt man mit n-Hexan und saugt den ausgefallenen Niederschlag ab, wäscht mit n-Hexan nach und trocknet im Exsikkator. Ausbeute: 35 g (23,4% der Theorie)
- ¹H-NMR (CDCl₃): δ =: 1,1 - 1,3 (m, 18H); 4,05 - 4,25 (m, 6H); 5,0 (s, 1H); 6,13 (s, 1H); 6,88 (m, 2H); 7,2 (m, 2H) ppm.

### Beispiel 3

2,6-Diisopropyl-4-(4-fluorphenyl)-pyridin-3,5-dicarbonsäure-diethylester 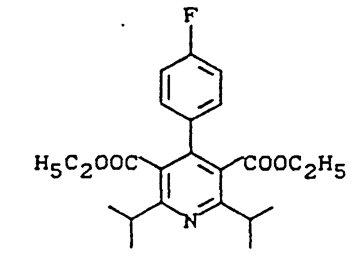 Zu einer Lösung von 6,6 g (16,4 mmol) der Verbindung aus Beispiel 2 in 200 ml Methylenchlorid p.A. gibt man 3,8 g (16,4 mmol) 2,3-Dichlor-5,6-dicyan-p-benzochinon und rührt 1 h bei Raumtemperatur. Dann wird über Kieselgur abgesaugt, die Methylenchloridphase dreimal mit je 100 ml Wasser extrahiert und über Magnesiumsulfat getrocknet. Nach Einengen im Vakuum wird der Rückstand an einer Säule (100 g Kieselgel 70-230 mesh, ø 3,5 cm, mit Essigester/Petrolether 1:9) chromatographiert.
- Ausbeute:: 5,8 g (87,9% der Theorie)
- ¹H-NMR (CDCl₃): δ =: 0,98 (t, 6H); 1,41 (d, 12H); 3,1 (m, 2H); 4,11 (q, 4H); 7,04 (m, 2H); 7,25 (m, 2H) ppm.

### Beispiel 4

2,6-Diisopropyl-4-(4-fluorphenyl)-5-hydroxymethyl-pyridin-3-carbonsäure-ethylester 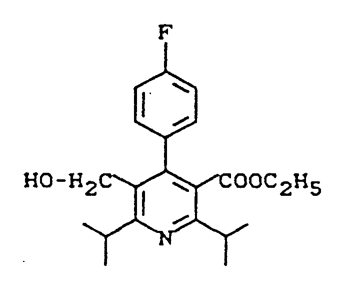 Unter Stickstoff gibt man zu einer Lösung von 9,2 g (23 mmol) der Verbindung aus Beispiel 3 in 100 ml trockenem Tetrahydrofuran bei -10°C bis -5°C 21 ml (80,5 mmol) einer 3,5 molaren Lösung von Natrium-bis-(2-methoxyethoxy)-dihydroaluminat in Toluol und rührt 5 h bei Raumtemperatur. Nach Abkühlen auf 0°C tropft man vorsichtig 100 ml Wasser zu und extrahiert dreimal mit je 100 ml Essigester. Die vereinigten organischen Phasen werden mit gesättigter Natriumchlorid-Lösung gewaschen, über Magnesiumsulfat getrocknet und im Vakuum eingedampft. Der Rückstand wird an einer Säule (200 g Kieselgel 70-230 mesh, ø 4,5 cm, mit Essigester/Petrolether 3:7) chromatographiert.
- Ausbeute:: 7,2 g (87,2% der Theorie)
- ¹H-NMR (CDCl₃): δ =: 0,95 (t, 3H); 1,31 (m, 12H); 3,05 (m, 1H); 3,48 (m, 1H), 3,95 (q, 2H); 4,93 (d, 2H); 7,05 - 7,31 (m, 4H) ppm.

### Beispiel 5

5-(tert.Butyldimethylsilyloxymethyl)-2,6-diisopropyl-4-(4-fluorphenyl)-pyridin-3-carbonsäure-ethylester 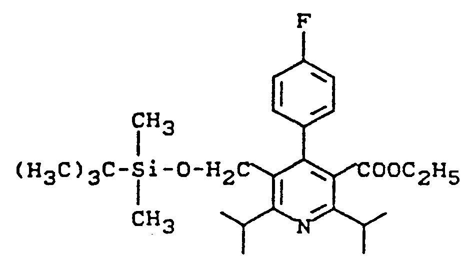 Zu einer Lösung von 4,5 g (12,5 mmol) der Verbindung aus Beispiel 4 in 50 ml Dimethylformamid gibt man bei Raumtemperatur 2,1 g (13,8 mmol) tert.Butyldimethylsilylchlorid, 1,8 g (27,5 mmol) Imidazol und 0,05 g 4-Dimethylaminopyridin. Es wird über Nacht bei Raumtemperatur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Magnesiumsulfat getrocknet und im Vakuum eingeengt. Der Rückstand wird an einer Säule (150 g Kieselgel, 70-230 mesh, ø 4 cm, mit Essigester/Petrolether 1:9) chromatographiert.
- Ausbeute:: 4,2 g (73,7% der Theorie)
- ¹H-NMR (CDCl₃): δ =: 0,0 (s, 6H); 0,9 (s, 9H); 1,02 (t, 3H); 1,35 (m, 12H); 3,1 (m, 1H); 3,47 (m, 1H); 4,03 (q, 2H); 4,4 (s, 2H); 7,05 - 7,40 (m, 4H) ppm.

### Beispiel 6

3-(tert.Butyldimethylsilyloxymethyl)-2,6-diisopropyl-4-(4-fluorphenyl)-5-hydroxymethyl-pyridin 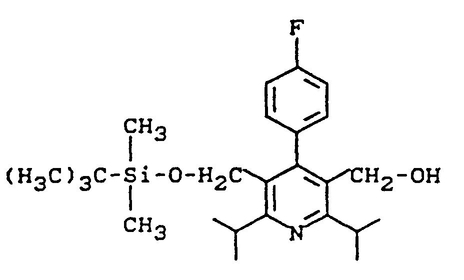 Unter Stickstoff gibt man zu einer Lösung von 4,2 g (9,2 mmol) der Verbindung aus Beispiel 5 in 100 ml trockenem Tetrahydrofuran bei 0°C 9,2 ml (32,2 mmol) einer 3,5 molaren Lösung von Natrium-bis-(2-methoxyethoxy)-dihydroaluminat in Toluol und rührt über Nacht bei Raumtemperatur. Nach Abkühlen auf 0°C tropft man vorsichtig 100 ml Wasser zu und extrahiert dreimal mit je 100 ml Essigester. Die vereinigten organischen Phasen werden einmal mit gesättigter Natriumchlorid-Lösung gewaschen, über Magnesiumsulfat getrocknet und im Vakum eingeengt. Der Rückstand wird an einer Säule (100 g Kieselgel 70-230 mesh, ø 3,5 cm, mit Essigester/Petrolether 2:8) chromatographiert.
- Ausbeute:: 2,4 g (60% der Theorie)
- ¹H-NMR (CDCl₃): δ =: 0,2 (s, 6H); 1,11 (s, 9H); 1,6 (m, 12H); 3,7 (m, 2H); 4,55 (s, 2H); 4,65 (d, 2H); 7,35 - 7,55 (m, 4H) ppm.

### Beispiel 7

5-(tert.Butyldimethylsilyloxymethyl)-2,6-diisopropyl)-4-(4-fluorphenyl)-pyridin-3-carbaldehyd 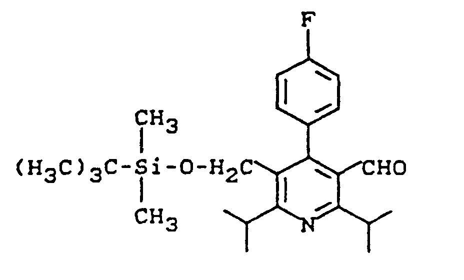 Zu einer Lösung von 2,7 g (6,2 mmol) der Verbindung aus Beispiel 6 in 50 ml Methylenchlorid gibt man 1,24g (12,4 mmol) neutrales Aluminiumoxid und 2,7 g (12,4 mmol) Py-ridiniumchlorochromat und rührt 1 h bei Raumtemperatur. Man saugt über Kieselgur ab und wäscht mit 200 ml Methylenchlorid nach. Die Methylenchloridphase wird im Vakuum eingeengt und der Rückstand an einer Säule (100 g Kieselgel 70-230 mesh, ø 3,5 cm, mit Essigester/Petrolether 1:9) chromatographiert.
- Ausbeute:: 2 g (77% der Theorie)
- ¹H-NMR (CDCl₃): δ =: 0,0 (s, 6H); 0,9 (s, 9H); 1,35 (m, 12H); 3,5 (m, 1H); 3,9 (m, 1H); 4,38 (s, 2H); 7,15 - 7,35 (m, 4H); 9,8 (s, 1H) ppm.

### Beispiel 8

(E)-3-[5-tert.Butyldimethylsilyloxymethyl-2,6-diisopropyl-4-(4-fluorphenyl)-pyrid-3-yl]-prop-2-enal 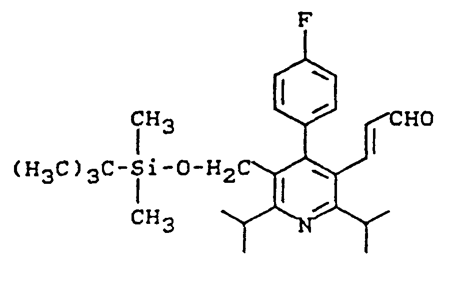 Unter Stickstoff tropft man zu einer Suspension von 180 mg (6 mmol) 80%igem Natriumhydrid in 15 ml trockenem Tetrahydrofuran bei -5°C 1,6 g (6 mmol) Diethyl-2-(cyclohexylamino)-vinylphosphonat gelöst in 30 ml trockenem Tetrahydrofuran. Nach 30 min werden bei derselben Temperatur 2 g (4,7 mmol) der Verbindung aus Beispiel 7 in 40 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Magnesiumsulfat getrocknet. Nach Einengen im Vakuum wird der Rückstand in 70 ml Toluol aufgenommen, mit einer Lösung von 0,9 g (7 mmol) Oxalsäure-Dihydrat in 30 ml Wasser versetzt und 30 min zum Rückfluß erhitzt. Nach Abkühlen auf Raumtemperatur werden die Phasen getrennt, die organische Phase mit gesättigter Natriumchlorid-Lösung gewaschen, über Magnesiumsulfat getrocknet und im Vakuum eingeengt. Der Rückstand wird an einer Säule (100 g Kieselgel 70-230 mesh ø 3,5 cm, mit Essigester/Petrolether 1:9) chromatographiert.
- Ausbeute:: 2 g (95% der Theorie)
- ¹H-NMR (CDCl₃): δ =: 0,0 (s, 6H); 0,9 (s, 9H); 1,38 (m, 12H); 3,36 (m, 1H); 3,48 (m, 1H); 4,48 (s, 2H); 6,03 (dd, 1H); 7,12-7,35 (m, 5H); 9,45 (d, 1H) ppm.

### Beispiel 9

Methyl-(E)-7-[5-tert.butyldimethylsilyloxymethyl-2,6-diisopropyl-4-(4-fluorphenyl)-pyrid-3-yl]-5-hydroxy-3-oxo-hept-6-enoat 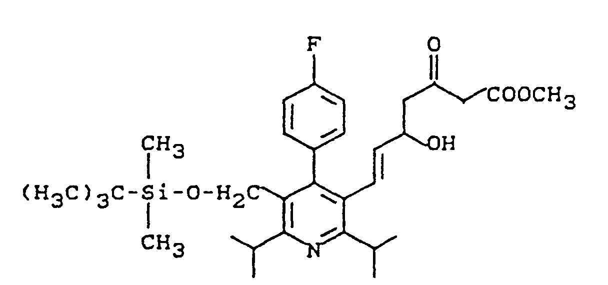 Unter Stickstoff tropft man zu einer Suspension von 330 mg (11 mmol) 80%igem Natriumhydrid in 30 ml trockenem Tetrahydrofuran bei -5°C 1,02 g (8,8 mmol) Acetessigsäuremethylester in 5 ml trockenem Tetrahydrofuran. Nach 15 min werden bei derselben Temperatur 5,5 ml (8,8 mmol) 15%iges Butyllithium in n-Hexan zugetropft und 15 min nachgerührt. Anschließend werden 2 g (4,4 mmol) der Verbindung aus Beispiel 8 gelöst in 20 ml trockenem Tetra-Zu einer Lösung von 1,9 g (3,7 mmol) der Verbindung aus Beispiel 9 in 40 ml trockenem Tetrahydrofuran gibt man bei Raumtemperatur 4,5 ml (4,5 mmol) 1 M Triethylboran-Lösung in Tetrahydrofuran, leitet während 5 min Luft durch die Lösung und kühlt auf -30°C Innentemperatur ab. Es werden 160 mg (4,5 mmol) Natriumborhydrid und langsam 3 ml Methanol zugegeben, 30 min bei -30°C gerührt und dann mit einem Gemisch von 12 ml 30%igem Wasserstoffperoxid und 25 ml Wasser versetzt. Die Temperatur läßt man dabei bis 0°C ansteigen und rührt noch 30 min nach. Die Mischung wird dreimal mit je 70 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Säule (80 g Kieselgel 230-400 mesh, ø 2,5 cm, mit Essigester/Petrolether 4:6) chromatographiert.
- Ausbeute:: 1,5 g (78,9% der Theorie)
- ¹H-NMR (CDCl₃): δ =: 0,0 (s, 6H); 0,9 (s, 9H); 1,35 (m, 12H); 1,5 (m, 2H); 2,5 (m, 2H); 3,35 (m, 1H); 3,45 (m, 1H); 3,8 (s, 3H); 4,15 (m, 1H); 4,45 (m, 3H); 5,32 (dd, 1H); 6,38 (d, 1H) ; 7,05 - 7,25 (m, 4H) ppm.

### Beispiel 11

Methyl-erythro-(E)-7-[2,6-diisopropyl-4-(4-fluorphenyl)-5-hydroxymethyl-pyrid-3-yl]-3,5-dihydroxy-hept-6-enoat 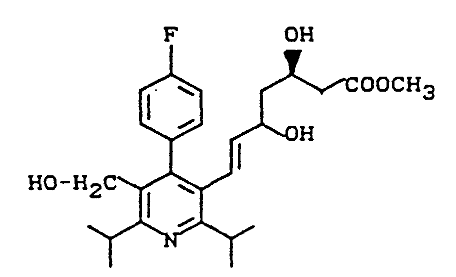 Zu 8,4 g (14,6 mmol) der Verbindung aus Beispiel 10 gelöst in 135 ml Methanol gibt man 15 ml 0,1 N Salzsäure und rührt 4 Tage bei Raumtemperatur. Die Mischung wird im Vakuum eingeengt, der Rückstand in Dichlormethan aufgenommen und mehrmals mit gesättigter Natriumhydrogencarbonat-Lösung gewaschen. Die organische Phase wird über Magnesiumsulfat getrocknet, im Vakuum eingeengt und der Rückstand an einer Säule (Kieselgel 70-230 mesh, mit Essigester/Petrolether 4:6) chromatographiert.
- Ausbeute:: 3,5 g (52,5% der Theorie)
- ¹H-NMR (CDCl₃): δ =: 1,25 (m, 6H); 1,33 (d, 6H); 1,40 (m, 2H); 2,41 (m, 2H); 3,30 (m, 1H); 3,45 (m, 1H); 3,71 (s, 3H); 4,07 (m, 1H); 4,28 (m, 1H); 4,39 (d, 2H); 5,25 (dd, 1H); 6,30 (d, 1H); 7,08 (m, 4H) ppm.

### Beispiel 12

5-Benzyloxymethyl-2,6-diisopropyl-4-(4-fluorphenyl)-pyridin-3-carbonsäure-ethylester 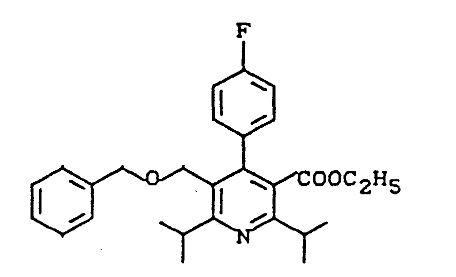 Unter Stickstoff tropft man zu einer Suspension von 414 mg (13,8 mmol) 80%igem Natriumhydrid in 20 ml Dimethylformamid bei 0°C 4,5 g (12,5 mmol) der Verbindung aus Beispiel 4 in 50 ml Dimethylformamid und rührt 30 min. bei derselben Temperatur. Anschließend werden 1,65 ml (13,8 mmol) Benzylbromid in 20 ml Dimethylformamid zugetropft und weitere 3 h bei Raumtemperatur gerührt. Die Mischung wird bei 0°C auf 300 ml Wasser gegossen und dreimal mit je 150 ml Ether extrahiert. Die vereinigten organischen Phasen werden einmal mit gesättigter Natriumchlorid-Lösung gewaschen, über Magnesiumsulfat getrocknet und im Vakuum eingeengt. Der Rückstand wird an einer Säule (100 g Kieselgel 70-230 mesh, ø 3,5 cm, mit Essigester/Petrolether 1:10) chromatographiert.
- Ausbeute:: 2,6 g (46,4% der Theorie)
- ¹H-NMR( CDCl₃): δ =: 0,95 (t, 3H); 1,3 (m, 12H); 3,05 (m, 1H); 3,38 (m, 1H); 3,97 (q, 2H); 4,2 (s, 2H); 4,38 (s, 2H); 7,02 (m, 2H); 7,25 (m, 7H) ppm.

### Beispiel 13

3-Benzyloxymethyl-2,6-diisopropyl-4-(4-fluorphenyl)-5-hydroxymethyl-pyridin 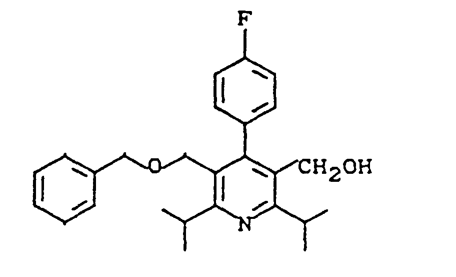 2,5 g (5,5 mmol) der Verbindung aus Beispiel 12 werden analog Beispiel 6 umgesetzt.
- Ausbeute:: 1,5 g (68% der Theorie)
- ¹H-NMR (CDCl₃): δ =: 1,3 (m, 12H); 3,35 (m, 1H); 3,45 (m, 1H); 4,13 (s, 2H); 4,35 (m, 4H); 7,08 (m, 2H); 7,25 (m, 7H) ppm.

### Beispiel 14

5-Benzyloxymethyl-2,6-diisopropyl-4-(4-fluorphenyl)-pyridin-3-carbaldehyd 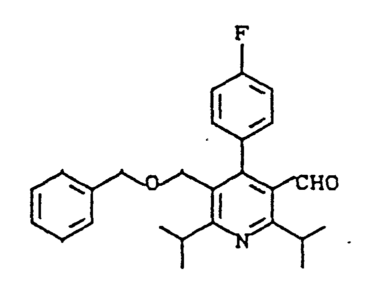 1,5 g (3,6 mmol) der Verbindung aus Beispiel 13 werden analog Beispiel 7 umgesetzt,
- Ausbeute:: 1,1 g (75,9% der Theorie)
- ¹H-NMR (CDCl₃): δ =: 1,3 (m, 12H); 3,4 (m, 1H); 3,85 (m, 1H); 4,18 (s, 2H); 4,38 (s, 2H); 7,05 - 7,35 (m, 9H); 9,75 (s, 1H) ppm.

### Beispiel 15

(E)-3-[5-Benzyloxymethyl-2,6-diisopropyl-4-(4-fluorphenyl)-pyrid-3-yl]-prop-2-enal 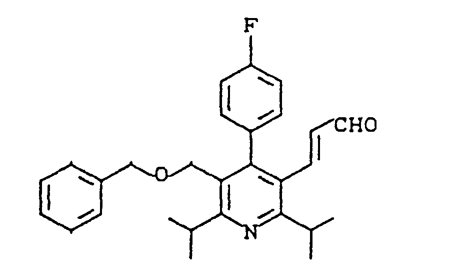 1,1 g (2,7 mmol) der Verbindung aus Beispiel 14 werden analog Beispiel 8 umgesetzt.
- Ausbeute:: 450 mg (38,8% der Theorie)
- ¹H-NMR (CDCl₃): δ =: 1,35 (m, 12H); 3,35 (m, 1H); 3,42 (m, 1H); 4,21 (s, 2H); 4,41 (s, 2H); 6,0 (dd, 1H); 7,05 - 7,4 (m, 10H); 9,38 (d, 1H) ppm.

### Beispiel 16

Methyl-(E)-7-[5-benzyloxymethyl-2,6-diisopropyl-4-(4-fluorphenyl)-pyrid-3-yl]-5-hydroxy-3-oxo-hept-6-enoat 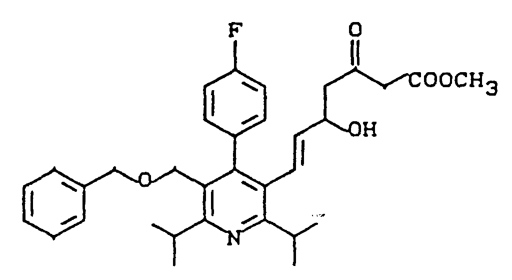 431 mg (1 mmol) der Verbindung aus Beispiel 15 werden analog Beispiel 9 umgesetzt,
- Ausbeute:: 300 mg (54,8% der Theorie)

### Beispiel 17

Methyl-erythro-(E)-7-[3-benzyloxymethyl-2,6-diisopropyl-4-(4-fluorphenyl)-pyrid-5-yl]-3,5-dihydroxy-hept-6-enoat 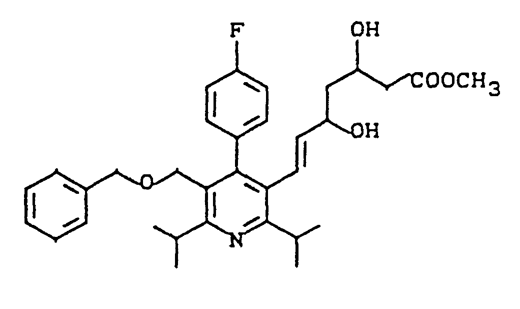 300 mg (0,55 mmol) der Verbindung aus Beispiel 16 werden analog Beispiel 10 umgesetzt.
- Ausbeute:: 180 mg (59,6% der Theorie)
- ¹H-NMR (CDCl₃): δ =: 1,2 - 1,35 (m, 12H); 1,4 (m, 2H); 2,41 (m, 2H); 3,3 (m, 2H); 3,73 (s, 3H); 4,05 (m, 1H); 4,15 (s, 2H); 4,28 (m, 1H); 4,35 (s, 2H); 5,25 (dd, 1H); 6,3 (d, 1H); 6,95 - 7,35 (m, 9H) ppm.

### Beispiel 18

1,4-Dihydro-4-(4-fluorphenyl)-2-isopropyl-6-methyl-pyridin-3,5-dicarbonsäure-3-ethyl-5-methylester 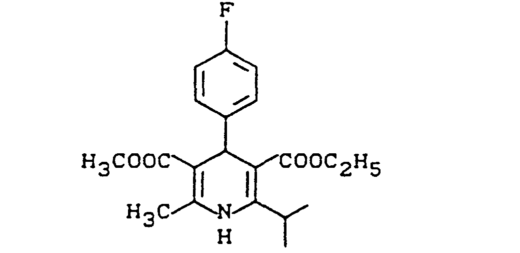 15 g (56,8 mmol) der Verbindung aus Beispiel 1 und 6,5 g (56,8 mol) 3-Aminocrotonsäuremethylester werden in 150 ml Ethanol 20 h am Rückfluß gekocht. Die Mischung wird abgekühlt, abfiltriert und im Vakuum eingeengt. Der Rückstand wird an einer Säule (250 g Kieselgel 70-230 mesh, ø 4,5 cm, mit Essigester/Petrolether 3:7) chromatographiert.
- Ausbeute:: 13,6 g (66,3% der Theorie)
- ¹H-NMR (CDCl₃): δ =: 1,2 (m, 9H); 2,35 (s, 3H); 3,65 (s, 3H); 4,12 (m, 3H); 4,98 (s, 1H); 5,75 (s, 1H); 6,88 (m, 2H); 7,25 (m, 2H) ppm.

### Beispiel 19

4-(4-Fluorphenyl)-2-isopropyl-6-methyl-pyridin-3,5-dicarbonsäure-3-ethyl-5-methylester 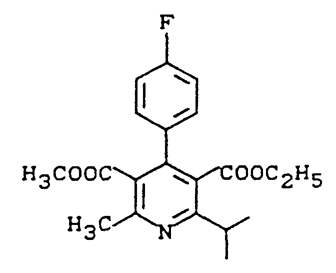 13,5 g (37,4 mmol) der Verbindung aus Beispiel 18 werden analog Beispiel 3 umgesetzt.
- Ausbeute:: 9,5 g (70,9% der Theorie)
- ¹H-NMR (CDCl₃): δ =: 0,98 (t, 3H); 1,31 (d, 6H); 2,6 (s, 3H); 3,11 (m, 1H); 3,56 (s, 3H); 4,03 (q, 2H); 7,07 (m, 2H); 7,25 (m, 2H) ppm.

### Beispiel 20

4-(4-Fluorphenyl)-5-hydroxymethyl-2-isopropyl-6-methyl-pyridin-3-carbonsäure-ethylester 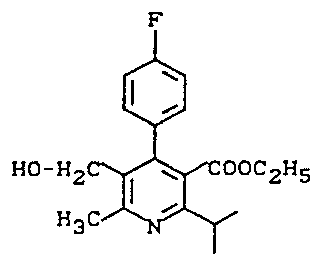 Unter Stickstoff gibt man zu einer Lösung von 9,5 g (26,5 mmol) der Verbindung aus Beispiel 19 in 200 ml absolutem Tetrahydrofuran bei 0°C 26,5 ml (92,75 mmol) einer 3,5 molaren Lösung von Natrium-bis-(2-methoxyethoxy)-dihydroaluminat in Toluol und rührt 30 min bei Raumtemperatur. Nach erneutem Abkühlen auf 0°C, tropft man vorsichtig 200 ml Wasser zu und extrahiert dreimal mit je 150 ml Essigester. Die vereinigten organischen Phasen werden einmal mit gesättigter Natriumchlorid-Lösung gewaschen, über Magnesiumsulfat getrocknet und im Vakuum eingeengt. Der Rückstand wird an einer Säule (200 g Kieselgel 70-230 mesh, ø 4,5 cm, mit Essigester/ Petrolether 2:8) chromatographiert.
- Ausbeute:: 4,2 g (48,2% der Theorie)
- ¹H-NMR (CDCl₃): δ =: 0,98 (t, 3H); 1,3 (d, 6H); 2,73 (s, 3H); 3,05 (m, 1H); 3,98 (q, 2H); 4,45 (d, 2H); 7,1 (m, 2H); 7,25 (m, 2H) ppm.

### Beispiel 21

(E/Z)-4-Carboxyethyl-5-(4-fluor-3-phenoxyphenyl)-2-methyl-pent-4-en-3-on 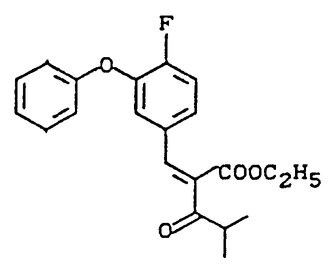 49 g (0,31 mol) Isobutyrylessigsäureethylester und 67 g (0,31 mol) 3-Phenoxy-4-fluorbenzaldehyd werden in 300 ml Isopropanol vorgelegt und mit einem Gemisch aus 1,81 ml (18 mmol) Piperidin und 1,06 ml (18,6 mmol) Essigsäure in 30 ml Isopropanol versetzt. Man rührt über Nacht bei Raumtemperatur, engt dann im Vakuum ein und trocknet im Hochvakuum.
- Ausbeute:: 110 g (wurde ohne weitere Reinigung in Beispiel 22 eingesetzt).

### Beispiel 22

1,4-Dihydro-2,6-diisopropyl-4-(4-fluor-3-phenoxy-phenyl)-pyridin-3,5-dicarbonsäure-diethylester 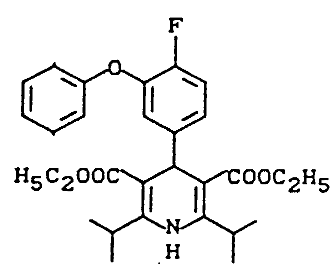 30 g (84,3 mmol) der Verbindung aus Beispiel 21 und 13,2 g (84,3 mmol) 3-Amino-4-methyl-pent-2-en-säure-ethyl-ester werden in 150 ml Ethanol über Nacht am Rückfluß gekocht. Man kühlt die Mischung auf 0°C, saugt den ausgefallenen Niederschlag ab, wäscht mit Petrolether nach und trocknet im Exsikkator.
- Ausbeute:: 18,4 g (44,2% der Theorie)
- ¹H-NMR (CDCl₃): δ =: 1,05 - 1,25 (m, 18H); 4,05 - 4,2 (m, 6H); 4,95 (s, 1h); 6,03 (s, 1H); 6,85 - 7,1 (m, 6H); 7,3 (m, 2H) ppm.

### Beispiel 23

2,6-Diisopropyl-4-(4-fluor-3-phenoxy-phenyl)-pyridin-3,5-dicarbonsäure-diethylester 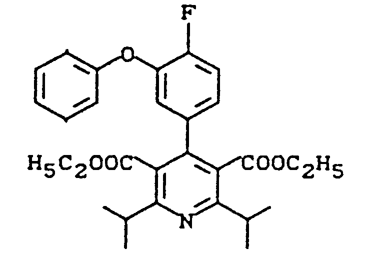 18,4 g (37,2 mmol) der Verbindung aus Beispiel 22 werden analog Beispiel 3 umgesetzt.
- Ausbeute:: 17,6 g (96% der Theorie)
- ¹H-NMR (CDCl₃): δ =: 1,05 (t, 6H); 1,29 (d, 12H); 3,08 (m, 2H); 4,05 (q, 4H); 6,95 - 7,35 (m, 8H) ppm.

### Beispiel 24

2,6-Diisopropyl-4-(4-fluor-3-phenoxy-phenyl)-5-hydroxy-methyl-pyridin-3-carbonsäure-ethylester 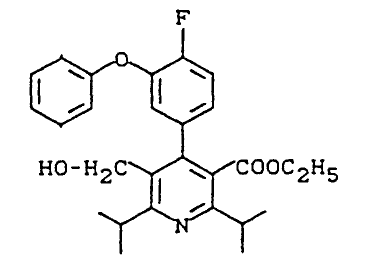 10 g (20,3 mmol) der Verbindung aus Beispiel 23 werden analog Beispiel 4 umgesetzt.
- Ausbeute:: 4,9 g (59,0% der Theorie)
- ¹H-NMR (CDCl₃): δ =: 1,07 (t, 3H); 1,3 (m, 12H); 3,04 (m, 1H); 3,47 (m, 1H); 4,05 (m, 2H); 4,45 (s, 2H); 6,95 - 7,4 (m, 8H) ppm.

### Beispiel 25

1,4-Dihydro-2,6-dimethyl-4-(4-fluorphenyl)-pyridin-3,5-dicarbonsäuremethyl-(2-cyanethyl)-ester 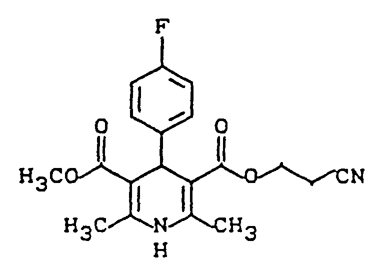 In 150 ml Ethanol werden 15,4 g (0,1 mol) 3-Aminocrotonsäure-2-cyanethylester, 12,4 g (0,1 mol) p-Fluorbenzaldehyd und 11,6 g Acetessigsäuremethylester über Nacht unter Rückfluß erhitzt. Nach dem Entfernen des Lösemittels am Rotationsverdampfer nimmt man in Essigester auf, wäscht mit Wasser, trocknet und erhält nach Entfernen des Lösemittels im Vakuum 33,8 g Rohprodukt.
- Rohausbeute:: 94,4% der Theorie
- ¹H-NMR (DMSO): δ =: 1,15 (tr, 3H, CH₃); 2,3 (m, 6H, CH₃); 2,75 (m, 2H, CH₂CN); 3,55 (s, 3H, OCH₃); 4,15 (m, 2H, OCH₂); 4,9 (m, 1H, p-FC₆H₄-CH); 6,9 - 7,3 (m, 4H, Aromaten -H); 8,8, 9,0 (2s, 1H, NH) ppm.

### Beispiel 26

1,4-Dihydro-2,6-dimethyl-4-(4-fluorphenyl)-pyridin-3,5-dicarbonsäure-methylester 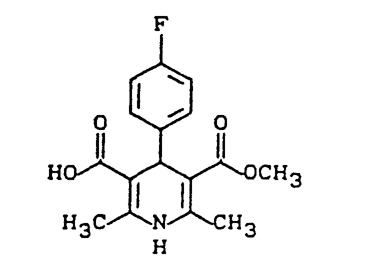 Zu einer Lösung von 12 g (0,3 mol) Natriumhydroxid in 300 ml Wasser/150 ml 1,2-Dimethoxyethan gibt man 33,8 g Rohprodukt aus Beispiel 25. Die Suspension erwärmt sich, es bildet sich eine klare Lösung. Nach Rühren bei 25°C über Nacht gibt man 100 ml Wasser hinzu, wäscht dreimal mit Dichlormethan, stellt mit verdünnter Salzsäure auf pH 1 ein und extrahiert das klebrig ausgefallene Produkt mit Dichlormethan. Nach dem Trocknen und Einengen des Lösemittels im Vakuum erhält man 25,8 g Rohprodukt.
- Rohausbeute:: 84,5% der Theorie
- ¹H-NMR (DMSO): δ =: 2,25 (s, 6H, CH₃); 3,55 (s, 3H, OCH₃); 4,85 (breites s, 1H, FC₆H₄-CH); 6,9 - 7,3 (m, 4H, Aromaten-H); 8,85 (breites s, 1H, NH); 1,7 (breit, 1H, COOH) ppm.

### Beispiel 27

1,4-Dihydro-2,6-dimethyl-4-(4-fluorphenyl)-pyridin-3-carbonsäuremethylester 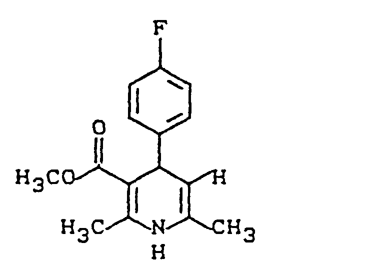 In 90 ml Bis-(2-hydroxyethyl)-ether (Diglycol) werden 12,5 g (41 mmol) Rohprodukt aus Beispiel 26 suspendiert und auf 200°C Badtemperatur erhitzt, wobei eine starke Gasentwicklung stattfindet. Nach beendeter Gasentwicklung wird die nun klare Lösung schnell abgekühlt, mit 500 ml Wasser/500 ml Ether gewaschen, die wäßrige Phase noch zweimal mit Ether gewaschen, die vereinigten Etherphasen mit Wasser, 1 N Natronlauge und Wasser gewaschen und getrocknet. Nach dem Entfernen des Lösemittels am Rotationsverdampfer erhält man 8,7 g Rohprodukt. Rohausbeute: 81,2% der Theorie

### Beispiel 28

2,6-Dimethyl-4-(4-fluorphenyl)-pyridin-3-carbonsäuremethylester 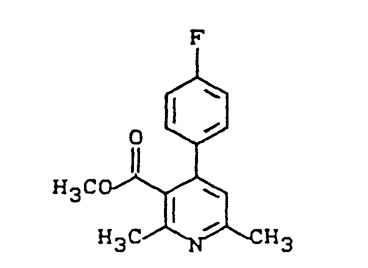 In 90 ml Eisessig werden 8,6 g (33 mmol) Rohprodukt aus Beispiel 27 und 3,3 g Chrom-VI-oxid 1 h unter Rückfluß erhitzt. Das Lösemittel wird am Rotationsverdampfer entfernt, der Rückstand mit Essigester/Petrolether 1:1 versetzt und vom Ungelöstem abgesaugt. Die Mutterlauge wird im Vakuum eingeengt und über 500 g Kieselgel mit Essigester/Petrolether 1:1 chromatographiert.
- Ausbeute:: 1,45 g (16,3% der Theorie)
- ¹H-NMR (CDCl₃): δ =: 2,6 (s, 6H, CH₃); 3,65 (s, 3H, OCH₃); 7,0 (s, 1H, Pyridin-H); 7,1-7,4 (m, 4H, Aromaten-H) ppm.

### Beispiel 29

2,6-Dimethyl-4-(4-fluorphenyl)-3-hydroxymethyl-pyridin 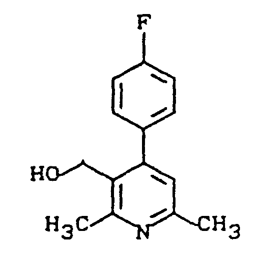 Unter Stickstoff werden bei -78°C 1,35 g (5,2 mmol) der Verbindung aus Beispiel 28 in 25 ml absolutem Tetrahydrofuran mit 5,3 ml (5,3 mmol) Diisobutylaluminiumhydrid (1 m in Toluol) versetzt und nach Aufwärmen auf 25°C mit 20%iger Kaliumhydroxydlösung hydrolysiert. Die wäßrige Phase wird mit Essigester gewaschen und die vereinigten organischen Phasen getrocknet. Nach dem Einengen im Vakuum erhält man 1,12 g Rohprodukt.
- Ausbeute:: 93% der Theorie
- ¹H-NMR (CD₃OD): δ =: 2,5 (s, 3H, CH₃); 2,7 (s, 3H, CH₃); 4,5 (s, 2H, CH₂OH); 4,6 (s, OH); 7,0 (s, 1H, Pyridin-H); 7,1 - 7,6 (m, 4H, Aromaten-H) ppm.

### Beispiel 30

2,6-Dimethyl-4-(4-fluorphenyl)-pyridin-3-carbaldehyd 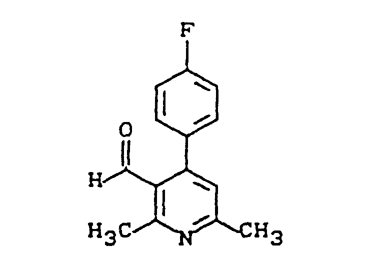 Zu 1,0 g (4,3 mmol) der Verbindung aus Beispiel 29 in 20 ml Dichlormethan gibt man portionsweise 1,5 g (7 mmol) Pyridiniumdichromat, rührt 2 h bei 25°C, chromatographiert nach Einengen über 150 g Kieselgel mit Dichlormethan/Methanol 10:1 und erhält 0,71 g Produkt.
- Ausbeute:: 72% der Theorie
- ¹H-NMR (DMSO): δ =: 2,5 (s, 3H, CH₃); 2,7 (s, 3H, CH₃); 7,2 (s, 1H, Pyridin-H); 7,3 - 7,6 (m, 4H, Aromaten-H); 9,95 (s, 1H, CHO) ppm.

### Beispiel 31

(E)-3-[2,6-Dimethyl-4-(4-fluorphenyl)-pyrid-3-yl]-prop-2-enal 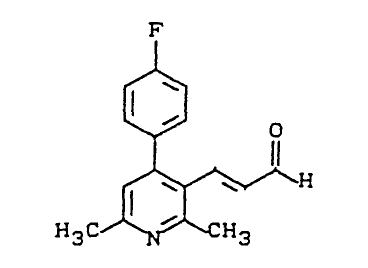 Zu 75,5 mg (3,2 mmol) Natriumhydrid in 3 ml absolutem Tetrahydrofuran gibt man unter Stickstoffatmosphäre innerhalb 15 min bei 0°C 778 mg (3,2 mmol) [2-(Cyclohexylamino)vinyl3-phosphorsäurediethylester in 3 ml Tetrahydrofuran, rührt 15 min bei 0°C und tropft eine Lösung von 0,6 g (2,6 mmol) der Verbindung aus Beispiel 30 in 3 ml Acetonitril/3 ml Dimethylformamid zu. Nach 1 h 15 min tropft man bei 0°C 0,55 ml (0,77 mmol) n-Butyllithium (1,5 m in Hexan) hinzu, rührt 15 min bei 0°C und tropft 180 mg (0,7 mmol) der Verbindung aus Beispiel 31 in 3 ml Tetrahydrofuran hinzu. Nach 1 h hydrolysiert man mit gesättigter Ammoniumchloridlösung, wäscht dreimal mit Dichlormethan, trocknet die organische Phase und erhält nach Entfernen des Lösemittels im Vakuum 0,25 g Öl.
- Rohausbeute:: 95,5% der Theorie
- ¹H-NMR (CDCl₃): δ =: 2,55, 2,58 (2s, 6H, CH₃); 2,6 (2H, CH₂); 3,45 (s, 2H, CH₂CO₂); 3,75 (s, 3H, OCH₃); 4,6 (m, 1H, CHOH); 5,45 (dd, 1H, CH-CHOH); 6,5 (d, 1H, CH=CH-CHOH); 6,9 (s, 1H, Pyridin-H); 7,0 - 7,4 (m, 4H, Aromaten-H) ppm.

### Beispiel 33

Methyl-erythro-(E)-7-[2,6-dimethyl-4-(4-fluorphenyl)-pyridin-3-yl]-3,5-dihydroxy-hept-6-enoat 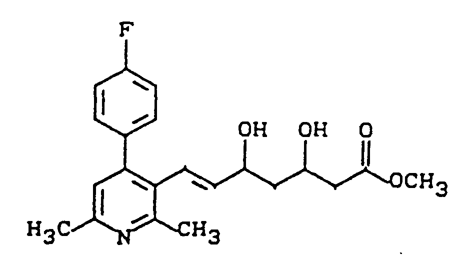 Durch eine Lösung von 0,25 g (0,67 mmmol) der Verbindung aus Beispiel 32 und 0,81 ml (0,81 mmol) Triethylboran Analog Beispiel 25 erhält man aus 2,75 g (0,1 mol) Isopropyliden-4-fluor-benzoylessigsäureethylester und 15,4 g (0,1 mol) 3-Aminocrotonsäure-2-cyanethylester 32,6 g Rohprodukt.
- Rohausbeute:: 93,6% der Theorie
- ¹H-NMR (CDCl₃): δ =: 0,7 - 1,3 (m, 9H, CH₃); 2,3, 2,35 (2s, 3H, CH₃); 2,75 (m, 2H, CH₂CN); 3,9 - 4,4 (m, 5H, CHCH₃, CH₂O); 5,6 5,7, 6,1, 6,2 (4s, 1H, CH); 7,0-8,0 (m, 4H, Aromaten-H) ppm.

### Beispiel 35

1,4-Dihydro-2-(4-fluorphenyl)-4-isopropyl-6-methyl-pyridin-3,5-dicarbonsäure-3-ethylester 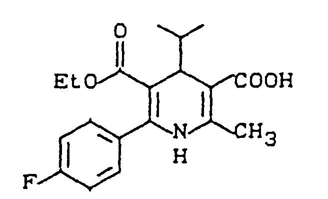 Analog Beispiel 26 erhält man aus 36 g (93,2 mmol) der Verbindung aus Beispiel 34 7,17 g Rohprodukt.
- Rohausbeute:: 22,2% der Theorie
- ¹H-NMR (DMSO): δ =: 0,8 (m, 9H, CH₃); 1,6 (m, 1H, CHCH₃); 2,2, 2,25 (2s, 3H, CH₃); 3,8 (m, 3H, CH₂O, CH); 7,2 - 7,5 (m, 4H, Aromaten-H) ppm.

### Beispiel 36

1,4-Dihydro-2-(4-fluorphenyl)-4-isopropyl-6-methyl-pyridin-3-carbonsäureethylester 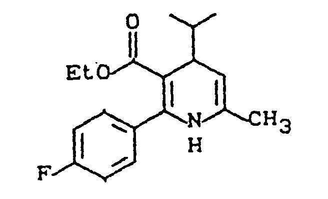 Analog Beispiel 27 erhält man aus 11,3 g (32,6 mmol) Beispiel 35 7,15 g Rohprodukt.
- Rohausbeute:: 7,25% der Theorie
- ¹H-NMR (CDCl₃): δ =: 0,8 - 1,3 (m, 9H, CH₃); 2,5, 2,6 (2s, 3H, CH₃); 3,1 (m, 1H, CHCH₃); 3,8 - 4,2 (m, 2H, CH₂O); 4,55, 5,2 (br, 1H, CH); 6,8 (s, 1H, CH); 6,9-8,0 (m, 4H, Aromaten-H) ppm.

### Beispiel 37

2-(4-Fluorphenyl)-4-isopropyl-6-methyl-pyridin-3-carbon-säureethylester 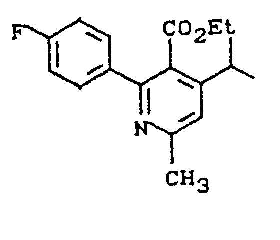 Analog Beispiel 28 erhält man aus 6,95 g (22,9 mmol) der Verbindung aus Beispiel 36 nach Chromatographie (Kieselgel, Toluol/Ethanol 95:5) 2,82 g.
- Ausbeute:: 41% der Theorie
- ¹H-NMR (CDCl₃): δ =: 1,0 (tr, 3H, CH₃); 1,3 (d, 6H, CH₃CH); 2,6 (s, 3H, CH₃); 3,1 (sept, 1H, CH); 4,1 (q, 2H, CH₂O); 7,0 (s, 1H, Pyridin-H); 7,1 - 7,6 (m, 4H, Aromaten-H) ppm.

### Beispiel 38

2-(4-Fluorphenyl)-3-hydroxymethyl-4-isopropyl-6-methyl-pyridin 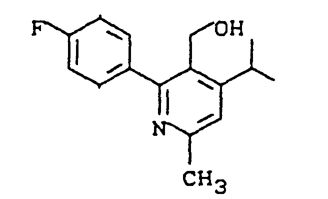 5,5 g (18,3 mmol) der Verbindung aus Beispiel 37 werden analog Beispiel 29 umgesetzt. Nach Trocknen im Exsikkator erhält man 4,24 g Rohprodukt,
- Ausbeute:: 89% der Theorie
- ¹H-NMR (CDCl₃): δ =: 1,3 (d, 6H); 2,55 (s, 3H); 3,36 (m, 1H); 4,49 (s, 2H); 7,09 (m, 3H); 7,53 (m, 2H) ppm.

### Beispiel 39

2-(4-Fluorphenyl)-4-isopropyl-6-methyl-pyridin-3-carbaldehyd 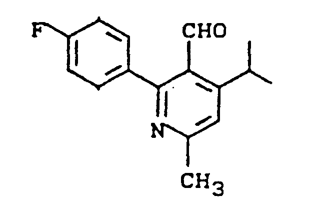 4,1 g (15,85 mmol) der Verbindung aus Beispiel 38 werden analog Beispiel 30 umgesetzt.
- Ausbeute:: 2,23 g (54,7% der Theorie)
- ¹H-NMR(CDCl₃): δ =: 1,3 (d, 6H); 2,65 (s, 3H); 3,91 (m, 1H); 7,10 - 7,28 (m, 3H); 7,5 (m, 2H); 9,91 (s, 1H) ppm.

### Beispiel 40

(E)-3-[2-(4-Fluorphenyl)-4-isopropyl-6-methyl-pyrid-3-yl]-prop-2-enal 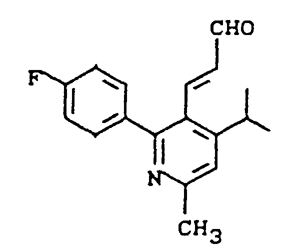 2,13 g (8,3 mmol) der Verbindung aus Beispiel 39 werden analog Beispiel 8 umgesetzt.
- Ausbeute:: 1,34 g Rohprodukt (57% der Theorie)
- ¹H-NMR (CDCl₃): δ =: 12,8 (d, 6H); 2,6 (s, 3H); 3,27 (m, 1H); 6,11 (dd, 1H); 7,05 - 7,55 (m, 6H); 9,55 (d, 1H) ppm.

### Beispiel 41

Methyl-(E)-7-[2-(4-fluorphenyl)-4-isopropyl-6-methyl-pyrid-3-yl]-5-hydroxy-3-oxo-hept-6-enoat 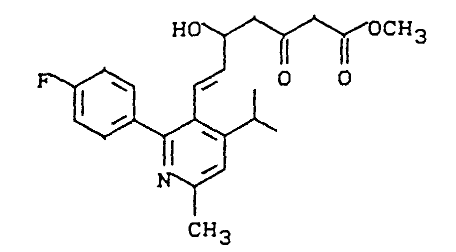 1,07 g (3,78 mmol) der Verbindung aus Beispiel 40 werden analog Beispiel 32 umgesetzt.
- Ausbeute:: 0,34 g Rohprodukt (22,5% der Theorie)
- ¹H-NMR (CDCl₃): δ =: 1,25 (d, 6H); 2,5 (m, 2H); 2,57 (s, 3H); 3,2 (m, 1H); 3,42 (s, 2H); 3,75 (s, 3H); 4,45 (m, 1H); 5,3 (dd, 1H); 6,6 (d, 1H); 7,05 (m, 3H); 7,43 (m, 2H) ppm.

### Beispiel 42

Methyl-erythro-(E)-7-[2-(4-fluorphenyl)-2-isopropyl-6-methyl-pyrid-3-yl]-3,5-dihydroxy-hept-6-enoat 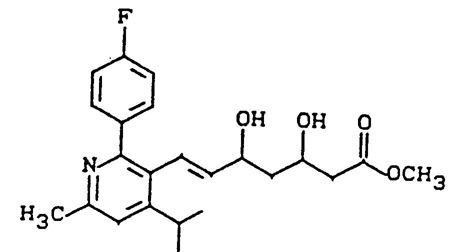 200 mg (0,5 mmol) der Verbindung aus Beispiel 41 werden analog Beispiel 33 umgesetzt.
- Ausbeute:: 21,5 mg (10,7% der Theorie)
- ¹H-NMR (CDCl₃): δ =: 1,23 (d, 6H); 1,5 (m, 2H); 2,45 (m, 2H); 2,58 (s, 3H); 3,21 (m, 1H) ; 3,72 (s, 3H); 4,11 (m, 1H); 4,38 (m, 1H); 5,31 (dd, 1H); 6,55 (d, 1H); 7,05 (m, 3H); 7,4 (m, 2H) ppm.

### Beispiel 43

1,4-Dihydro-4-(4-fluorphenyl)-2-isopropyl-6-methyl-pyridin-3,5-dicarbonsäure-5-(2-cyanethyl)-3-ethylester 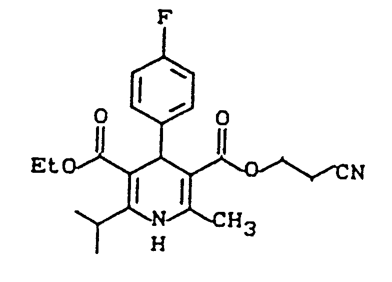 Analog Beispiel 25 erhält man aus 26,4 g (0,1 mol) 4-Fluorbenzyliden-2-butanoylessigsäureethylester und 15,4 g (0,1 mol) 3-Aminocrotonsäure-2-cyanethylester 44,6 g Rohprodukt.
- Rohausbeute:: 100% der Theorie
- ¹H-NMR (DMSO): δ =: 1,15 (m, 9H, CH₃-CH₂, CH₃-CH-CH₃); 2,3 (s, 3H, CH₃); 2,45 (m, 2H, CH₂-CN); 4,0 (q, 2H, CH₂O); 4,1 (m, 1H, CHCH₃); 4,15 (m, 2H, CH₂O); 4,4 (s, 1H, p-FC₆H₄CH); 6,9 - 7,3 (m, 4H, Aromaten-H); 8,3 (s, 1H, NH) ppm.

### Beispiel 44

1,4-Dihydro-4-(4-fluorphenyl)-2-isopropyl-6-methyl-pyridin-3,5-dicarbonsäure-3-ethylester 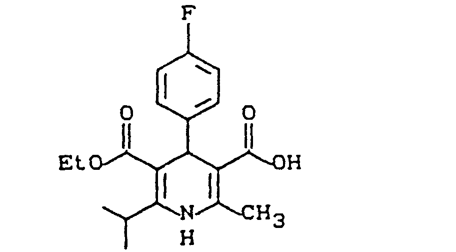 Analog Beispiel 26 erhält man aus 10,2 g (25,6 mmol) der Verbindung des Beispiels 43 8,5 g Rohprodukt.
- Rohausbeute:: 95% der Theorie
- ¹H-NMR (DMSO): δ =: 1,15 (m, 9H, CH₃CH₂, CH₃CHCH₃); 2,25, 2,3 (2s, 3H, CH₃); 4,0 (m, 3H, CH₂O, CH₃CH); 4,85, 6,3 (2s, 1H, FC₆H₄-CH); 6,9 - 7,3 (m, 4H, Aromaten-H); 8,1 (s, 1H, NH); 10,9 (s, 1H, COOH) ppm.

### Beispiel 45

1,4-Dihydro-4-(4-fluorphenyl)-2-isopropyl-6-methyl-pyridin-3-carbonsäureethylester 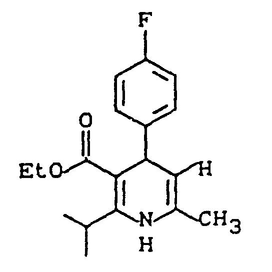 Analog Beispiel 27 erhält man aus 8,35 g (24 mmol) der Verbindung aus Beispiel 44 5,6 g Rohprodukt.
- Rohausbeute:: 77,5% der Theorie
- ¹H-NMR (CDCl₃): δ =: 1,2 (m, 9H, CH₃CH₂, CH₃CHCH₃); 2,6 (s, 3H, CH₃); 4,1 (m, CHCH₃, CH₂O); 4,5, 4,6 (2d, 1H, FC₆H₄CH); 5,3 (s, 1H, NH); 6,9 - 7,4 (m, >5H, Aromaten-H) ppm.

### Beispiel 46

4-(4-Fluorphenyl)-2-isopropyl-6-methyl-pyridin-3-carbon-säureethylester 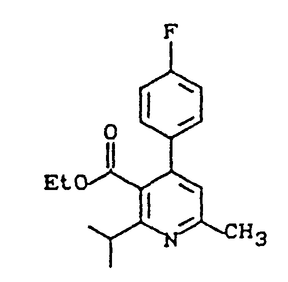 Analog Beispiel 37 erhält man aus 5,5 g (18,2 mmol) der Verbindung des Beispiels 45 nach Chromatographie über Kieselgel (Dichlormethan) 2,9 g rotes Öl.
- Ausbeute:: 53% der Theorie
- ¹H-NMR (CDCl₃): δ =: 1,05 (tr, 3H, CH₃CH₂); 1,35 (d, 6H, CH₃CH); 2,6 (s, 3H, CH₃); 3,15 (sept., 1H, CH); 4,1 (q, 2H, CH₂); 6,95 (s, 1H, Pyridin-H); 7,1 - 7,4 (m, 4H, Aromaten-H) ppm.

### Beispiel 47

4-(4-Fluorphenyl)-3-hydroxymethyl-2-isopropyl-6-methyl-pyridin 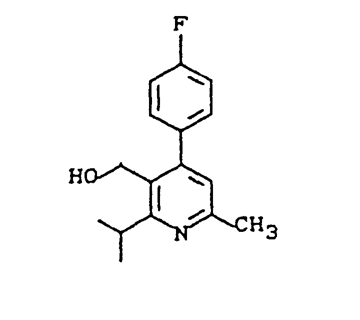 Analog Beispiel 29 erhält man aus 2,8 g (9,3 mmol) der Verbindung des Beispiels 46 2,19 g Produkt.
- Ausbeute:: 91% der Theorie
- ¹H-NMR (CDCl₃): δ =: 1,3 (d, 6H, CH₃CH); 1,5 (br, 1H, OH); 2,5 (s, 3H, CH₃); 3,5 (sept., 1H, CH); 4,6 (s, 2H, CH₂); 6,9 (s, 1H, Pyridin-H); 7,1 - 7,5 (m, 4H, Aromaten-H) ppm.

### Beispiel 48

4-(4-Fluorphenyl)-2-isopropyl-6-methyl-pyridin-3-carbaldehyd 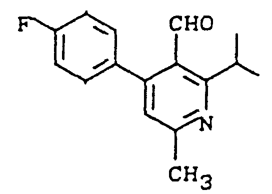 Analog Beispiel 30 erhält man aus 2,0 g (7,7 mmol) der Verbindung des Beispiels 47 0,56 g Produkt.
- Ausbeute:: 28,3% der Theorie
- ¹H-NMR (CDCl₃); δ =: 1,3 (d, 6H, CH₃CH); 2,6 (s, 3H, CH₃); 3,6 (sept, 1H, CH); /,0 (s, 1H, Pyridin-H); 7,1 - 7,4 (m, 4H, Aromaten-H); 10,0 (s, 1H, CHO) ppm.

### Beispiel 49

(E)-3-[4-(4-Fluorphenyl)-2-isopropyl-6-methyl-pyrid-3-yl]-prop-2-enal 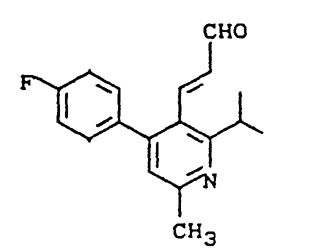 Analog Beispiel 31 erhält man aus 0,51 g (1,99 mmol) der Verbindung des Beispiels 48 0,48 g.
- Ausbeute:: 85,5% der Theorie
- ¹H-NMR (CDCl₃): δ =: 1,2 (d, 6H, CH₃CH); 2,5 (s, 3H, CH₃); 3,3 (sept, 1H, CH); 6,0 (dd, 1H, CHCHO); 6,9 (s, 1H, Pyridin-H); 7,0 - 7,3 (m, 4H, Aromaten-H); 7,5 (d, 1H,CH); 9,5 (d, 1H, CHO) ppm.

### Beispiel 50

Methyl-(E)-7-[4-(4-fluorphenyl)-2-isopropyl-6-methyl-pyrid-3-yl]-5-hydroxy-3-oxo-hept-6-enoat 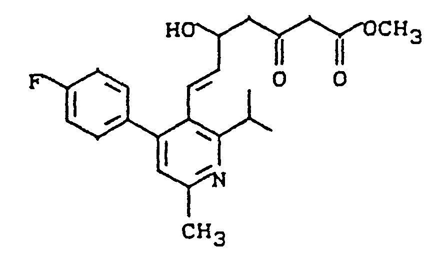 Analog Beispiel 32 erhält man aus 0,41 g (1,44 mmol) der Verbindung des Beispiels 49 0,22 g.
- Ausbeute:: 38,2% der Theorie
- ¹H-NMR (CDCl₃): δ =: 1,3 (d, 6H, CH₃CH); 2,5 (s, 3H, CH₃); 3,3 (sept, 1H, CH); 3,5 (s, 2H, CH₂); 3,25 (s, 3H, OCH₃); 4,6 (m, 1H; CHOH); 5,3 (dd, 1H, CH); 6,6 (d, 1H, CH); 6,9 (s, 1H, Pyridin-H); 7,0 - 7,3 (m, 4H, Aromaten-H) ppm.

### Beispiel 51

Methyl-erythro-(E)-7-[4-(4-fluorphenyl)-2-isopropyl-6-methyl-pyrid-3-yl]-3,5-dihydroxy-hept-6-enoat 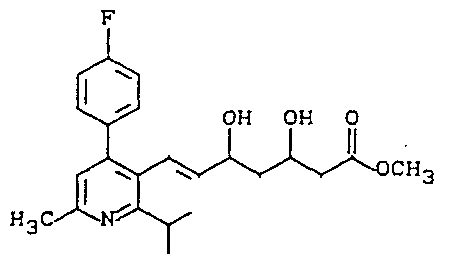 1,2 g (3,01 mmol) der Verbindung aus Beispiel 50 werden analog Beispiel 33 umgesetzt.
- Ausbeute:: 320 mg (26,6% der Theorie)
- ¹H-NMR (CDCl₃): δ =: 1,28 (d, 6H); 1,40 (m, 2H); 2,45 (m, 2H); 2,55 (s, 3H); 3,35 (m, 1H); 3,72 (s, 3H); 4,15 (m, 1H); 4,39 (m, 1H); 5,30 (dd, 1H); 6,55 (d, 1H); 6,88 (s, 1H); 7,0 - 7,30 (m, 4H) ppm.

### Beispiel 52

1,4-Dihydro-2,6-dimethyl-4-(4-fluorphenyl)-5-phenyl-pyridin-3-carbonsäuremethylester 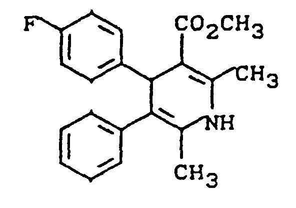 In 150 ml Ethanol werden 24,0 g (0,1 mol) 1-(4-Fluorphe-nyl)-2-phenyl-buten-3-on, 23 g (0,2 mol) 3-Amino-croton-säuremethylester und 6 ml (0,1 mol) Eisessig über Nacht unter Rückfluß erhitzt, nach Zugabe von 11,5 g (0,1 mol) 3-Aminocrotonsäuremethylester und 3 ml Eisessig 18 h unter Rückfluß erhitzt und nach wiederholter Zugabe von 11,5 g (0,1 mol) 3-Aminocrotonsäuremethylester und 3 ml Eisessig nochmals 18 h unter Rückfluß erhitzt. Das Lösemittel wird im Vakuum entfernt, der Rückstand mit 80 ml Methanol versetzt, Ungelöstes abgesaugt, die methanolische Lösung in Vakuum eingeengt, aus dem Rückstand bei 73°C - 90°C/18 mbar und anschließend bei 60°C bis 70°C/0,2 mbar überschüssigen Aminocrotonester abdestilliert. Man erhält 37,5 g Rohprodukt als glasharte Schmelze.
- Rohausbeute:: > 100% der Theorie
- ¹H-NMR (CDCl₃): δ =: 1,85 (s, 3H, CH₃); 2,85 (s, 3H, CH₃); 3,6 (s, 3H, CH₃); 4,65, 5,4 (2br, s, 1H, CH); 6,7 - 7,4 (m, 9H, Aromaten-H) ppm.

### Beispiel 53

2,6-Dimethyl-4-(4-fluorphenyl)-5-phenyl-pyridin-3-carbonsäuremethylester 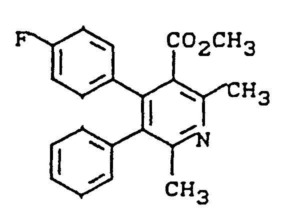 Analog Beispiel 37 erhält man aus 37,3 g (0,1 mol, roh) der Verbindung des Beispiels 52 20,4 g Feststoff.
- Ausbeute:: 49,4% der Theorie
- ¹H-NMR (CDCl₃): δ =: 2,45 (s, 3H,CH₃); 2,6 (s, 3H, CH₃); 3,5 (s, 3H, CH₃); 6,7 - 7,4 (m, 9H, Aromaten-H) ppm.

### Beispiel 54

2,6-Dimethyl-4-(4-fluorphenyl)-3-hydroxymethyl-5-phenyl-pyridin 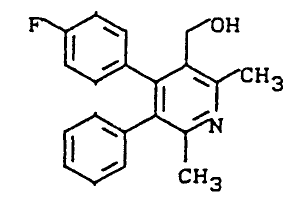 Analog Beispiel 29 erhält man aus 20,2 g (60 mmol) der Verbindung des Beispiels 53, 12,4 g Rohprodukt.
- Ausbeute:: 67% der Theorie)
- ¹H-NMR (CDCl₃): δ =: 2,0 (br, s, 1H, OH); 2,3 (s, 3H, CH₃); 2,75 (s, 3H, CH₃); 4,45 (s, 2H, CH₂); 6,8 - 7,3 (m, 9H, Aromaten-H) ppm.

### Beispiel 55

2,6-Dimethyl-4-(4-fluorphenyl)-5-phenyl-pyridin-3-carbaldehyd 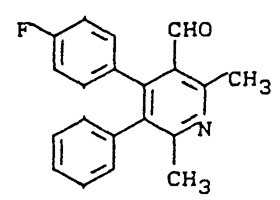 Analog Beispiel 30 erhält man aus 6,0 g (19,5 mmol) der Verbindung des Beispiels 54 nach Chromatographie über Kieselgel (Dichlormethan) 3,8 g Feststoff.
- Ausbeute:: 64% der Theorie
- ¹H-NMR (CDCl₃): δ =: 2,4 (s, 3H,CH₃); 2,9 (s, 3H, CH₃); 6,8 - 7,3 (m, 9H, Aromaten-H) 9,8 (s, 1H, CHO) ppm.

### Beispiel 56

(E)-3-[2,6-Dimethyl-4-(4-fluorphenyl)-5-phenyl-pyrid-3-yl]-prop-2-enal 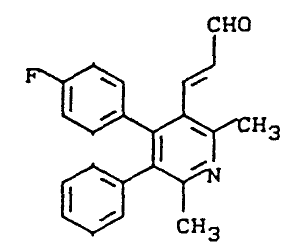 Analog Beispiel 31 erhält man aus 3,1 g (10 mmol) der Verbindung des Beispiels 55 2,0 g Rohprodukt.
- Ausbeute:: 60% der Theorie
- ¹H-NMR (CDCl₃): δ =: 2,4 (s, 3H, CH₃); 2,75 (s, 3H, CH₃); 6,15 (dd, 1H, CHCHO); 6,85 (d, 1H, CH); 6,9 - 7,3 (m, 9H, Aromaten-H); 9,4 (d, 1H, CHO) ppm.

### Beispiel 57

Methyl-(E)-7-[2,6-dimethyl-4-(4-fluorphenyl)-5-phenyl-pyrid-3-yl]-5-hydroxy-3-oxo-hept-6-enoat 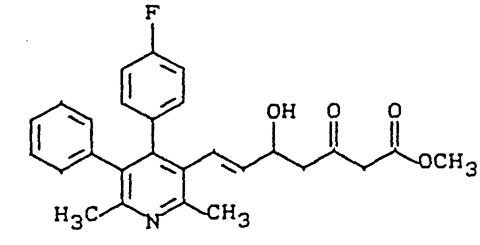 Analog Beispiel 32 erhält man aus 2,0 g (6 mmol) der Verbindung des Beispiels 56 2,4 g Rohprodukt.
- Rohausbeute:: 89% der Theorie
- ¹H-NMR (CDCl₃): δ =: 2,3 (s, 3H, CH₃); 2,6 (s, 3H, CH₃); 2,7 (m, 2H, CH₂); 3,45 (d, 2H, CH₂); 3,75 (2s, 3H, OCH₃); 4,5 (m, 1H, CH); 5,4 (dd, 1H, CHCHO); 6,3 (2d, 1H, CH); 6,7 - 7,3 (m, 9H, Aromaten-H) ppm.

### Beispiel 58

Methyl-erythro-(E)-7-[2,6-dimethyl-4-(4-fluorphenyl)-5-phenyl-pyrid-3-yl]-3,5-dihydroxy-hept-6-enoat 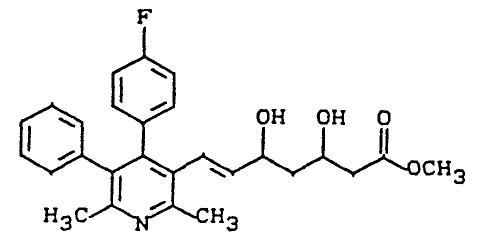 Analog Beispiel 33 erhält man aus 2,4 g (5,3 mmol) der Verbindung des Beispiels 57 nach Chromatographie über Kieselgel (Essigester) 550 mg Produkt.
- Ausbeute:: 23,1% der Theorie
- ¹H-NMR (CDCl₃): δ =: 1,7 (br, s, 2H, OH); 2,3 (s, 3H, CH₃); 2,4 - 2,6 (m, 2H, CH₂); 2,65 (s, 3H, CH₃); 3,7 (s, 3H, OCH₃); 4,1 (m, 1H, CHOH); 4,45 (m, 1H, CHOH); 5,4 (dd, 1H, CHCHOH); 6,3 (d, 1H, CH); 6,7 - 7,3 (m 9H, Aromaten-H) ppm.

### Beispiel 59

2,6-Diisopropyl-4-(4-fluorphenyl)-5-methoxymethyl-pyridin-3-carbonsäureethylester 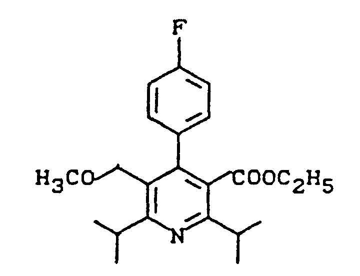 Zu 3 g (8,4 mmol) der Verbindung aus Beispiel 4 in 100 ml trockenem Tetrahydrofuran gibt man unter Stickstoffatmosphäre 0,57 ml (9,2 mmol) Methyljodid und bei -50°C 327 mg (10,9 mmol) 80%iges Natriumhydrid. Man rührt 2 Stunden nach und läßt dabei die Temperatur bis 25°C ansteigen. Die Mischung wird vorsichtig mit Wasser versetzt, mehrmals mit Ether extrahiert, die organische Phase über Magnesiumsulfat getrocknet und im Vakuum eingeengt.
- Ausbeute:: 2,9 g (92,7% der Theorie)
- ¹H-NMR (CDCl₃): δ =: 0,97 (t, 3H); 1,3 (m, 12H); 3,05 (m, 1H); 3,21 (s, 3H); 3,38 (m, 1H); 3,96 (q, 2H); 4,1 (s, 2H); 7,08 (m, 2H); 7,25 (m, 2H) ppm.

### Beispiel 60

2,6-Diisopropyl-4-(4-fluorphenyl)-3-hydroxymethyl-5-methoxymethyl-pyridin 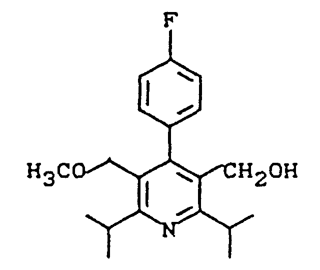 Unter Stickstoffatmosphäre werden zu 0,5 g (13,2 mmol) Lithiumaluminiumhydrid in 20 ml absolutem Tetrahydrofuran bei 60°C 2,85 g (7,6 mmol) der Verbindung aus Beispiel 59 gelöst in 30 ml absolutem Tetrahydrofuran zugetropft. Man erhitzt 1 Stunde zum Rückfluß, kühlt anschließend auf 0°C ab und tropft vorsichtig 1,5 ml Wasser und 0,3 ml 15%ige Kaliumhydroxyd-Lösung zu. Es wird vom ausgefallenen Niederschlag abgesaugt und dieser mehrmals mit Ether ausgekocht. Die vereinigten Phasen werden über Magnesiumsulfat getrocknet und im Vakuum eingeengt. Der Rückstand wird an einer Säule (Kieselgel 70-230 mesh, mit Essigester/Petrolether 1:9) chromatographiert.
- Ausbeute:: 1,9 g (74,8% der Theorie)
- ¹H-NMR (CDCl₃): δ =: 1,3 (m, 12H); 3,17 (s, 3H); 3,35 (m, 1H); 3,43 (m, 1H); 4,02 (s, 2H); 4,35 (d, 2H); 7,12 (m, 2H); 7,25 (m, 2H) ppm.

### Beispiel 61

Methyl-erythro-(E)-7-[2,6-diisopropyl-4-(4-fluorphenyl)-5-methoxymethyl-pyrid-3-yl]-3,5-dihydroxy-hept-6-enoat 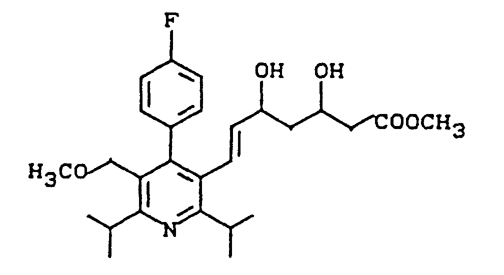 Aus der Verbindung aus Beispiel 60 wurde, in Analogie zu den Reaktionen aus den Beispielen 7, 8, 9, 10, das Beispiel 61 hergestellt.
- ¹H-NMR (CDCl₃): δ =: 1,23 (m, 6H); 1,32 (d, 6H); 1,40 (m, 2H); 2,43 (m, 2H); 3,18 (s, 3H); 3,32 (m, 2H); 3,73 (s, 3H); 4,05 (s, 2H); 4,08 (m, 1H); 4,29 (m, 1H); 5,23 (dd, 1H); 6,31 (d, 1H); 7,0-7,20 (m, 4H) ppm.

### Beispiel 62

Methyl-erythro-(E)-7-[5-tert.butyldimethylsilyloxymethyl-4-(4-fluorphenyl)-2-isopropyl-6-methyl-pyrid-3-yl]-3,5-dihydroxy-hept-6-enoat 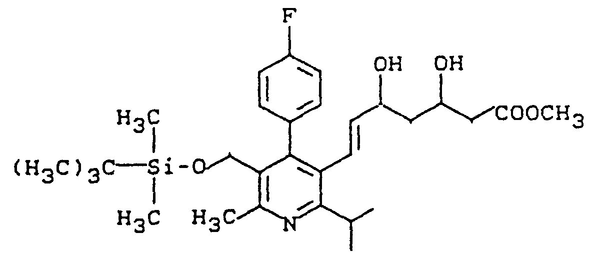 Aus der Verbindung aus Beispiel 20 wurde in Analogie zu den Reaktionen aus den Beispielen 5, 6, 7, 8, 9, 10, 11, 12 die obige Verbindung synthetisiert.
- ¹H-NMR (CDCl₃): δ =: 0,0 (s, 6H); 0,82 (s, 9H); 1,22 (d, 6H); 1,40 (m, 2H); 2,42 (m, 2H); 2,65 (s, 3H); 3,28 (m, 1h); 3,70 (s, 3H); 4,08 (m, 1H); 4,26 (m, 1H); 4,29 (s, 2H); 5,22 (dd, 1H); 6,30 (d, 1H); 7,0 - 7,20 (m, 4H) ppm.

### Beispiel 63

Methyl-erythro-(E)-7-[4-(4-fluorphenyl)-5-hydroxymethyl-1-isopropyl-6-methyl-pyrid-3-yl]-3,5-dihydröxy-hept-6-enoat 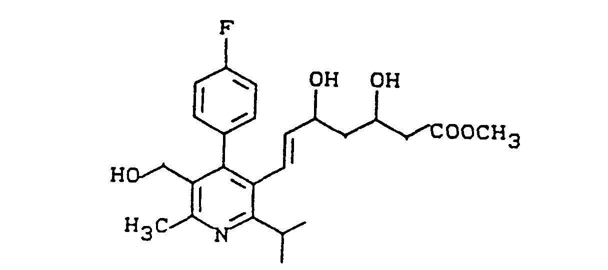 Aus der Verbindung aus Beispiel 20 wurde in Analogie zu den Reaktionen aus den Beispielen 5, 6, 7, 8, 9, 10, 11, 12 die obige Verbindung synthetisiert.
- ¹H-NMR (CDCl₃): δ =: 1,22 (d, 6H); 1,30 - 1,60 (m, 2H); 2,43 (m, 2H); 2,69 (s, 3H) ; 3,32 (m, 1H); 3,72 (s, 3H); 4,07 (m, 1H); 4,30 (m, 1H); 4,41 (s, 2H); 5,25 (dd, 1H); 6,31 (d, 1H); 7,11 (m, 4H) ppm.

### Beispiel 64

Methyl-erythro-(E)-7-[3-benzyloxymethyl-4-(4-fluorphenyl)-1-isopropyl-6-methyl-pyrid-5-yl]-3,5-dihydroxy-hept-6-enoat 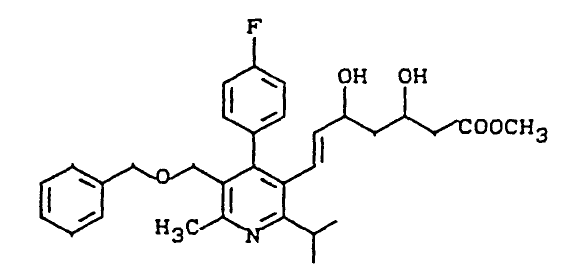 Aus der Verbindung aus Beispiel 20 wurde in Analogie zu den Reaktionen aus den Beispielen 5, 6, 7, 8, 9, 10, 11, 12 die obige Verbindung synthetisiert.
- ¹H-NMR (CDCl₃): δ =: 1,23 (d, 6H); 1,40 (m, 2H); 2,42 (m, 2H); 2,63 (s, 3H); 3,30 (m, 1H); 3,72 (s, 3H); 4,10 (m, 1H); 4,15 (s, 2H); 4,32 (m, 1H); 4,38 (s, 2H); 5,20 (dd, 1H); 6,31 (d, 1H); 7,0-7,40 (m, 9H) ppm.

### Beispiel 65

3-(tert.Butyldimethylsilyloxymethyl)-2,6-diisopropyl-5-(2,2-dimethyl-butyryloxymethyl)-4-(4-fluorphenyl)-pyridin 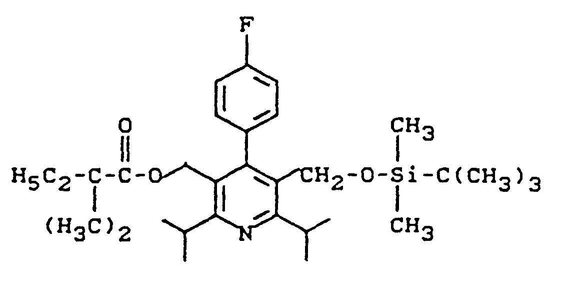 Zu 1,29 g (3 mmol) der Verbindung aus Beispiel 6 in 50 ml absolutem Tetrahydrofuran werden bei 0°C nacheinander 865 mg (3,3 mmol) Triphenylphosphin, 0,41 ml (3,3 mmol) 2,2-Dimethylbuttersäure und 0,52 ml (3,3 mmpl) Azodicarbonsäurediethylester gegeben und über Nacht bei Raumtemperatur gerührt. Die Mischung wird im Vakuum eingeengt und der Rückstand an einer Säule (Kieselgel 70-230 mesh, mit Essigester/Petrolether 1:9) chromatographiert.
- Ausbeute:: 1,32 g (87,4% der Theorie)
- ¹H-NMR (CDCl₃): δ =: 0,01 (s, 6H); 0,86 (t, 3H); 0,91 (s, 9H); 1,2 (s, 6H); 1,39 (m, 12H); 1,6 (q, 2H); 3,24 (m, 1H); 3,48 (m, 1H); 4,38 (s, 2H); 4,79 (s, 2H); 7,05-7,35 (m, 4H) ppm.

### Beispiel 66

2,6-Diisopropyl-3-(2,2-dimethyl-butyryloxymethyl)-4-(4-fluorphenyl)-5-hydroxymethyl-pyridin 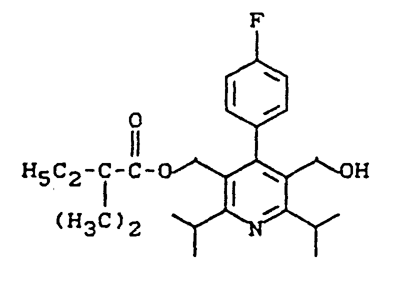 1,3 g (2,6 mmol) der Verbindung aus Beispiel 65 gelöst in 20 ml absolutem Tetrahydrofuran werden mit 2,6 ml (2,6 mmol) einer 1 molaren Tetrabutylammoniumfluorid-Lösung in Tetrahydrofuran versetzt und 1 Stunde bei Raumtemperatur gerührt. Die Mischung wird mit gesättigter Natriumhydrogencarbonat-Lösung versetzt und mehrmals mit Dichlormethan extrahiert. Die organische Phase wird über Magnesiumsulfat getrocknet, im Vakuum eingeengt und der Rückstand an einer Säule (Kieselgel 70-230 mesh, mit Laufmittel Essigester/Petrolether 1:9) chromatographiert.
- Ausbeute:: 1 g (95,2% der Theorie)
- ¹H-NMR (CDCl₃): δ =: 0,71 (t, 3H); 1,02 (s, 6H); 1,21 (d, 6H); 1,25 (d, 6H); 1,43 (q, 2H); 3,09 (m, 1H); 3,38 (m, 1H); 4,3 (d, 2H); 4,61 (s, 2H); 6,95 - 7,18 (m, 4H) ppm.

### Beispiel 67

2,6-Diisopropyl-5-(2,2-dimethyl-butyryloxymethyl)-4-(4-fluorphenyl)-pyridin-3-carbaldehyd 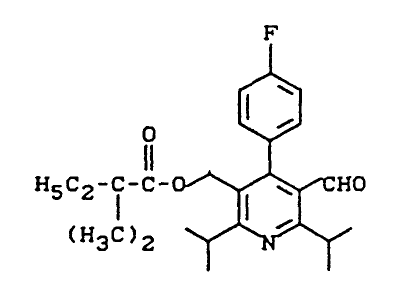 1 g (2,5 mmol) der Verbindung aus Beispiel 66 wird analog Beispiel 7 umgesetzt.
- Ausbeute:: 890 mg (86,4% der Theorie)
- ¹H-NMR (CDCl₃): δ =: 0,82 (t, 3H); 1,25 (s, 6H); 1,32 (m, 12H); 1,55 (q, 2H); 3,26 (m, 1H); 3,88 (m, 114); 4,77 (s, 2H); 7,09-7,27 (m, 4H); 9,77 (s, 1H) ppm.

### Beispiel 68

(E)-3-[2,6-Diisopropyl-5-(2,2-dimethyl-butyryloxymethyl)-4-(4-fluorphenyl)-pyrid-3-yl]-prop-2-enal 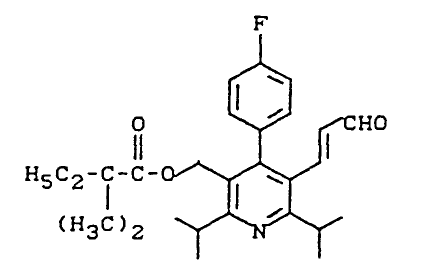 860 mg (2,1 mol) der Verbindung aus Beispiel 67 werden analog zu Beispiel 8 umgesetzt.
- Ausbeute:: 420 mg (45,6% der Theorie)
- ¹H-NMR (CDCl₃): δ =: 0,82 (t, 3H); 1,14 (s, 6H); 1,31 (m, 12H); 1,53 (q, 2H); 3,22 (m, 1H); 3,33 (m, 1H); 4,75 (6, 2H); 5,99 (dd, 1H); 7,05 - 7,29 (m, 5H); 9,4 (d, 1H) ppm.

### Beispiel 69

Methyl-(E)-7-[2,6-diisopropyl-5-(2,2-dimethyl-butyryloxymethyl)-4-(4-fluorphenyl)-pyrid-3-yl]-5-hydroxy-3-oxo-hept-6-enoat 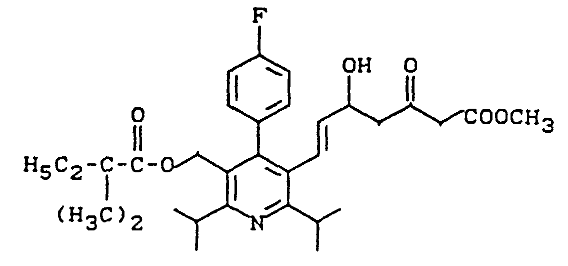 Unter Stickstoff tropft man zu einer Suspension von 54,6 mg (1,82 mmol) 80%igem Natriumhydrid in 5 ml absolutem Tetrahydrofuran bei -5°C 0,15 ml (1,4 mmol) Acetessigsäuremethylester in 2 ml absolutem Tetrahydrofuran. Nach 15 Minuten werden bei derselben Temperatur 0,89 ml (1,4 mmol) 15%iges Butyllithium in n-Hexan zugetropft und nach weiteren 15 Minuten 408 mg (1,8 mmol) trockenes Zinkbromid in 5 ml absolutem Tetrahydrofuran hinzugefügt. Man läßt noch 15 Minuten bei -5°C nachrühren, tropft 400 mg (0,91 mmol) der Verbindung aus Beispiel 68 gelöst in 10 ml trockenem Tetrahydrofuran zu und rührt über Nacht. Die Mischung wird mit gesättigter Ammoniumchlorid-Lösung versetzt und mehrmals mit Ether extrahiert. Die organische Phase wird über Magensiumsulfat getrocknet, im Vakuum eingeengt und der Rückstand an einer Säule (Kieselgel 70-230 mesh, mit Essigester/Petrolether 3:7) chromatographiert.
- Ausbeute:: 200 mg (38,5% der Theorie)
- ¹H-NMR (CDCl₃): δ =: 0,8 (t, 3H); 1,12 (s, 6H); 1,27 (d, 6H); 1,32 (d, 6H); 1,53 (q, 2H); 2,45 (m, 2H); 3,18 (m, 1H); 3,27 (m, 1H); 3,43 (s, 2H); 3,74 (s, 3H), 4,50 (m, 1H); 4,73 (s, 2H); 5,28 (dd, 1H); 6,38 (d, 1H); 7,0 - 7,10 (m, 4H) ppm.

### Beispiel 70

Methyl-erythro-(E)-7-[2,6-diisopropyl-5-(2,2-dimethylbutyryloxymethyl)-4-(4-fluorphenyl)-pyrid-3-yl]-3,5-dihydroxy-hept-6-enoat 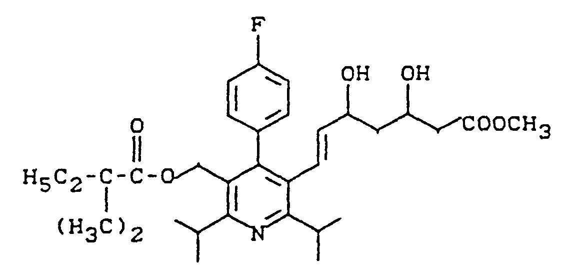 180 mg (0,32 mmol) der Verbindung aus Beispiel 69 werden analog Beispiel 10 umgesetzt.
- Ausbeute:: 138 mg (77,5% der Theorie)
- ¹H-NMR (CDCl₃); δ =: 0,81 (t, 3H); 1,12 (s, 6H); 1,28 (m, 6H); 1,40 (m, 2H); 1,53 (q, 2H); 2,43 (m, 2H); 3,17 (m, 1H); 3,32 (m, 1H); 3,73 (s, 3H); 4,08 (m, 1H); 4,31 (m, 1H); 4,75 (s. 2H); 5,28 (dd, 1H); 6,32 (d, 1H); 7,0 - 7,1 (m, 4H) ppm.

### Beispiel 71

Methyl-erythro-(E)-7-[5-benzyloxymethyl-2,6-diisopropyl-4-(4-fluorphenyl)-pyrid-3-yl]-3,5-dihydroxy-hept-6-enoat 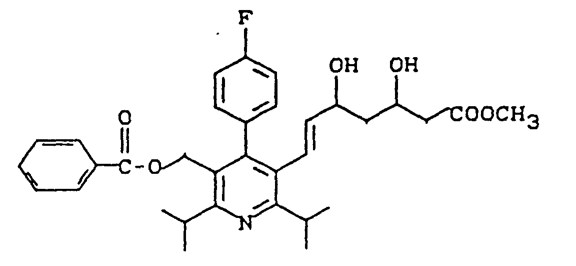 Aus der Verbindung aus Beispiel 6 wurde in Analogie zu den Reaktionen aus den Beispielen 65, 66, 67, 68, 69 und 70 die obige Verbindung synthetisiert.
- ¹H-NMR (CDCl₃): δ =: 1,31 (m, 6H); 1,40 (m, 2H); 2,43 (m, 2H); 3,32 (m, 2H); 3,73 (s, 3H); 4,07 (m, 1H); 4,32 (m, 1H); 5,07 (s, 2H); 5,30 (dd, 1H); 6,32 (d, 1H); 6,90 - 7,20 (m, 4H) ; 7,42 (m, 2H); 7,55 (m, 1H); 8,02 (m, 2H) ppm.

### Beispiel 72

Methyl-erythro-(E)-7-[3-acetoxymethyl-2,6-diisopropyl-4-(4-fluorphenyl)-pyrid-5-yl]-3,5-dihydroxy-hept-6-enoat 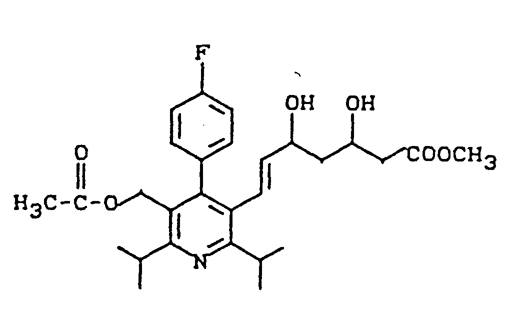 Aus der Verbindung aus Beispiel 6 wurde in Analogie zu den Reaktionen aus den Beispielen 65, 66, 67, 68, 69 und 70 die obige Verbindung synthetisiert.
- ¹H-NMR (CDCl₃): δ =: 1,25 (m, 12H); 1,40 (m, 2H); 2,02 (s, 3H); 2,43 (m, 3H); 3,20 (m, 1H); 3,32 (m, 1H); 3,72 (s, 3H); 4,07 (m, 1H); 4,29 (m, 1H); 4,81 (s, 2H); 5,28 (dd, 1H); 6,30 (d, 1H); 7,0-7,1 (m, 4H) ppm.

### Beispiel 73

(E/Z)-3-Carboxymethyl-4-(4-fluorphenyl)but-3-en-2-on 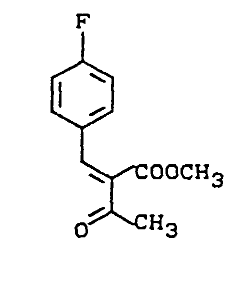 62 g (0,5 mol) 4-Fluorbenzaldehyd und 53,9 ml (0,5 mol) Acetessigsäuremethylester werden in 300 ml Isopropanol vorgelegt, mit einem Gemisch aus 2,81 ml (28 mmol) Piperidin und 1,66 ml (29 mmol) Essigsäure in 40 ml Isopropanol versetzt und 48 h bei Raumtemperatur gerührt. Die Mischung wird im Vakuum eingeengt und der Rückstand im Hochvakuum destilliert.
- Kp 0,5 mm:: 138°C
- Ausbeute:: 50,5 g (45,5% der Theorie)

### Beispiel 74

1,4-Dihydro-2,6-dimethyl-4-(4-fluorphenyl)-pyridin-3,5-dicarbonsäure-dimethylester 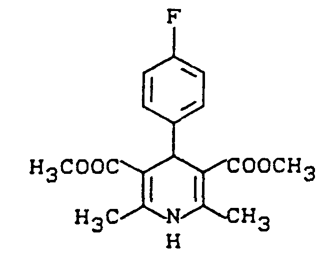 33,3 g (0,15 mol) der Verbindung aus Beispiel 73 werden mit 17,3 g (0,15 mol) 3-Aminocrotonsäuremethylester in 150 ml Ethanol 4 h unter Rückfluß gekocht. Die Mischung wird auf 0°C abgekühlt, der ausgefallene Niederschlag abgesaugt, mit wenig Petrolether nachgewaschen und im Exsikkator getrocknet.
- Ausbeute:: 32 g (66,8% der Theorie)
- ¹H-NMR (CDCl₃): δ =: 2,33 (s, 6H); 3,65 (s, 6H); 4,99 (s,1H); 5,77 (s, 1H); 6,89 (m, 2H); 7,22 (m, 2H) ppm.

### Beispiel 75

2,6-Dimethyl-4-(4-fluorphenyl)-pyridin-3,5-dicarbonsäure-dimethylester 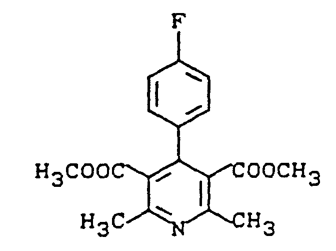 32 g (0,1 mol) der Verbindung aus Beispiel 74 werden analog Beispiel 3 umgesetzt.
- Ausbeute:: 27,2 g (87% der Theorie)
- ¹H-NMR (CDCl₃): δ =: 2,59 (s, 6H); 3,56 (s, 6H); 7,08 (m, 2H); 7,25 (m, 2H) ppm.

### Beispiel 76

2,6-Dimethyl-4-(4-fluorphenyl)-5-hydroxy-methyl-pyridin-3-carbonsäuremethylester 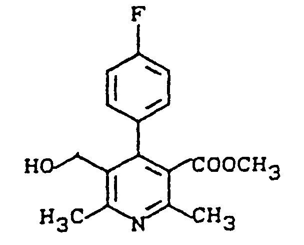 Unter Stickstoff gibt man zu einer Lösung von 14,9 g (47 mmol) der Verbindung aus Beispiel 75 in 300 ml trockenem Tetrahydrofuran bei -10°C bis -5°C 40,3 ml (141 mmol) einer 3,5 molaren Lösung von Natrium-bis-(2-methoxy-ethoxy)-dihydroaluminat in Toluol und rührt 30 Minuten bei Raumtemperatur. Nach Abkühlen auf 0°C tropft man vorsichtig 150 ml Wasser hinzu und extrahiert mehrmals mit Essigester. Die vereinigten organischen Phasen werden mit gesättigter Natriumchlorid-Lösung gewaschen, über Magnesiumsulfat getrocknet und im Vakuum eingeengt. Der Rückstand kristallisiert mit Ether/Petrolether. Nach Trocknen im Exsikkator erhält man 7,5 g Substanz (55,2% der Theorie)
- ¹H-NMR (CDCl₃): δ =: 2,52 (s, 3H); 2,7 (s, 3H); 3,52 (s, 3H); 4,45 (s, 2H); 7,05 - 7,3 (m, 4H) ppm.

### Beispiel 77

Methyl-erythro-(E)-7-[5-tert.butyldimethylsilyloxymethyl-2,6-dimethyl-4-(4-fluorphenyl)pyrid-3-yl]-3,5-dihydroxy-hept-6-enoat 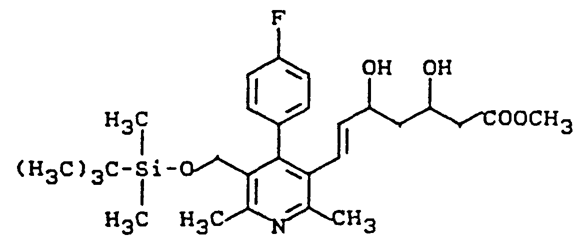 Aus der Verbindung aus Beispiel 76 wurde in Analogie zu den Reaktionen aus den Beispielen 5, 6, 7, 8, 9 und 10 das Beispiel 77 hergestellt.
- ¹H-NMR (CDCl₃): δ =: 0,01 (s, 6H); 0,92 (s, 9H); 1,40 (m, 2H); 2,49 (m, 2H); 2,62 (s, 3H); 2,71 (s, 3H); 3,80 (s, 3H); 4,17 (m, 1H); 4,36 (s, 2H); 4,38 (m, 1H); 5,42 (dd, 1H); 6,31 (d, 1H); 7,10-7,20 (m, 4H) ppm.

### Beispiel 78

Methyl-erythro-(E)-7-[2,6-dimethyl-4-(4-fluorphenyl)-5-hydroxymethyl-pyrid-3-yl]-3,5-dihydroxy-hept-6-enoat 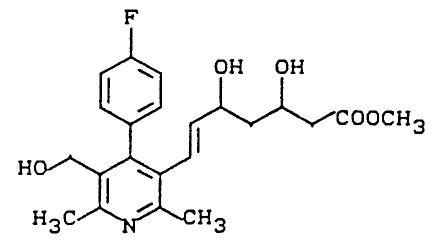 Aus der Verbindung aus Beispiel 77 wurde analog Beispiel 11 das Beispiel 78 hergestellt.

### Beispiel 79

(E/Z)-2-Carboxyethyl-1-cyclopropyl-3-(4-fluorphenyl)-2-prop-2-en-1-on 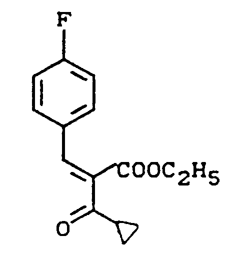 39 g (0,25 mol) Cyclopropylcarbonylessigsäureethylester und 31 g (0,25 mol) 4-Fluorbenzaldehyd werden in 150 ml trockenem Isopropanol vorgelegt und mit einem Gemisch aus 1,4 ml (14 mmol) Piperidin und 0,83 ml (14,5 mmol) Essigsäure in 20 ml Isopropanol versetzt. Man rührt 48 Stunden bie Raumtemperatur, engt im Vakuum ein und destilliert den Rückstand im Hochvakuum.
- Kp 0,5 mm:: 140°C
- Ausbeute:: 52,3 g (79,8% der Theorie)

### Beispiel 80

1,4-Dihydro-2-cyclopropyl-4-(4-fluorphenyl)-6-isopropyl-pyridin-3,5-dicarbonsäure-diethylester 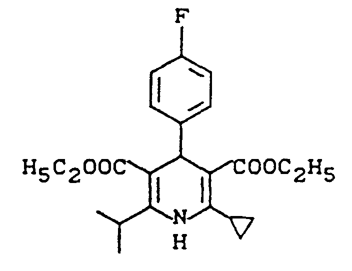 39,3 g (0,15 mol) der Verbindung aus Beispiel 79 und 23,6 g (0,15 mol) 3-Amino-4-methyl-pent-2-en-säure-ethylester werden in 150 ml Ethylenglykol über Nacht unter Rückfluß gekocht. Nach Abkühlen auf Raumtemperatur wird mehrmals mit Ether extrahiert, die vereinigten Etherphasen dreimal mit 10%iger Salzsäure, je einmal mit Wasser und gesättigter Natriumhydrogencarbonat-Lösung gewaschen, über Magnesiumsulfat getrocknet und im Vakuum eingeengt. Den Rückstand verrührt man mit Petrolether/ Ether, saugt ab und trocknet im Exsikkator.
- Ausbeute:: 22,8 g (37,8% der Theorie)
- ¹H-NMR (CDCl₃): δ =: 0,65 (m, 2H); 1,03 (m, 2H); 1,15 (m, 13H); 2,78 (m, 1H); 4,15 (m, 4H); 5,03 (s, 1H); 5,72 (s, 1H); 6,90 (m, 2H); 7,22 (m, 2H) ppm.

### Beispiel 81

2-Cyclopropyl-4-(4-fluorphenyl)-6-isopropyl-pyridin-3,5-dicarbonsäure-diethylester 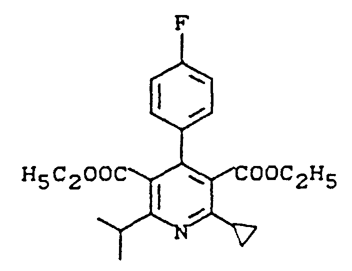 19,1 g (47 mmol) der Verbindung aus Beispiel 80 werden analog Beispiel 3 umgesetzt.
- Ausbeute:: 9,8 g (52,5% der Theorie)
- ¹H-NMR (CDCl₃): δ =: 0,97 (m, 8H); 1,25 (m, 8H); 2,09 (m, 1H); 3,06 (m, 1h); 4,02 (m, 4H); 7,06 (m, 2H); 7,26 (m, 2H) ppm.

### Beispiel 82

6-Cyclopropyl-4-(4-fluorphenyl)-5-hydroxymethyl-2-isopropyl-pyridin-3-carbonsäure-ethylester 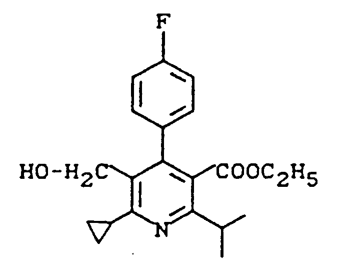 6 g (15 mmol) der Verbindung aus Beispiel 81 werden analog Beispiel 4 umgesetzt.
- Ausbeute:: 3,1 g (57,9% der Theorie)
- ¹H-NMR (CDCl₃): δ =: 0,97 (t, 3H); 1,03 (m, 2H); 1,22 (m, 8H), 2,38 (m, 1H); 3,03 (m, 1H); 4,0 (q, 2H); 4,58 (s, 2H); 7,1 (m, 2H); 7,25 (m, 2H) ppm.

### Beispiel 83

6-Cyclopropyl-4-(4-fluorphenyl)-2-isopropyl-5-methoxymethyl-pyridin-3-carbonsäure-ethylester 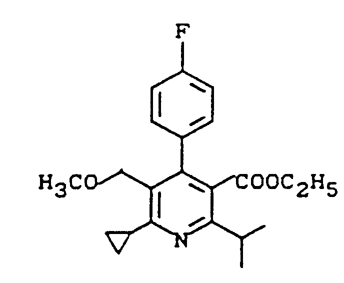 2,9 g (8 mmol) der Verbindung aus Beispiel 82 werden analog Beispiel 59 umgesetzt.
- Ausbeute:: 2 g (67,4% der Theorie)
- ¹H-NMR (CDCl₃): δ =: 0,93 (t, 3H); 0,98 (m, 2H); 1,22 (d, 6H); 1,24 (m, 2H); 2,32 (m, 1H); 3,03 (m, 1H); 3,28 (s, 3H); 3,97 (q, 2H); 4,25 (s, 2H); 7,08 (m, 2H); 7,25 (m, 2H) ppm.

### Beispiel 84

6-Cyclopropyl-4-(4-fluorphenyl)-3-hydroxy-methyl-2-isopropyl-5-methoxymethyl-pyridin 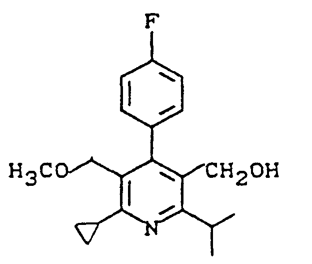 Unter Stickstoff gibt man zu einer Lösung von 1,9 g (5 mmol) der Verbindung aus Beispiel 83 in 50 ml trockenem Tetrahydrofuran 44,3 ml (15 mmol) einer 3,5 molaren Lösung von Natrium-bis-(2-methoxyethoxy)-dihydroaluminat in Toluol, rührt 2 Stunden bei Raumtemperatur und 1 Stunde unter Rückfluß. Nach Abkühlen auf 0°C tropft man vorsichtig 50 ml Wasser hinzu und extrahiert mehrmals mit Essigester. Die Essigesterphasen werden vereinigt, mit gesättigter Natriumchlorid-Lösung gewaschen, über Magnesiumsulfat getrocknet und im Vakuum eingeengt.
- Ausbeute:: 1,6 g (97% der Theorie)
- ¹H-NMR (CDCl₃): δ =: 0,89 (m, 2H), 1,17 (d, 6H); 1,19 (m, 2H); 2,20 (s, 1H); 3,13 (s, 3H); 3,32 (m, 1H); 4,07 (s, 2H); 4,26 (s, 2H); 7,05 (m, 2H); 7,16 (m, 2H) ppm.

### Beispiel 85

Methyl-erythro-(E)-7-[6-cyclopropyl-2-isopropyl-4-(4-fluorphenyl)-5-methoxymethyl-pyrid-3-yl]-3,5-dihydroxy-hept-6-enoat 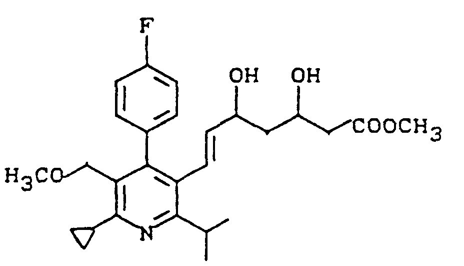 Aus der Verbindung aus Beispiel 84 wurde, in Analogie zu den Reaktionen aus den Beispielen 7, 8, 9 und 10 das Beispiel 85 hergestellt.
- ¹H-NMR (CDCl₃): δ =: 0,95 (m, 2H); 1,17 (m, 6H); 1,22 (m, 2H); 1,40 (m, 2H); 2,25 (m, 1H); 2,44 (m, 2H); 3,22 (s, 3H); 3,23 (m, 1H); 3,73 (6, 3H); 4,07 (m, 1H); 4,18 (s, 2H); 4,28 (m, 1H); 5,22 (dd, 1H); 6,30 (d, 1H); 7,0 - 7,20 (m, 4H) ppm.

### Beispiel 86

5-Chlormethyl-2,6-diisopropyl-4-(4-fluorphenyl)-pyridin-3-carbonsäure-ethylester 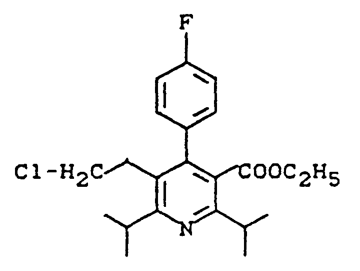 5 g (13,9 mmol) der Verbindung aus Beispiel 4 gelöst in 100 ml trockenem Tetrahydrofuran werden bei -5°C nacheinander mit 1,69 ml (20,9 mmol) Pyridin und 1,5 ml (20,9 mmol) Thionylchlorid versetzt und 15 Minuten bei derselben Temperatur gerührt. Die Mischung wird mit Essigester verdünnt, mehrmals mit gesättigter Natriumhydrogencarbonat-Lösung extrahiert, die organische Phase über Magnesiumsulfat getrocknet und im Vakuum eingeengt. Der Rückstand wird an einer Säule (Kieselgel 70 bis 230 mesh, in Petrolether/Essigester 95:5) chromatographiert.
- Ausbeute:: 3,2 g (65,2% der Theorie)
- ¹H-NMR (CDCl₃): δ =: 0,98 (t, 3H); 1,30 (d, 6H); 1,35 (d, 6H); 3,05 (m, 1H); 3,45 (m, 1H); 3,98 (q, 2H); 4,38 (s, 2H); 7,13 (m, 2H); 7,31 (m, 2H) ppm.

### Beispiel 87

2,6-Diisopropyl-4-(4-fluorphenyl)-5-phenoxymethyl-pyridin-3-carbonsäure-ethylester 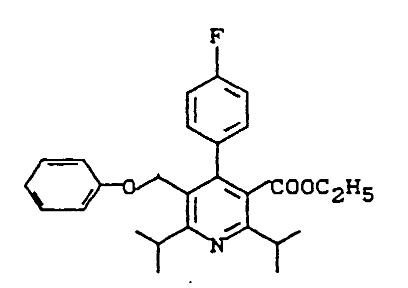 Zu einer Lösung von 2,11 g (18,2 mmol) Natriumphenolat in 50 ml absolutem Tetrahyrofuran tropft man bei 0°C 3,22 g (9,1 mmol) der Verbindung aus Beispiel 86 gelöst in 50 ml absolutem Tetrahyrofuran und kocht 4 Tage am Rückfluß. Nach Abkühlen auf Raumtemperatur wird mit 150 ml Wasser verdünnt und mehrmals mit Ether extrahiert. Die vereinigten organischen Phasen werden über Magnesiumsulfat getrocknet, im Vakuum eingeengt und der Rückstand an einer Säule (Kieselgel 70-230 mesh, mit Petrolether/Essigester 95:5) chromatographiert.
- Ausbeute:: 3,2 g (80,8% der Theorie)
- ¹H-NMR (CDCl₃): δ =: 0,97 (t, 3H); 1,3 (d, 6H); 1,33 (d, 6H); 3,1 (m, 1H); 3,32 (m, 1H); 4,0 (q, 2H); 4,7 (s, 2H); 6,78 - 7,31 (m, 9H) ppm.

### Beispiel 88

2,6-Diisopropyl-4-(4-fluorphenyl)-5-hydroxymethyl-3-phenoxymethyl-pyridin 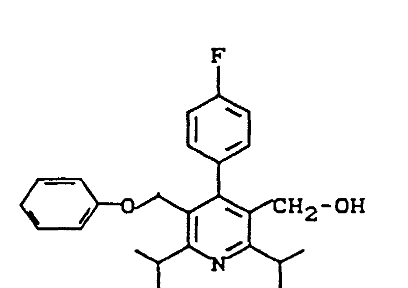 3,25 g (7,5 mmol) der Verbindung aus Beispiel 87 werden analog Beispiel 60 umgesetzt.
- Ausbeute:: 2,75 g (93,2% der Theorie)
- ¹H-NMR (CDCl₃): δ =: 1,35 (m, 12H); 3,30 (m, 1H); 3,48 (m, 1H); 4,42 (d, 2H); 4,62 (s, 2H); 6,75 - 7,30 (m, 9H) ppm.

### Beispiel 89

Methyl-erythro-(E)-7-[2,6-diisopropyl-4-(4-fluorphenyl)-5-phenoxymethyl-pyrid-3-yl]-3,5-dihydroxy-hept-6-enoat 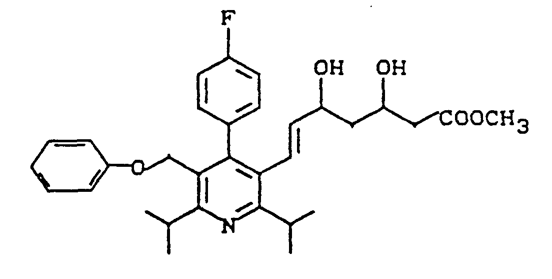 Aus der Verbindung aus Beispiel 88 wurde, in Analogie zu den Beispielen 7, 8, 9 und 10 das Beispiel 89 hergestellt.
- ¹H-NMR (CDCl₃): δ =: 1,28 (m, 6H); 1,31 (d, 6H) ; 1,43 (m, 2H); 2,41 (m, 2H); 3,30 (m, 2H); 3,71 (s, 3H); 4,08 (m, 1H); 4,30 (m, 1H); 4,65 (s, 2H); 5,28 (dd, 1H); 6,35 (d, 1H); 6,75 - 7,30 (m, 9H) ppm.

### Beispiel 90

2,6-Diisopropyl-4-(4-fluorphenyl)-5-(tetrahydropyran-2-yl-oxymethyl)-pyridin-3-carbonsäure-ethylester 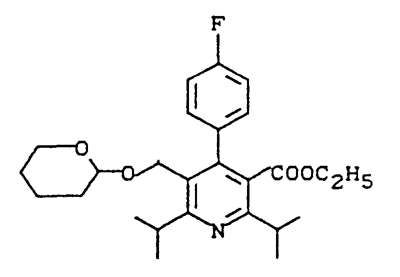 Zu einer Lösung von 5,36 g (14,9 mmol) der Verbindung aus Beispiel 4 in 100 ml trockenem Dichlormethan gibt man 1,88 g(22,4 mmol) Dihydropyran und 0,525 g (1,49 mmol) Pyridinium-p-toluol-sulfonat und kocht 48 Stunden am Rückfluß. Nach Abkühlen auf Raumtemperatur wird mit Dichlormethan verdünnt und mehrmals mit Wasser extrahiert. Die organische Phase wird über Magnesiumsulfat getrocknet, im Vakuum eingeengt und der Rückstand an einer Säule (Kieselgel 70-230 mesh, mit Dichlormethan) chromatographiert.
- Ausbeute:: 4,4 g (66,7% der Theorie)
- ¹H-NMR (CDCl₃): δ =: 0,98 (t, 3H); 1,31 (m, 12H); 1,40-1,80 (m, 6H); 3,05 (m, 1H); 3,43 (m, 2H); 3,61 (m, 1H); 3,98 (q, 2H); 4,05 (d, 1H); 4,45 (m, 1H); 4,55 (d, 1H); 7,05 (m, 2H); 7,25 (m, 2H) ppm.

### Beispiel 91

2,6-Diisopropyl-4-(4-fluorphenyl)-3-hydroxymethyl-5-(tetrahydropyran-2-yl-oxymethyl)-pyridin 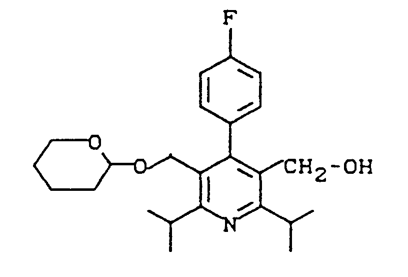 4,4 g (9,9 mmol) der Verbindung aus Beispiel 90 werden analog Beispiel 60 umgesetzt.
- Ausbeute:: 2,5 g (63,5% der Theorie)
- ¹H-NMR (CDCl₃): δ =: 1,32 (m, 12H); 1,40 - 1,80 (m, 6H); 3,40 (m, 3H); 3,57 (m, 114); 3,95 (d, 1H); 4,35 (m, 3H); 4,5 (d, 1H); 7,11 (m, 2H); 7,25 (m, 2H) ppm.

### Beispiel 92

Methyl-erythro-(E)-7-[2,6-diisopropyl-4-(4-fluorphenyl)-5-(tetrahydropyran-2-yl-oxymethyl)-pyrid-3-yl]-3,5-dihydroxy-hept-6-enoat 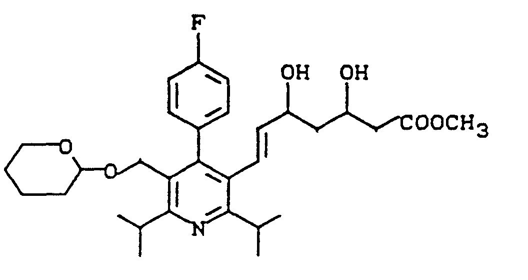 Aus der Verbindung aus Beispiel 91 wurde, in Analogie zu den Reaktionen aus den Beispielen 7,8, 9 und 10 das Beispiel 92 hergestellt.
- ¹H-NMR (CDCl₃): δ =: 1,20 - 1,80 (m, 20H); 2,43 (m, 2H); 3,32 (m, 1H); 3,41 (m, 2H); 3,58 (m, 1H); 3,72 (s, 3H); 3,98 (d, 1H); 4,08 (m, 1H); 4,29 (m, 1H); 4,43 (m, 1H); 4,54 (d, 1H); 5,28 (dd, 1H); 6,31 (d, 1H); 7,10 (m, 4H) ppm.

### Beispiel 93

3-Amino-4-methyl-pent-2-en-säure-2-(trimethylsilyl)-ethylester 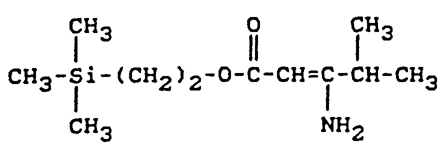 Zu 150 g (0,65 Mol) Isobutyryl-essigsäure-2-(trimethylsilyl)ethylester in 700 ml Toluol werden 3 g p-Toluolsulfonsäure gegeben und die Mischung bei Raumtemperatur mit Ammoniak-Gas gesättigt. Man läßt über Nacht stehen und kocht anschließend 8 Stunden am Rückfluß, wobei ständig Ammmoniak-Gas eingeleitet wird. Nach Abkühlen auf Raumtemperatur wird filtriert, die Toluol-Lösung mehrmals mit Wasser extrahiert, über Magnesiumsulfat getrocknet und im Vakuum eingeengt.
- Ausbeute:: 134 g (90% der Theorie)
- ¹H-NMR (CDCl₃): δ =: 0,21 (s, 9H); 1,15 (m, 2H); 1,30 (m, 6H); 2,48 (m, 1H); 4,31 (m, 2H); 4,71 (s, 1H) ppm.

### Beispiel 94

1,4-Dihydro-2,6-diisopropyl-4-(4-fluorphenyl)-pyridin-3,5-dicarbonsäure-5-ethyl-3-(2-trimethylsilyl)-ethyl-ester 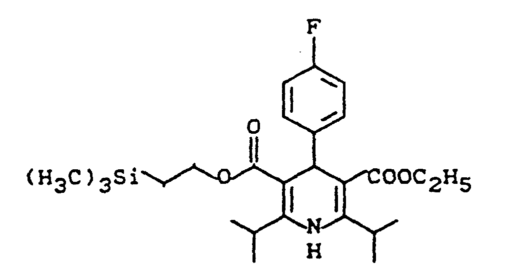 Zu einer Lösung von 45,8 g (0,2 mol) der Verbindung aus Beispiel 93 in 100 ml Ethylenglykol werden 52,7 g (0,2 mol) der Verbindung aus Beispiel 1 gegeben und über Nacht am Rückfluß gekocht. Die Mischung wird abgekühlt, mit 5 ml konz. Salzsäure versetzt und erneut 30 Minuten auf 100°C erwärmt. Nach Abkühlen auf Raumtemperatur wird im Vakuum eingeengt, der Rückstand in Essigester aufgenommen und mehrmals mit verdünnter Salzsäure extrahiert. Die organisch Phase wird anschließend je einmal mit gesättiger Natriumhydrogencarbonat- und Natriumchlorid-Lösung gewaschen, über Magnesiumsulfat getrocknet und im Vakuum eingengt, Der Rückstand wird an einer Säule (Kieselgel 70-230 mesh, mit Dichlormethan) chromatographiert.
- Ausbeute:: 39,2 g (41,3% der Theorie)
- ¹H-NMR (CDCL₃): δ =: 0,01 (s, 9H); 0,95 (m, 2H); 1,18 (m, 12H); 1,22 (t, 3H); 4,10 (m, 6H); 4,97 (s, 1H); 6,10 (s, 1H); 6,85 (m, 2H); 7,18 (m, 2H) ppm.

### Beispiel 95

1,4-Dihydro-2,6-diisopropyl-4-(4-fluorphenyl)-pyridin-3,5-dicarbonsäure-3-ethylester 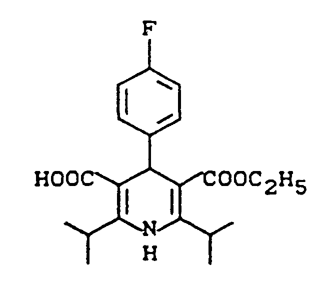 Zu einer Lösung von 38,9 g (81,8 mmol) der Verbindung aus Beipsiel 94 in 300 ml trockenem Tetrahydrofuran werden 83,3 ml (83,3 mmol) einer 1 molaren Lösung von Tetrabutylammoniumfluorid in Tetrahydrofuran gegeben und 48 Stunden bei Raumtemperatur gerührt. Nach Einengen im Vakuum wird der Rückstand in Ether aufgenommen, je dreimal mit verdünnter Natronlauge und verdünnter Schwefelsäure gewaschen, die organische Phase über Magnesiumsulfat getrocknet und erneut im Vakuum eingeengt.
- Ausbeute:: 29 g (94,5% der Theorie)
- ¹H-NMR (CDCl₃): δ =: 1,09 - 1,3 (m, 15H); 4,02 (q, 2H); 4,08 (m 2H); 4,18 (m, 1H); 4,89 (s, 1H); 7,03 (m, 2H); 7,12 (m, 2H) ppm.

### Beispiel 96

Methyl-erythro-(E)-7-[2,6-diisopropyl-4-(4-fluorphenyl)-pyrid-3-yl]-3,5-dihydroxy-hept-6-enoat 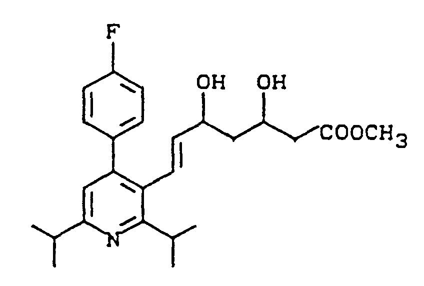 Aus der Verbindung aus Beispiel 95 wurde in Analogie zu den Reaktionen aus den Beispielen 27, 3, 29, 7, 8, 9 und 10 das Beispiel 96 hergestellt.
- ¹H-NMR (CDCl₃): δ =: 1,28 (m, 12H); 1,50 (m, 2H); 2,47 (m, 2H); 3,05 (m, 1H); 3,35 (m, 1H); 3,72 (s, 3H); 4,13 (m, 1H); 4,38 (m, 1H); 5,31 (dd, 1H); 6,55 (d, 1H); 6,85 (s, 1H); 7,05 (m, 2H); 7,25 (m, 2H) ppm.

### Beispiel 97

1-(4-Fluorphenyl)-4-methyl-2-phenyl-penten-3-on 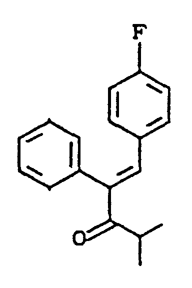 Zu 24,8 g (0,2 mol) 4-Fluorbenzaldehyd und 32,4 g (0,2 mol) Benzyl-isopropylketon in 150 ml Toluol werden 0,9 ml Piperidin gegeben und die Mischung über Nacht am Rückfluß gekocht. Nach Abkühlen auf Raumtemperatur wird mehrmals mit Wasser extrahiert, die organische Phase über Magnesiumsulfat getrocknet und im Vakuum eingeengt. Der Rückstand wird im Hochvakuum bei 0,1 mbar bis 150°C Badtemperatur andestilliert und man erhält 43,8 g Rohprodukt im Destillationsrückstand.
- Ausbeute:: 81% der Theorie

### Beispiel 98

1,4-Dihydro-2,6-diisopropyl-4-(4-fluorphenyl)-5-phenyl-pyridin-3-carbonsäure-ethylester 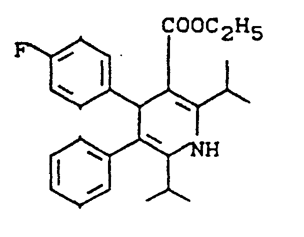 Zu 13,45 g (50 mmol) der Verbindung aus Beispiel 97 und 17,4 g (100 ml) 3-Amino-4-methyl-pent-2-en-säure-ethyl-ester in 80 ml Ethylenglykol werden 2,86 ml (50 mmol) Eisessig gegeben und die Mischung über Nacht am Rückfluß gekocht. Nach Abkühlen auf Raumtemperatur wird im Vakuum eingeengt, der Rückstand in Dichlormethan gelöst und mehrmals mit Wasser extrahiert. Die organische Phase wird über Magnesiumsulfat getrocknet, im Vakuum eingeengt und der Rückstand in Essigester aufgenommen. Nach Extrahieren mit 10%iger Salzsäure, Wasser und gesättigter Natriumhydrogencarbonat-Lösung wird die organische Phase erneut getrocknet, eingeengt und der Rückstand an einer Säule (Kieselgel 70-230 mesh, mit Essigester/Petrolether) chromatographiert.
- Ausbeute:: 2,3 g (11,3% der Theorie)
- ¹H-NMR (CDCl₃): δ =: 1,1 (m, 9H) ; 1,25 (m, 6H); 2,70 (m, 1H); 3,90 - 4,40 (m, 3H); 4,55 (s, 1H); 5,75 (s, 1H); 6,80 - 7,30 (m, 9H) ppm.

### Beispiel 99

Methyl-erythro-(E)-7-[2,6-diisopropyl-4-(4-fluorphenyl)-5-phenyl-pyrid-3-yl]-3,5-dihydroxy-hept-6-enoat 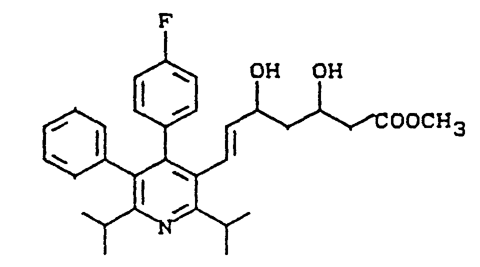 Aus der Verbindung aus Beispiel 98 wurde in Analogie zu den Reaktionen aus den Beispielen 3, 29, 7, 8, 9 und 10 das Beispiel 99 hergestellt.
- ¹H-NMR (CDCl₃); δ =: 1,18 (d, 6H); 1,32 (m, 6H); 1,42 (m, 2H); 2,40 (m, 2H); 2,70 (d, 1H); 2,88 (m, 1H); 3,38 (m, 1H); 3,48 (d, 1H); 3,71 (s, 3H); 4,05 (m, 114); 4,30 (m, 1H); 5,30 (dd, 1H); 6,39 (d, 1H); 6,70 - 7,20 (m, 9H) ppm.

### Beispiel 100

Methyl-erythro-(E)-7-[2,6-diisopropyl-4-(4-fluorphenyl)-5-carboxyethyl-pyrid-3-yl]-3,5-dihydroxy-hept-6-enoat 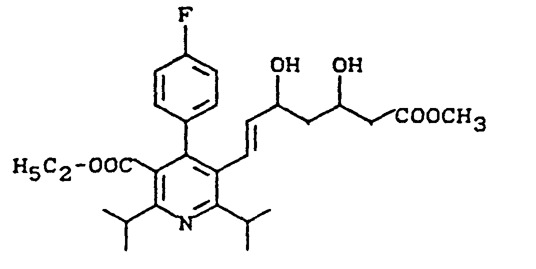 Aus der Verbindung aus Beispiel 4 wurde in Analogie zu den Reaktionen aus den Beispielen 7, 8, 69 und 10 das Beispiel 100 hergestellt.
- ¹H-NMR (CDCl₃): δ =: 0,98 (t, 3H); 1,25 (m, 6H); 1,32 (d, 6H); 1,45 (m, 2H); 2,42 (m, 2H); 3,05 (m, 1H); 3,32 (m, 1H); 3,72 (s, 3H); 3,98 (q, 2H); 4,10 (m, 1h); 4,32 (m, 1H); 5,29 (dd, 1H); 6,38 (d, 1H); 7,02 (m, 2H); 7,12 (m, 2H) ppm.

### Beispiel 101

1,4-Dihydro-2,6-diisopropyl-4-(4-fluorphenyl)-5-morpholinocarbonyl-pyridin-3-carbonsäureethylester 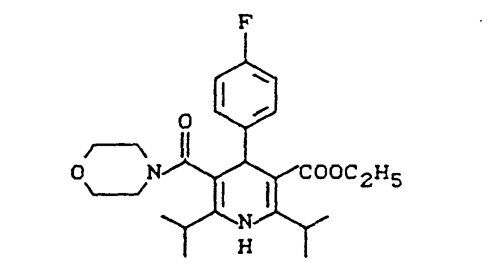 Zu 1,875 g (5 mmol) der Verbindung aus Beispiel 95 gelöst in 20 ml trockenem Tetrahydrofuran gibt man unter Stickstoffatmosphäre 1,05 g (6,5 mmol) N,N'-Carbonylimidazol und rührt 30 Minuten bei Raumtemperatur. Anschließend wird 30 Minuten am Rückfluß gekocht, mit einer Lösung von 0,87 ml (10 mmol) Morpholin in 5 ml trockenem Tetrahydrofuran versetzt und weitere 2 Stunden zum Sieden erhitzt. Nach Abkühlen auf Raumtemperatur wird im Vakuum eingeengt, der Rückstand in Dichlormethan aufgenommen und nacheinander mit 1 N Salzsäure, 1 N Natronlauge und Wasser gewaschen. Die organische Phase wird über Magnesiumsulfat getrocknet, im Vakuum eingeengt und der kristalline Rückstand im Exsikkator getrocknet.
- Ausbeute:: 1,96 g (88% der Theorie)
- ¹H-NMR (CDCl₃): δ =: 1,0 - 1,28 (m, 15H); 3,20 - 4,40 (komplexer Bereich, 12H); 4,70 (s, 1H); 5,50 (s, 1H); 6,90 (m, 2H); 7,20 (m, 2H) ppm.

### Beispiel 102

2,6-Diisopropyl-4-(4-fluorphenyl)-pyridin-3,5-dicarbonsäure-3-ethylester-5-morpholid 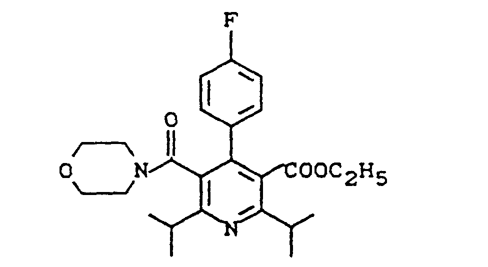 9 g (21,5 mmol) der Verbindung aus Beispiel 101 werden analog Beispiel 3 umgesetzt.
- Ausbeute:: 7,6 g (80% der Theorie)
- ¹H-NMR (CDCl₃): δ =: 0,95 (t, 3H); 1,25 (m, 6H); 1,35 (m, 6H); 2,70 - 3,80 (komplexer Bereich, 10H); 4,0 (m, 2H); 7,0 - 7,50 (m, 4H) ppm.

### Beispiel 103

Methyl-erythro-(E)-7-[2,6-diisopropyl-4-(4-fluorphenyl)-5-morpholinocarbonyl-pyrid-3-yl]-3,5-dihydroxy-hept-6-enoat 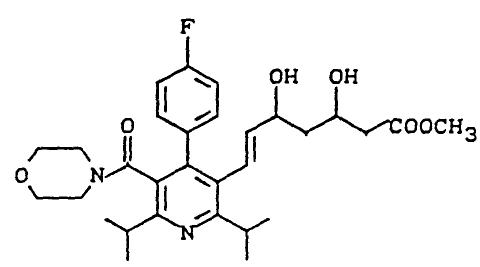 Aus der Verbindung aus Beispiel 102 wurde in Analogie zu den Reaktionen aus den Beispielen 29, 7, 8, 9 und 10 das Beispiel 103 hergestellt.
- ¹H-NMR (CDCl₃): δ =: 1,10 - 1,50 (komplexer Bereich, 14H); 2,40 (m, 2H); 2,80 - 3,65 (komplexer Bereich, 10H); 3,75 (s, 3H); 4,10 (m, 1H); 4,35 (m, 1H); 5,25 (m, 1H); 6,45 (dd, 1H); 6,95-7,50 (m, 4H) ppm.

### Bèispiel 104

2,6-Diisopropyl-4-(4-fluorphenyl)-3-hydroxymethyl-5-morpholinomethyl-pyridin 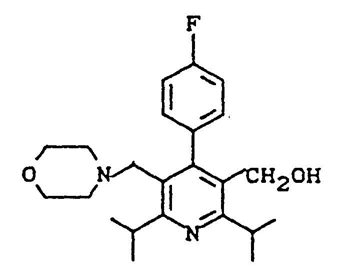 Zu 1,37 g (3,1 mmol) der Verbindung aus Beispiel 102 gelöst in 30 ml trockenem Toluol gibt man unter Stickstoffatmosphäre bei -78°C 20,6 ml (31 mmol) Diisobutylaluminiumhydrid (1 m in Toluol) und rührt 1 Stunde bei derselben Temperatur. Anschließend wird 48 Stunden bei Raumtemperatur gerührt, die Mischung unter Eiskühlung mit 20%iger Kaliumhydroxyd-Lösung hydrolysiert und mehrmals mit Toluol extrahiert. Die organische Phase wird über Magnesiumsulfat getrocknet, im Vakuum eingeengt und im Exsikkator getrocknet. Ausbeute: 1,04 g (87% der Theorie)
- ¹H-NMR (CDCl₃): δ =: 1,28 (d, 6H); 1,32 (d, 6H); 2,15 (m, 4H); 3,18 (s, 2H); 3,45 (m, 2H) ; 3,52 (m, 4H); 4,32 (d, 2H); 7,05-7,20 (m, 4H) ppm.

### Beispiel 105

Methyl-erythro-(E)-7-[2,6-diisopropyl-4-(4-fluorphenyl)-5-morpholinomethyl-pyrid-3-yl]-3,5-dihydroxy-hept-6-enoat 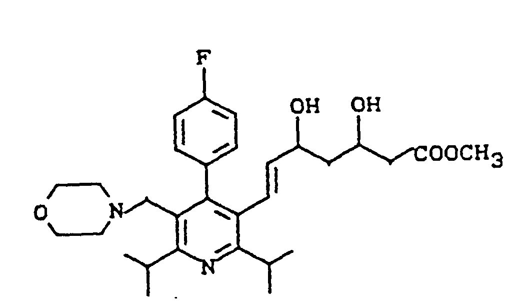 Aus der Verbindung aus Beispiel 104 wurde in Analogie zu den Reaktionen aus den Beispielen 7, 8, 9 und 10 das Beispiel 105 hergestellt.
- ¹H-NMR (CDCl₃): δ =: 1,25 (m, 12H); 1,40 (m, 2H); 2,20 (m, 4H); 2,45 (m, 2H); 3,20 (s, 2H); 3,30 (m, 1H); 3,45 (m, 1H); 3,55 (m, 4H); 3,75 (s, 3H); 4,10 (m, 1H); 4,30 (m, 1H); 5,30 (dd, 1H); 6,25 (d, 1H); 7,0 - 7,20 (m, 4H) ppm.

### Beispiel 106

Methyl-erythro-(E)-7-(2,6-diisopropyl)-4-(4-fluorphenyl)-5-iodomethyl-pyrid-3-yl]-3,5-dihydroxy-hept-6-enoat 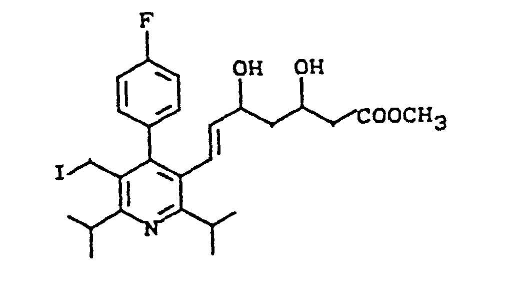 80 mg (0,1514 mmol) der Verbindung aus Beispiel 105 werden in 5 ml Methyljodid gelöst, 3 Stunden auf 30°C und über Nacht bei 60°C unter Lichtausschuß gerührt. Die Mischung wird im Vakuum eingeengt und im Exsikkator über Phosphorpentoxid getrocknet. Man erhält 120 mg Rohprodukt.
- ¹H-NMR (CDCl₃); δ =: 1,20 - 1,70 (komplexer Bereich, 14H); 2,45 (m, 2H); 3,30 (m, 2H); 3,75 (s, 3H); 4,05 (m, 1H); 4,20 (s, 2H); 4,30 (m, 1H); 5,30 (dd, 1H); 6,25 (d, 1H); 7,0 - 7,25 (m, 4H) ppm.

### Beispiel 107

Methyl-erythro-(E)-7-[2,6-diisopropyl-4-(4-fluorphenyl)-5-benzylthio-methyl-pyrid-3-yl]-3,5-dihydroxy-hept-6-enoat 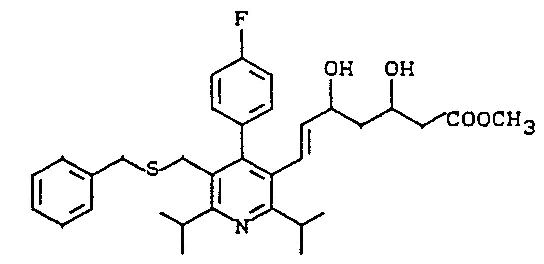 Zu 90 mg (0,158 mmol) der Verbindung aus Beispiel 106 gelöst in 2 ml trockenem Dichlormethan gibt man unter Stickstoffatmosphäre hintereinander 22,3 µl (0,19 mmol) Benzylmercaptan, 32,7 µl (0,237 mmol) Triethylamin und rührt über Nacht bei Raumtemperatur. Die Mischung wird mit Dichlormethan verdünnt und mehrmals mit Wasser extrahiert. Nach Trocknen der organischen Phase über Magnesiumsulfat und Einengen im Vakuum, wird der Rückstand an einer Säule (Kieselgel 70-230 mesh, mit Essigester/ Petrolether 1:1) chromatographiert.
- Ausbeute:: 20 mg (22,4% der Theorie)
- ¹H-NMR (CDCl₃): δ =: 1,23 (m, 12H); 1,4 (m, 2H); 2,40 (m, 2H); 3,20 (m, 2H); 3,28 (s, 2H); 3,55 (s, 2H); 3,73 (s, 3H); 4,05 (m, 1H); 4,25 (m, 1H); 5,25 (dd, 1H); 6,25 (d, 1H); 6,90 - 7,28 (m, 9H) ppm.

### Beispiel 108

4-{[5-(3,5-dihydroxy-6-methoxycarbonylhex-1-enyl)-2,6-diisopropyl-4-(4-fluorphenyl]-pyrid-3-yl}methylmorpholinoxid 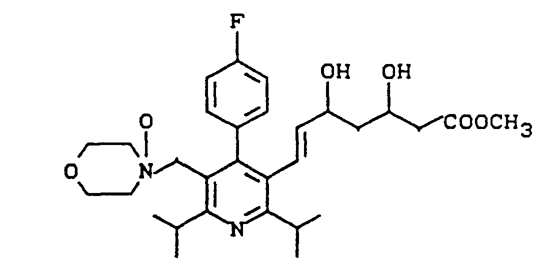 Zu 80 mg (0,1515 mmol) der Verbindung aus Beispiel 105 gelöst in 3 ml trockenem Dichlormethan gibt man 52 mg (0,303 mmol) m-Chlorperbenzoesäure und rührt 1 Stunde bei Raumtemperatur. Die Mischung wird nacheinander mit Kaliumjodid-Lösung, Natriumthiosulfat-Lösung und Natriumhydrogencarbonat-Lösung gewaschen, die organische Phase über Magnesiumsulfat getrocknet und im Vakuum eingeengt.
- Ausbeute:: 60 mg (73% der Theorie)
- ¹H-NMR (CDCl₃): δ =: 1,10 - 1,55 (komplexer Bereich, 14H); 2,45 (m, 2H); 2,70 - 3,70 (komplexer Bereich, 10H); 3,75 (s, 3H); 4,0 (m, 1H); 4,10 (m, 1H); 4,30 (m, 2H); 5,25 (dd, 1H); 6,25 (d, 1H); 7,0 - 7,30 (m, 4H) ppm.

### Beispiel 109

2-(4-Fluorbenzoyl)-4-methyl-pent-2-en-carbonsäureethylester 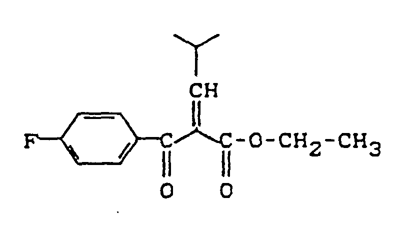 Eine Lösung von 210 g (1 mol) 4-Fluorbenzoylessigsäure-ethylester und 144 g (2 mol) 2-Methylpropanal wird in 100 ml Isopropanol mit 7 ml Piperidin und 5 ml Essigsäure über Nacht bei 50°C gerührt. Nach vollständiger Umsetzung wird der Ansatz bei ca. 15 Torr eingeengt und das Rohprodukt (270 g, ca. 85%ig) ohne weitere Reinigung weiter umgesetzt.

### Beispiel 110

3-Ethoxycarbonyl-2-(4-Fluorphenyl)-1,4-dihydro-4-isopropyl-6-methyl-5-methoxycarbonyl-pyridin 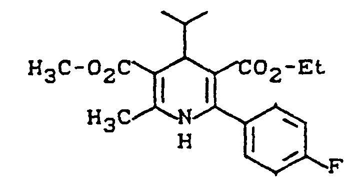 62,9 g (0,2 mol) der Verbindung aus Beispiel 109 und 21,9 g (0,19 mol) 3-Amino-crotonsäure-methylester werden in 200 ml Ethylenglykol über Nacht am Rückfluß gekocht. Man extrahiert dreimal mit Ether, wäscht die vereinigten organischen Phasen mit 2 N Salzsäure und gesättigter Kochsalzlösung, trocknet über Magnesiumsulfat und engt zur Trockene ein. Der Rückstand (65 g ) wird in zwei Portionen an je 750 g Kieselgel (230 - 400 mesh) in einer Säule (7,5 cm ø) mit Petrolether/Essigester 10:1 → 5:1 chromatographiert.
- Ausbeute:: 21,5 g (31%) gelbe Kristalle
- Schmp. :: 109°C

### Beispiel 111

3-Ethoxycarbonyl-2-(4-fluorphenyl)-4-isopropyl-5-methoxycarbonyl-6-methyl-pyridin 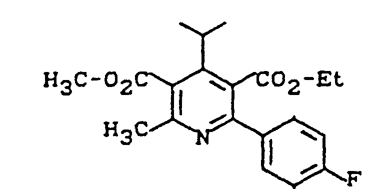 Analog Beispiel 3 wurde aus 14,9 g (14,5 mmol) der Verbindung aus Beispiel 110 das Beispiel 111 hergestellt.
- Ausbeute:: 15,2 g (102%) Rohprodukt, farbloses Öl, das ohne weitere Reinigung umgesetzt wird.
- ¹H-NMR (CDCl₃): δ =: 1,02 (t, 3H, CH₂CH₃); 1,33 (d, 6H, CH(CH₃)₂); 2,55 (s, 3H, 6-CH₃); 3,15 (sept, 1H, CH(CH₃)₂); 3,95 (s, 3H, O-CH₃); 4,08 (q, 2H, CH₂-CH₃); 7,1 (m, 2H, 3'-H); 7,55 (m, 2H, 2'-H); ppm.

### Beispiel 112

3-Ethoxycarbonyl-2-(4-fluorphenyl)-5-hydroxymethyl-4-isopropyl-6-methyl-pyridin 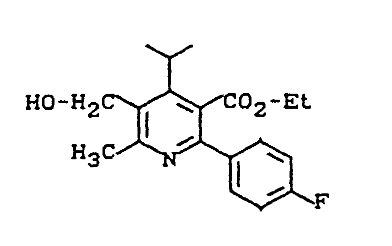 Analog Beispiel 4 wurde aus 10 g (27,8 mmol) der Verbindung aus Beispiel 111 das Beispiel 112 hergestellt.
- Ausbeute:: 4,53 g (49% der Theorie) gelbliche Kristalle
- Schmp. :: 113°C

### Beispiel 113

Methyl-erythro-(E)-7-[5-tert.butyldimethyl-silyloxymethyl-2(4-fluorphenyl)-4-isopropyl-6-methyl-pyrid-3-yl]-3,5-dihydroxy-hept-6-enoat 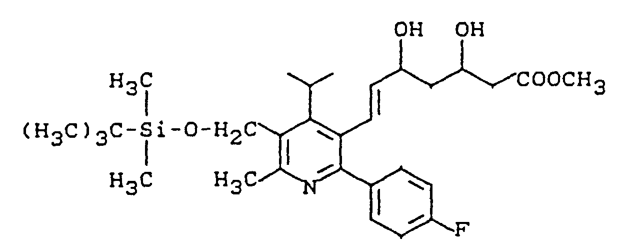 Aus der Verbindung des Beispiels 112 wurde in Analogie zu den Reaktionen aus den Beispielen 5, 6, 7, 8, 9 und 10 das Beispiel 113 hergestellt.
Man erhielt einen farblosen Schaum.
- ¹H-NMR (CDCl₃): δ =: 0,2 (s, 6H, Si(CH₃)₂); 9,13 (s, 9H, Si-C(CH₃)₃);; 1,3 (m, 8H, CH(CH₃)₂ + CH(OH)-CH₂-CH(OH)); 2,4 (m, 2H, CH₂-COOCH₃); 2,65 (s, 3H, 6'-CH₃); 3,1 (b, 1H, OH); 3,65 (b, 1H, OH); 3,7 (s, 3H, O-CH₃); 4,1 (m, 1H, CH-OH); 4,35 (m, 1H, CH-OH); 4,8 (s, 2H, 5'-CH₂); 5,15 (dd, 1H, 6-H); 6,7 (d, 1H, 7-H); 7,0 (m, 2H, 3"-H); 7,35 (m, 2H, 2''-H) ppm.

### Beispiel 114

Methyl-erythro-(E)-7-[2-(4-fluorphenyl)-5-hydroxymethyl-4-isopropyl-6-methyl-pyrid-3-yl]-3,5-dihydroxy-hept-6-enoat 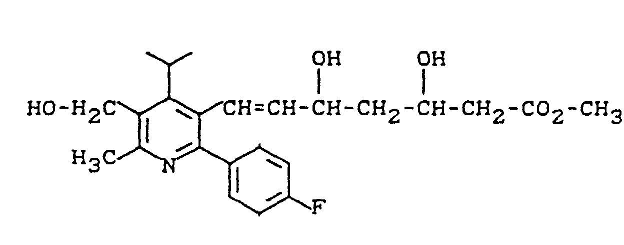 223 g (0,4 mmol) der Verbindng aus Beispiel 113 werden in 5 ml Methanol mit 0,5 ml 1 N Salzsäure 2 Tage bei Raumtemperatur gerührt. Einengen und Säulenchromatographie an 18 g Kieselgel 230-400 mesh, ø 2 cm, Chloroform/Methanol 10:1 ergeben 100 mg (57% der Theorie) farblosen Schaum.
- ¹H-NMR (CDCl₃): δ =: 1,2 - 1,45 (m, 8H, CH(CH₃)₂ + CH(OH)-CH₂-CH(OH)); 2,4 (m, 2H, CH₂-COOCH₃); 2,7 (s, 3H, 6'-CH₃); 3,1 (b, 1H, OH); 3,6 (m, 2H, CH(CH₃)₂ + OH); 3,7 (s, 3H, O-CH₃); 4,1 (m, 1H, CHOH); 4,35 (m, 1H, CH-OH); 4,88 (s, 2H, 5'-CH₂); 5,18 (dd, 1H, 6'-H); 6,7 (d, 1H, 7-H); 7,03 (m, 2H, 3''-H); 7,38 (m, 2H, 2"-H) ppm.

### Beispiel 115

Methyl-erythro-(E)-7-[5-benzyloxymethyl-2-(4-fluorphenyl)-4-isopropyl-6-methyl-pyrid-3-yl]-3,5-dihydroxy-hept-6-enoat 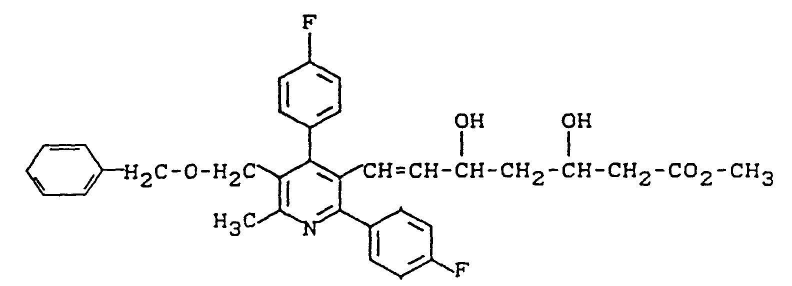 Aus der Verbindung des Beispiels 112 wurde in Analogie zu den Reaktionen aus den Beispielen 12, 6, 7, 8, 9 und 10 das Beispiel 115 hergestellt. Man erhielt einen farblosen Schaum.
- ¹H-NMR (CDCl₃): δ =: 1,2 - 1,45 (m, 8H, CH(CH₃)₂ + CH(OH)-CH₂-CH(OH); 2,4 (m, 2H, CH₂-COOCH₃); 2,6 (s, 3H, 6'-CH₃); 3,05 (b, 1H, OH); 3,5 (m, 2H, CH(CH₃)₂ + OH); 3,72 (s, 3H, O-CH₃); 4,1 (m, 1H, CH-OH); 4,35 (m, 1H, CHOH); 4,62 (s, 2H, O-CH₂); 4,66 (s, 2H, OCH₂); 5,15 (dd, 7H, 6-H); 6,2 (d, 1H, 7-H); 7,12 (m, 2H, 3"-H); 7,3 - 7,45 (m, 7H, Aromaten-H) ppm.

### Beispiel 116

Methyl-erythro-(E)-7-[2-(4-fluorphenyl)-4-isopropyl-5-methoxymethyl-6-methyl-pyrid-3-yl]-3,5-dihydroxy-hept-6-enoat 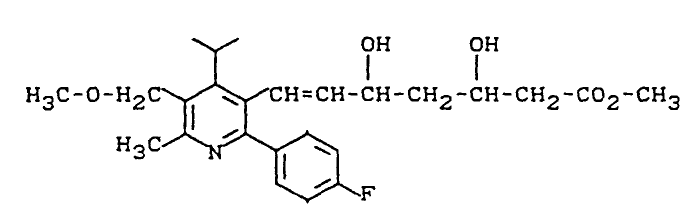 Aus der Verbindung des Beispiels 112 wurde in Analogie zu den Reaktionen aus den Beispielen 59, 6, 7, 8, 9 und 10 das Beispiel 116 hergestellt. Man erhielt einen farblosen Schaum.
- ¹H-NMR (CDCl₃): δ =: 1,2 - 1,45 (m, 8H, CH(CH₃)₂ + CH(OH)-CH₂-CH(OH); 2,4 (m, 2H, CH₂-COOCH₃); 2,63 (s, 3H, 66' -CH₃); 3,15 (b, 1H, OH); 3,5 (m, 4H, O-CH₃ + CH(CH₃)₂); 3,62 (b, 1H, OH); 3,71 (s, 3H, COOCH₃); 4,1 (m, 1H, CH-OH); 4,35 (m, 1H, CH-OH); 4,55 (s, 2H, O-CH2); 5,15 (dd, 1H, 6-H); 6,65 (d, 1H, 7-H); 7,0 (m, 2H, 3''-H); 7,35 (m, 2H, 2''-H) ppm.

### Beispiel 117

3-Benzyloxymethyl-4-(4-fluorphenyl)-6-isopropyl-5-(methyl-erythro-(E)-3,5-dihydroxy-hept-6-enoat-7-yl)-pyridin-N-oxid 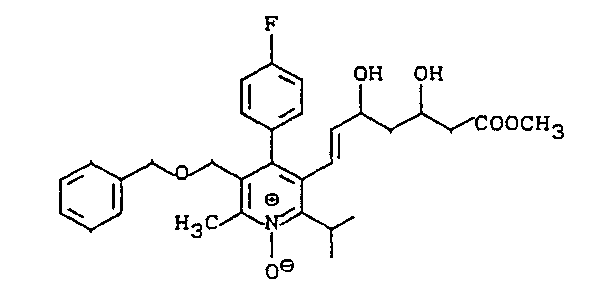 208,4 mg (0,4 mmol) der Verbindung aus Beispiel 64 gelöst in 10 ml Dichlormethan werden mit 863 mg (4 mmol) 80 %iger meta-Chlorperoxybenzoesäure versetzt und über Nacht bei Raumtemperatur gerührt. Nach Einengen im Vakuum wird der Rückstand an einer Säule (Kieselgel 70-230 mesh, mit Dichlormethan/Methanol 96:4) chromatographiert.
- Ausbeute:: 107 mg (50% der Theorie)
- ¹H-NMR (CDCl₃): δ =: 1,20 - 1,60 (m, 2H); 1,45 (d, 6H); 2,40 (m, 2H); 2,58 (s, 3H); 3,62 (m, 1H); 3,72 (s, 3H); 4,08 (m, 1H); 4,12 (s, 2H); 4,30 (m, 1H); 4,38 (s, 2H); 5,23 (dd, 1H); 6,28 (d, 1H); 7,00 - 7,40 (m, 9H) ppm.

### Beispiel 118

3-Amino-4'-fluor-zimtsäurenitril 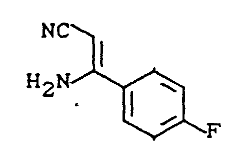 Zu einer Suspension von 30 g (1 mol) Natriumhydrid in 300 ml p.a. Tetrahydrofuran tropft man bei Raumtemperatur unter Rühren eine Lösung von 41 g (1 mol) Acetonitril, 135 g (1,1 mol) 4-Fluorbenzonitril und 7,4 g (0,1 mol) tert.-Butanol in 300 ml Tetrahydrofuran zu und erhitzt auf ca. 30°C, bis die Reaktion anspringt. Unter externem Kühlen wird der Rest bei 35 - 40°C zugetropft und anschließend 30 min unter Rückfluß gekocht. Dann werden vorsichtig 500 ml Wasser zugetropft, die wäßrige Phase zweimal mit Essigester extrahiert und die vereinigten organischen Phasen über Natriumsulfat getrocknet. Nach Abziehen des Lösemittels bleibt ein Kristallbrei, der mit Ether verrührt und abgesaugt wird (65,1 g). Das Filtrat wird zur Trockene einrotiert, mit Toluol über 300 g Kieselgel filtriert und wie oben aus Ether kristallisiert (46,3 g).
- Ausbeute:: 111,4 g (69% der Theorie) farblose Kristalle
- Schmp.:: 108°C

### Beispiel 119

E/Z-4-Methoxycarbonyl-2,6-dimethyl-hept-4-en-3-on 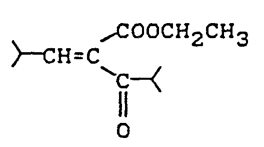 158 g (1 mol) Isobutyrylessigsäureethylester, 108 g (1,5 mol) Isobutyraldehyd, 8,75 ml Piperidin und 6,25 ml Essigsäure werden in 400 ml Isopropanol 20 h bei 50°C gerührt. Man zieht im Wasserstrahlvakuum flüchtige Komponenten ab und destilliert anschließend im Hochvakuum.
- Ausbeute:: 145 g (68 % der Theorie) farbl, Öl,
- Kp =: 60°C, 0,2 mbar

### Beispiel 120

3-Cyano-5-ethoxycarbonyl-2-(4-fluorphenyl)-1,4-dihydro-4,6-diisopropyl-pyridin 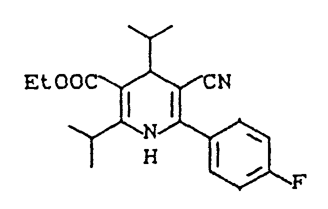 26,7 g (165 mmol) der Verbindung aus Beispiel 118 und 35 g (165 mmol) E/Z-4-Ethoxycarbonyl-2,6-dimethyl-hept-4-en-3-on aus Beispiel 130 werden 4 h bei 200°C Badtemperatur erhitzt. Dann gibt man weitere 17 g (80 mmol) der letzten Komponente zu und erhitzt über Nacht. Der Rückstand wird mit 3 1 Toluol/Petrolether (1:1), 3 1 Toluol und 2 1 Toluol/Essigester (10:1) über 450 g Kieselgel (230-400 mesh) vorgereinigt. Aus dem eingeengten Filtrat kristallisieren aus Ether 5,7 g (9,7%) gelbliche Kristalle (Schmp.: 140°C). Das Filtrat wird nochmal an 750 g Kieselgel mit Petrolether/Essigester (10:1) chromatographiert. Man erhält zwei Zonen:
1. 7,4 g (12,5%) farblose Kristalle vom Schmp.: 141°C (aus Ether/Petrolether) und als Nebenprodukt
2. 3,5-Bis-cyano-2,6-bis-4-fluorphenyl-1,4-dihydro-4-isopropyl-pyridin [2,2 g (3,7%) gelbliche Kristalle vom Schmp.: 227 - 228°C, aus Ether/Petrolether].

- Gesamtausbeute:: 13,1 g (22% der Theorie)
- ¹H-NMR (CDCl₃): δ =: 0,93 (d, 3H); 1,02 (d, 3H); 1,2 (m, 6H); 1,3 (t, 3H); 1,8 (m, 1H) ; 3,6 (d, 1H); 4,2 (m, 2H); 6,15 (b, 1H); 7,2 (t, 2H); 7,53 (m, 2H) ppm.

### Beispiel 121

3-Cyano-5-ethoxycarbonyl-2-(4-fluorphenyl)-4,6-diisopropyl-pyridin 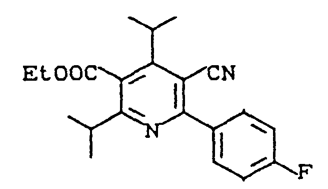 Die Darstellung erfolgt analog der Vorschrift von Beispiel 3 aus 16,0 g (45 mmol) der Verbindung aus Beispiel 120.
- Ausbeute:: 14,5 g (91% der Theorie)
- Schmp.:: 82°C

### Beispiel 122

2-(4-Fluorphenyl)-3-formyl-5-hydroxymethyl-4,6-diisopropyl-pyridin 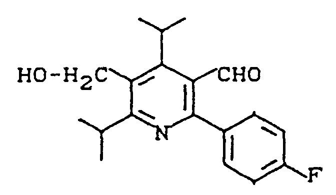 Zu einer Lösung von 14,5 g (41 mmol) der Verbindung aus Beispiel 121 in 320 ml Toluol p.a. tropft man unter Argon bei -78°C bis -75°C 110 ml (165 mmol) einer 1,5 M Lösung von Diisobutylaluminiumhydrid in Toluol, rührt 2 h bei dieser Temperatur und anschließend 1 h bei -20°C. Dann werden 350 ml Wasser und 250 ml Essigester zugetropft, mit Kieselgur abgesaugt, mit Essigester nachgewaschen und die wäßrige Phase mit 300 ml Essigester extrahiert. Die vereinigten organischen Phasen werden mit Kochsalzlösung gewaschen, über Natriumsulfat getrocknet und eingeengt. Säulenchromatographie an 500 g Kieselgel (230-400 mesh) mit Petrolether/Essigester (5:1) und Umkristallisation aus Ether/Petrolether ergibt 5,4 g (42% der Theorie) gelbliche Kristalle vom Schmp.: 147°C.

### Beispiel 123

(E) -3-[2-(4-Fluorphenyl)-5-hydroxymethyl-4,6-diisopropyl-pyridin-3-yl]-prop-2-enal 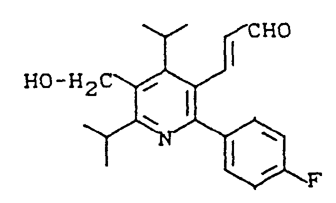 Die Darstellung erfolgt analog Beispiel 8 aus 200 mg (6,6 mmol) 80%igem Natriumhydrid, 0,86 g (3,3 mmol) Diethyl-2-(cyclohexylamino)-vinylphosphonat und 0,94 g (3 mmol) der Verbindung aus Beispiel 122.
- Ausbeute:: 0,46 g (45% der Theorie)
- Schmp.:: 210°C

### Beispiel 124

Methyl-(E)-7-[2-(4-fluorphenyl)-5-hydroxymethyl-4,6-diisopropyl-pyridin-3-yl]-5-hydroxy-3-oxo-hept-6-enoat 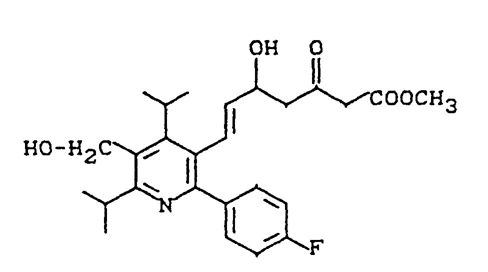 Zu einer Suspension von 0,15 g (5 mmol) 80%igem Natriumhydrid in 6,5 ml Tetrahydrofuran p.a. tropft man bei 0°C - 5°C unter Argon 0,5 ml (4,5 mmol) Acetessigsäuremethylester. Nach jeweils 15 min. werden bei 0°C zuerst innerhalb 10 min 3,65 ml (6 mmol) 15%iges Butyllithium in Hexan zugetropft, dann eine Lösung von 1,01 g (4,5 mmol) trockenem Zinkbromid in 4,5 ml Tetrahydrofuran und schließlich 0,51 g (1,5 mmol) der Verbindung aus Beispiel 123. Man rührt über Nacht bei Raumtemperatur, versetzt langsam mit gesättigter Ammoniumchlorid-Lösung, extrahiert die wäßrige Phase mit Essigester, wäscht die vereinigten organischen Phasen mit gesättigter Kochsalz-Lösung, trocknet über Natriumsulfat und engt ein. Nach Säulenchromatographie (ø 3 cm) an 20 g Kieselgel (230-400 mesh) mit Petrolether-Essigester (1:1) erhält man 0,17 g (25% der Theorie) gelbliches Öl.
R_{f} = 0,35 (Petrolether-Essigester (1:1)).

### Beispiel 125

Methyl-erythro-(E)-7-[2-(4-fluorphenyl)-5-hydroxymethyl-4,6-diisopropyl-pyridin-3-yl]-3,5-dihydroxy-hept-6-enoat 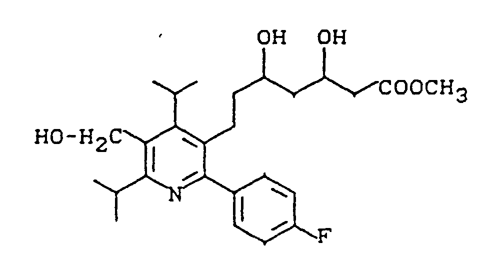 Die Darstellung erfolgt analog der Vorschrift von Beispiel 10 aus 0,15 g (0,33 mmol) der Verbindung aus Beispiel 124.
- Ausbeute:: 85 mg (56% der Theorie)
- ¹H-NMR (CDCl₃): δ =: 1,25 - 1,6 (m, 14H, CH(OH)-CH₂-CH(OH) + CH(CH₃)₂); 2,45 (m, 2H, CH₂-COOCH₃); 3,1 (b, 1H, OH); 3,45-3,7 (m, 3H, CH(CH₃)₂ + OH); 3,7 (s, 3H, OCH₃); 4,1 (m, 1H, CH(OH); 4,35 (m, 1H, CH(OH); 4,85 (s, 2H, CH₂-OH); 5,7 (dd, 1H, 6-H); 6,25 (d, 1H, 7-H); 7,05 (t, 2H, 3"-H); 7,45 (m, 2H, 2"-H) ppm.

### Beispiel 126

5-Ethoxycarbonyl-2-(4-fluorphenyl)-3-formyl-4,6-diisopropyl-pyridin 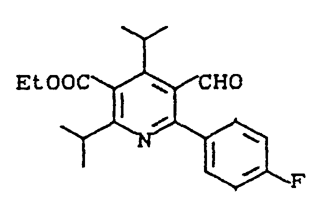 Die Darstellung erfolgt analog dem Verfahren für Beispiel 122 aus 3,5 g (10 mmol) der Verbindung aus Beispiel 121 und 35 ml 1 M Lösung von Diisobutylaluminiumhydrid in Toluol, wobei 1,5 h die Temperatur bei -75°C gehalten wird, dann auf -30°C erwärmt wird, bevor man mit Wasser versetzt.
- Ausbeute:: 0,8 g (22% der Theorie) farblose Kristalle
- Schmp.:: 88°C (aus Petrolether)

### Beispiel 127

(E) -3-[5-Ethoxycarbonyl-2-(4-fluorphenyl)-4,6-diisopropyl-pyridin-3-yl]-prop-2-enal 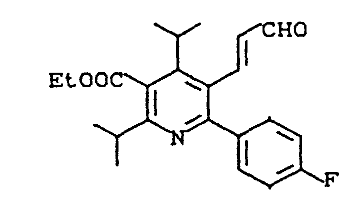 Die Darstellung erfolgt analog der Vorschrift von Beispiel 8 aus 1,25 g (3,5 mmol) der Verbindung aus Beispiel 126.
- Ausbeute:: 0,17 g (72% der Theorie) farbloses Öl
- R_{f} =: 0,35 (Petrolether-Essigester (5:1)).

### Beispiel 128

Methyl-(E)-7-[5-ethoxycarbonyl-2-(4-fluorphenyl)-4,6-diisopropyl-pyridin-3-yl]-5-hydroxy-3-oxo-hept-6-enoat 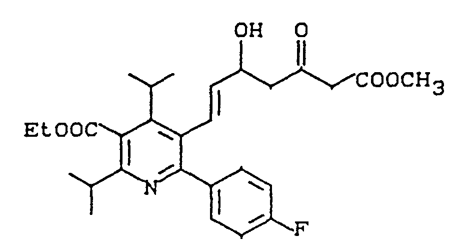 Die Darstellung erfolgt analog der Vorschrift von Beispiel 123 aus 0,95 g (2,48 mmol) der Verbindung aus Beispiel 127.
- Ausbeute:: 0,83 g (67% der Theorie) farbloses Öl
- R_{f} =: 0,27 (Petrolether-Essigester (2:1))

### Beispiel 129

Methyl-erythro-(E)-7-[5-ethoxycarbonyl-2-(4-fluorphenyl)-4,6-diisopropyl-pyridin-3-yl]-3,5-dihydroxy-hept-6-enoat 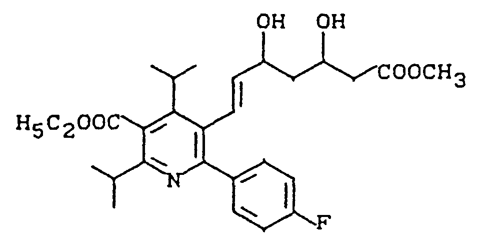 Die Darstellung erfolgt analog der Vorschrift von Beispiel 10 aus 0,89 g (1,66 mmol) der Verbindung aus Beispiel 128.
- Ausbeute:: 0,65 g (78% der Theorie) farbloses Öl
- ¹H-NMR (CDCl₃): δ =: 1,25 - 1,5 (m, 17H, CH(CH₃)₂ + CH₂-CH₃ + CH(OH)-CH₂); 2,42 (m, 2H, CH₂-COOCH₃); 2,95 (m, 1H, CH(CH₃)₂); 3,15 (b, 1H, OH); 3,2 (m 1H, CH(CH₃)₂); 3,6 (b, 1H, OH); 3,7 (s, 3H, O-CH₃); 4,1 (m, 1H, CH-OH); 4,4 (m, 3H, CH-OH + O-CH₂-CH₃); 5,2 (dd, 1H, 6-H); 6,75 (d, 1H, 7-H); 7,05 (t, 2H, 3"-H); 7,45 (m, 2H, 2"-H) ppm.

### Beispiel 130

Methyl-erythro-(E)-7-[2-(4-fluorphenyl)-4-isapropyl-5-methoxy-methyl-6-methyl-1-oxyl-pyridin-3-yl]-3,5-dihydroxy-hept-6-enoat 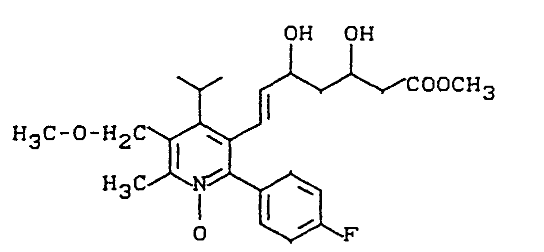 67 mg (0,15 mmol) der Verbindung aus Beispiel 116 und 54 mg (0,17 mmol) 55%ige Metachlorperbenzoesäure werden in 6 ml Dichlormethan 2 h bei Raumtemperatur gerührt. Das Lösemittel wird abgezogen, der Rückstand in 20 ml Essigester aufgenommen und mit 20 ml gesättigter Natriumhydrogencarbonatlösung gewaschen. Die wäßrige Phase wird mit 20 ml Essigester gewaschen und die vereinigten organischen Phasen über Magnesiumsulfat getrocknet. Das Rohprodukt wird an 10 g Kieselgel (230-400 mesh) in einer Säule (ø 2 cm) mit Essigester und Essigester/Methanol (10:1) chromatographiert.
- Ausbeute:: 57 mg (82% der Theorie) amorpher Feststoff
- ¹H-NMR (CDCl₃): δ =: 1,1 - 1,3 (m, 2H, CH(OH)-CH₂-CH(OH)); 1,35 (d, 6H, CH(CH₃)₂); 2,38 (m, 2H, CH₂-COOCH₃); 2,6 (s, 3H, 6'-CH₃); 3,4 (b, 1H, OH); 3,5 (m, 4H, CH(CH₃)₂ + CH₂-O-CH₃); 3,28 (b, 1H, OH); 3,72 (s, 3H, COOCH₃); 4,05 (m, 1H, HO-C-H); 4,28 (m, 1H, HO-C-H); 4,55 (s, 2H, O-CH₂); 5,12 (dd, 1H, 6-H); 6,38 (d, 1H, 7-H); 7,05 - 7,25 (m, 4H, Aromaten-H) ppm.
- MS:: m/e = 461 (9%), M⁺.

### Beispiel 131

2-(4-Fluorphenyl)-3-formyl-4,6-diisopropyl-5-methoxymethyl pyridin 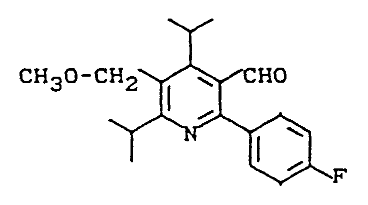 Aus 3,15 g (10 mmol) der Verbindung aus Beispiel 122 in Analogie zur Vorschrift aus Beispiel 59 erhält man 3,15 g (96 % der Theorie) farblose Kristalle vom Schmp. 77°C (aus MeOH)

### Beispiel 132

Methyl-erythro-(E)-7-[2-(4-fluorphenyl)-4,6-diisopropyl-5-methoxymethyl-pyridin-3-yl]-3,5-dihydroxy-hept-6-enoat 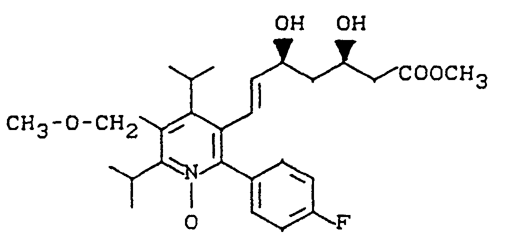 Aus der Verbindung aus Beispiel 131 erhält man gemäß den Vorschriften aus Beispiel 8, 9 und 10 farblose Kristalle vom Schmp. 92°C (aus Ether/Petrolether)
- ¹H-NMR (CDCl₃): δ =: 1,3 (m, 14H, CH(CH₃)₂ + 4-H); 2,4 (m, 2H, 2-H); 3,05 (b, 1H, OH); 3,3-3,57 (m, 5H, O-CH₃ + CH(CH₃)₂); 3,62 (b, 1H, OH); 3,72 (s, 3H, COOCH₃); 4,1 (m, 1H CH-OH) 4,35 (m, 1H, CH-OH); 4,55 (s, 2H, O-CH₂); 5,'5 (dd, 1H, 6-H); 6,75 (d, 1H, 7-H); 7,0 (+, 2H, 3"H); 7,45 (m, 2H, 2"-H).

### Beispiel 133

5-Cyano-3-ethoxycarbonyl-2-(4-fluorphenyl)-1,4-dihydro-4-isopropyl-6-methyl-pyridin 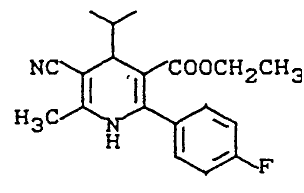 17,4 g (0,2 mol) 3-Amino-crotonsäurenitril und 56 g (0,2 mol) der Verbindung aus Beispiel 109 werden in 800 ml Ethanol über Nacht am Rückfluß gekocht. Das Lösungsmittel wird abgezogen und der Rückstand an Kieselgel mit Petrolether/Essigester 20:1 chromatographiert.
- Ausbeute:: 14,9 g (21 % der Theorie)
- ¹H-NMR ( COCl₃): δ =: 1,0 (m, 9H); 1,9 (m, 1H); 2,2 (m, 3H); 3,6 (d, 1H); 3,9 (m, 2H); 5,75 (b, 1H); 7,1 (t, 2H); 7,25 (m, 2H).

### Beispiel 134

5-Cyano-3-ethoxycarbonyl-2-(4-fluorphenyl)-4-isopropyl-6-methyl-pyridin 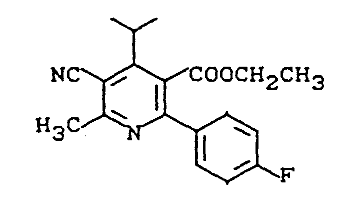 Analog Beispiel 3 aus 9,8 g (30 mmol) der Verbindung aus

Beispiel 133
Ausbeute: 9,4 g (96 % der Theorie), Schmp. 76°C.

### Beispiel 135

5-Cyano-2-(4-fluorphenyl)-3-hydroxymethyl-4-isopropyl-5-methyl-pyridin 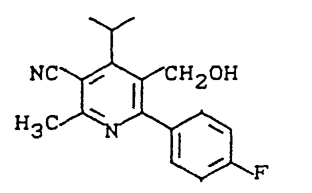 Unter Argon gibt man zu einer Lösung von 9,8 g (30 mmol) der Verbindung aus Beispiel 134 in 150 ml Toluol p.a. bei -75°C innerhalb von 1,5 h 50 ml (60 mmol) 1,2 M Diisobutylaluminiumhydrid-Lösung in Toluol zu und rührt weitere 30 min.

Bei -30°C werden vorsichtig 280 ml Wasser zugetropft, mit Kieselgur abgesaugt und mit Essigester nachgewaschen. Die wäßrige Phase wird dreimal mit Essigester extrahiert, die vereinigten organischen Phasen mit ges. Kochsalzlösung gewaschen, über Natriumsulfat getrocknet und eingeengt. Säulenchromatographie (400 g Kieselgel, 230-400 mesh, ø 6 cm, PetroletherlEssigester 10/1 → 5/1) ergibt 3 Zonen:
1. 3-Ethoxycarbonyl-2-(4-fluorphenyl)-5-formyl-4-isopropyl-6-methyl-pyridin 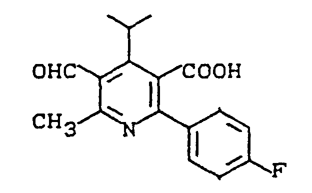 2,6 g (26 % der Theorie) vom Schmp. 53°C
2. 2,1 g (25 % der Theorie) der Titelverbindung (Beispiel 135) Schmp. 157°C aus Ether/Petrolether.
3. 2-(4-Fluorphenyl)-5-formyl-3-hydroxymethyl)-4-isopropyl-6-methyl-pyridin 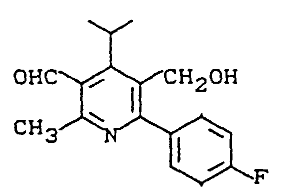 1,6 g (19 % der Theorie), Schmp. 150°C aus Ether/Petrolether.

### Beispiel 136

5-Cyano-2-(4-fluorphenyl)-3-formyl-4-isopropyl-6-methyl-pyridin. 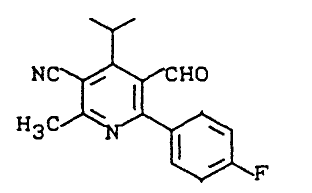 Zu 1,1 g (14 mmol) DMSO in 8 ml Methylenchlorid tropft man bei -75°C 2,2 g (10,5 mmol) Trifluoressigsäureanhydrid in 10 ml Methylenchlorid, rührt 10 min bei -70°C, tropft dann eine Lösung von 2,0 g (7 mmol) der Verbindung aus Beispiel 135 in 50 ml Methylenchlorid zu und rührt 1 h bei -70°C.

Zu der jetzt vorliegenden Suspension werden 2,1 ml (21 mmol) Triethylamin getropft und 10 min bei -65°C gerührt. Nach Erwärmen auf Raumtemperatur wird mit ges. Kochsalzlosung gewaschen, über Natriummsulfat getrocknet und eingeengt.
- Rohausbeute:: 2,0 g (100 % der Theorie), Schmp. 109°C
- ¹H-NMR (CDCl₃): δ =: 1,52 (d, 6H, CH(CH₃)₂); 2,9 (s,, 3H, 6-CH₃); 4,0 (sept. 1H, CH(CH₃)₂); 7,2 (m, 2H, 3'-H); 7,5 (m, 2H, 2'H); 9,95 (s, 1H, CHO).

### Beispiel 137

(E)-3-[5-Cyano-2-(4-fluorphenyl)-4-isopropyl-6-methyl-pyrid-3-yl]-prop-2-enal 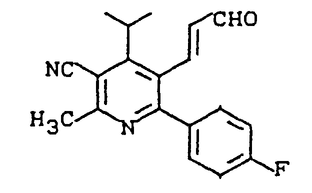 Analog Beispiel 8 aus 1,9 g der Verbindung aus Beispiel 136
Ausbeute: 0,6 g (28 % der Theorie), Schmp. 112°C (Ether-Petrolether).

### Beispiel 138

Methyl-(E)-7-[5-cyano-2-(4-fluorphenyl)-4-isopropyl-6-methyl-pyrid-3-yl]-5-hydroxy-3-oxo-hept-6-enoat 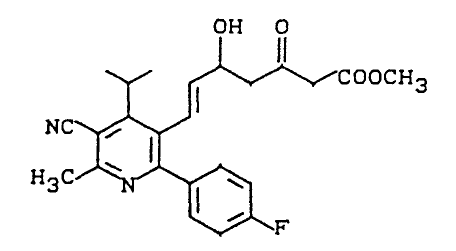 Analog Beispiel 9 aus 0,52 g (1,7 mmol) der Verbindung aus Beispiel 137
Ausbeute: 0,32 g (44 % der Theorie) gelbliches Öl.

### Beispiel 139

Methyl-erythro-(E)-7-[5-Cyano-2-(4-fluorphenyl)-4-isopropyl-6-methyl-pyrid-3-yl]-3,5-dihydroxy-hept-6-enoat 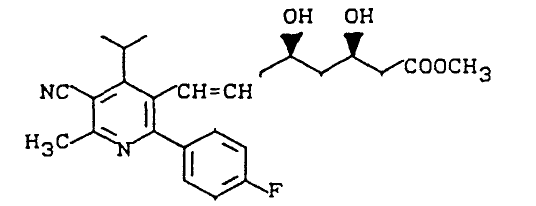 Analog Beispiel 10 aus 0,32 g der Verbindung aus Beispiel 138
- Ausbeute:: 0,15 g (47 % der Theorie) gelbliches Öl.
- ¹H-NMR (CDCl₃): δ =: 1,35 (m, 2H, CH(OH)CH₂-CH(OH); 1,45 (d, 6H, CH(CH₃)₂); 2,42 (m, 2H, CH₂-COOCH₃); 2,7 (s, 3H, 6-CH₃); 3,55 (m, 2H, CH(CH₃)₂ + OH); 3,68 (b, 1H, OH); 3,72 (s, 3H, O-CH₃); 4,13 (m, 1H, CH(OH)), 4,4 (m, 1H, CH(OH)); 5,32, (dd, 1H, Olefin-H); 6,6 (d, 1H, Olefin-H); 708 (m, 2H, 3"-H); 7,45 (m, 2H, 2"-H).

### Beispiel 140

4-Fluorbenzoylessigsäureethylester 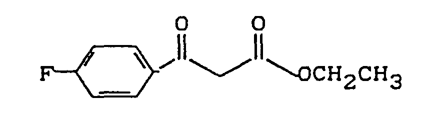 In einen Liter Diethylether p.a. werden 21,7 g (0,72 mol) Natriumhydrid (80 %ig, 20 % Mineralöl) eingewogen und anschließend 85,5 g (127 ml, 0,72 mol) Kohlensäurediethylester (VK 22-010) zugegeben. In dieser Lösung wird in der Siedehitze über einen Zeitraum von 4 Stunden eine Lösung von 100 g (0,72 mol) 4-Fluoracetophenon in 300 ml Diethylether zugetropft (kräftiger, mechanischer Rührer erforderlich; es bildet sich ein zäher Brei). Danach wird eine weitere Stunde am Rückfluß gekocht, dann auf ca. 5°C abgekühlt und bei dieser Temperatur unter N₂ zunächst eine Lösung aus 50 ml Essigsäure und 100 ml Et₂O zugetropft. Anschließend wird etwa 500 ml H₂O zugetropft und die organische Phase abgetrennt. Die Wasserphase wird nochmals mit Et₂O extrahiert (2 x 400 ml), die vereinigten etherischen Phasen mit NaHCO₃-Lösung gewaschen, über MgSO₄ getrocknet und eingeengt, Der Rückstand wird über eine kurze Vigreux-Kolonne destilliert.
- Ausbeute:: 93 g (60 %) Kp 0,4 mm 99-102°C

### Beispiel 141

5-Cyano-3-ethoxycarbonyl-4-(4-fluorphenyl)-1,4-dihydro-2-isopropyl-6-methyl-pyridin 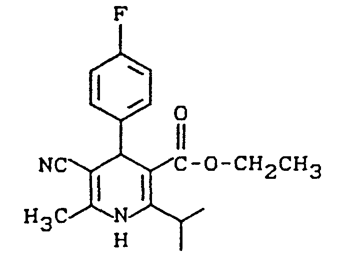 52,8 g (0,2 mol) der Verbindung aus Beispiel 1 und 16,4 g (0,2 mol) 3-Amino-crotonsäurenitril werden in 200 ml Ethylenglykol 2 h am Rückfluß gekocht. Nach dem Abkühlen hebt man das abgeschiedene Öl ab, und extrahiert noch dreimal mit Ether. Die Etherphasen werden mit Wasser gewaschen, getrocknet und eingeengt. Der Rückstand und das zuerst isolierte Öl werden aus Ether kristallisiert.
- Ausbeute:: 31,4 g (48 %), Schmp. 168°C.

### Beispiel 142

5-Cyano-3-ethoxycarbonyl-4-(4-fluorphenyl)-2-isopropyl-6-methyl-pyridin 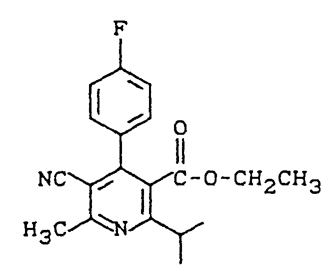 Analog Beispiel 3 werden 14,8 g (45 mmol) der Verbindung aus Beispiel 141 umgesetzt.
- Ausbeute:: 13,7 g (93 % der Theorie) farbl. Kristalle vom Schmp. 95°C.

### Beispiel 143

5-Cyano-4-(4-fluorphenyl)-3-hydroxymethyl-2-isopropyl-6-methyl-pyridin 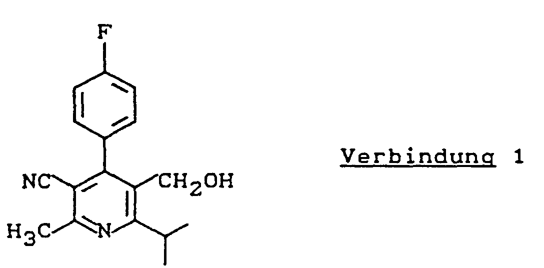 5-Cyano-4-(4-fluorphenyl)-5-hydroxyiminomethyl-3-hydroxymethyl-2-isopropyl-6-methyl-pyridin 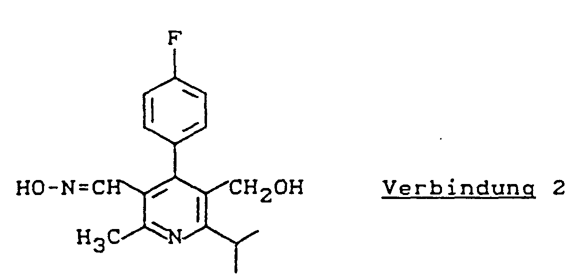 16,3 g (50 mmol) der Verbindung aus Beispiel 142 in 240 ml Toluol p.A. tropft man bei -78°C unter Argon innerhalb von 3 h 83,3 ml (0,1 mol) 1,2 M Diisobutylaluminiumhydrid Lösung in Toluol, daß die Temperatur unter -75°C bleibt.

Man rührt noch 30 min bei -75°C, läßt auf -30°C erwärmen und gibt dann vorsichtig 300 ml Wasser und 160 ml Essigester zu.

Über Kieselgur wird abgesaugt und mit Essigester nachgewaschen. Die wäßrige Phase wird dreimal mit Essigester extrahiert, die vereinigten org. Phasen mit ges. Kochsalzlösung gewaschen, über Natriumsulfat getrocknet und eingeengt. Zweimalige Säulenchromatographie über Kieselgel mit Toluol/Essigester 5:1 bzw. Petrolether/Essigester 5:1 ergibt 3,4 g einer Fraktion von R_{f}-Wert 0,2 (Petrolether/Essigester 5:1), die ein Gemisch aus 5-Cyano-4-(4-fluorphenyl)-3-hydroxymethyl-2-isopropyl-6-methyl-pyridin und 4-(4-Fluorphenyl)-5-formyl-3-hydroxy-2-isopropyl-6-methyl-pyridin darstellt.

3,16 g dieses Gemisches werden in 10 ml Methanol gelöst und zu einer Lösung von 1,22 g (17,6 mol) Hydroxylaminhydrochlorid und 1,22 g (14,9 mmol) Natriumacetat in 10 ml Wasser gegeben. Nach 3 h Rühren bei Raumtemperatur wird das Methanol abrotiert, etwas Wasser zugegeben und dreimal mit Essigester extrahiert. Die org. Phasen werden mit ges. Kochsalzlösung gewaschen, unter Natriumsulfat getrocknet und eingeengt. Den Rückstand chromatographiert man in einer Säule (100 g Kieselgel 230-400 mesh, ø 4 cm, Petrolether/Essigester 5:1). Man erhält zwei Fraktionen:
- Verbindung 1:: 0,65 g (4,9 %) farbl. Kristalle vom Schmp, 132°C
5-Cyano-4-(4-fluorphenyl)-3-hydroxymethyl-2-isopropyl-6-methyl-pyridin
- ¹H-NMR (CDCl₃): δ =: 1,32 (d, 6H,, CH(CH₃)₂); 1,6 (b, 1H, OH); 2,7 (s, 3H, 6-CH₃); 3,5 (sept, 1H, CH(CH₃)₂); 4,5 (d, 2H, CH₂-OH); 7,15-7,35 (m, 4H, Aromaten-H).
- Verbindung 2:: 2,15 g (15,3 %) amorpher Feststoff 5-Cyano-4-(4-fluorphenyl)-5-hydroxy-iminomethyl-3-hydroxymethyl-2-isopropyl-6-methyl-pyridin
- ¹H-NMR (CFCl₃): δ =: 1,33 (d, 6H, CH(CH₃)₂); 1,4 (b, 1H, -OH); 2,7 (s, 3H,, 6-CH₃); 3,48 (m, 1H, CH(CH₃)₂); 4,4 (d, 2H, CH₂-OH); 7,15 (m, 4H, Aromaten-H); 7,77 (s, 1H, CH=N-); 8,08 (s, 1H, =N-OH).

### Beispiel 144

5-Cyano-4-(4-fluorphenyl )-3-formyl-2-isopropyl-6-methyl-pyridin 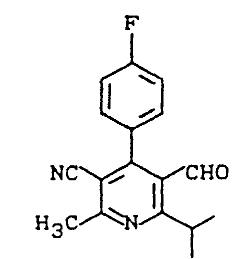
A) 0,63 g (2,2 mmol) der Verbindung 1 aus Beispiel 144 wird analog dem Verfahren aus Beispiel 136 umgesetzt. Ausbeute: 0,6 g (97 %).
B) Zu 1,1 g (14 mmol) DMSO in 8 ml Methylenchlorid p.A. tropft man bei -78°C 4,4 g (21 mmol) Trifluoressigsäureanhydrid in 15 ml Methylenchlorid und rührt 10 min bei -70°C. Dann gibt man 2,1 g (7 mmol) der Verbindung 2 aus Beispiel 143 in 50 ml Methylenchlorid zu, rührt 1 h bei -70°C, gibt jetzt 5,8 ml (42 mmol) Triethylamin zu und rührt 4 h bei Raumtemperatur. Es wird mit ges. Kochsalzlösung gewaschen, eingeengt, über 100 g Kieselgel (230-400 mesh, ø 4 cm, Petrolether/Essigester 10:1) chromatographiert und aus Essigester/Petrolether umkristallisiert.
   - Ausbeute:: 0,31 g (16 %), Schmp. 82°C
   - ¹H-NMR (CDCl₃): δ =: 1,32 (d, 6H, CH(CH₃)₂); 2,78 (s, 3H, 6-CH₃); 3,77 (m, 1H, CH(CH₃)₂); 7,23 (m, 2H, 3'-H); 7,36 (m, 2H, 2'-H); 9,86 (s, 1H, CHO).

### Beispiel 145

Methyl-erythro-(E)-7-[5-cyano-4-(4-fluorphenyl)-2-isopropyl-6-methyl-pyrid-3-yl]-3,5-dihydroxy-hept-6-enoat 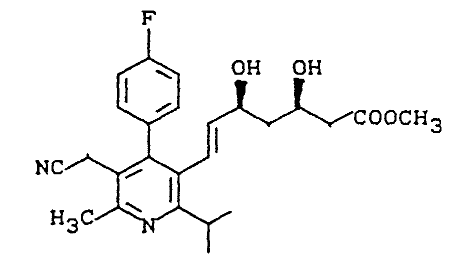 Die Umsetzung erfolgt aus der Verbindung aus Beispiel 144 in Analogie zu den Reaktionen aus Beispiel 8, 9 und 10. Farbloser Feststoff, Schmp. 124°C.
- ¹H-NMR (CDCl₃): δ =: 1,3 (m, 8H, CH(CH₃)₂ + CH(OH)-CH₂-CH(OH)); 2,4 (m, 2H, CH₂-COOCH₃); 2,75 (s, 3H, 6-CH₃); 3,35 (m, 2H, CH(CH₃) + OH); 3,6 (b, 1H, OH); 3,7 (s, 3H O-CH₃); 4,1 (m, 1H, CH-OH); 4,35 (m, 1H, CH-OH); 5,3 (dd, 1H, 6-H); 6,4 (d, 1H, 7-H); 7,05-7,3 (m, 4H, Ar-H).

### Beispiel 146

5-(tert.-Butyldimethylsilyloxymethyl)-6-cyclopropyl-4-(4-fluorphenyl)-2-isopropyl-pyridin-3-carbonsäureethylester 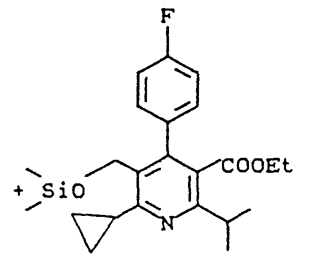 15 g (42 mmol) der Verbindung aus Beispiel 82 werden analog Beispiel 5 umgesetzt.
- Ausbeute:: 16,9 g (85,4 % der Theorie)

### Beispiel 147

3-(tert.-Butyldimethylsilyloxymethyl)-2-cyclopropyl-4-(4-fluorphenyl)-5-hydroxymethyl-6-isopropyl-pyridin 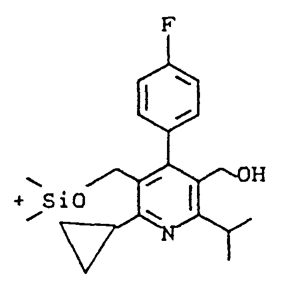 16,9 g (35,8 mmol) der Verbindung aus Beispiel 146 werden analog Beispiel 6 umgesetzt.
- Ausbeute:: 12,3 g (80,1 % der Theorie).

### Beispiel 148

3-(tert.-Butyldimethylsilyloxymethyl)-2-cyclopropyl-4-(4-fluorphenyl)-6-isopropyl-5-methoxymethyl-pyridin 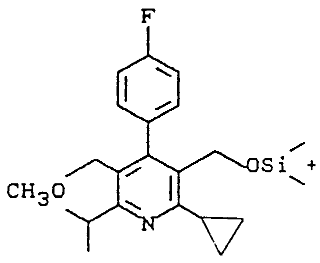 5,5 g (12,8 mmol) der Verbindung aus Beispiel 147 werden analog Beispiel 59 umgesetzt.
- Ausbeute:: 5,7 g Rohprodukt.

### Beispiel 149

2-Cyclopropyl-4-(4-fluorphenyl)-3-hydroxymethyl-6-isopropyl-5-methoxymethyl-pyridin 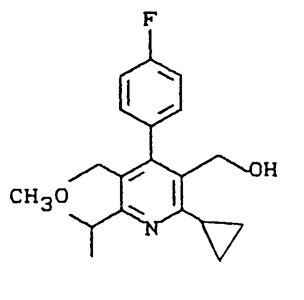 5,7 g (12,8 mmol) des Rohproduktes aus Beispiel 148 werden in absol. Tetrahydrofuran gelöst. Nach Zugabe von 12,8 ml Tetrabutylammoniumfluorid-Lösung (1 M in THF) wird über Nacht bei Raumtemperatur gerührt. Dann werden 50 ml gesättigte Natriumhydrogencarbonatlösung zugegeben und es wird mehrmals mit Methylenchlorid extrahiert. Die vereinigten organischen Phasen werden mit Natriumsulfat getrocknet, eingeengt und über Kieselgel chromatographiert (Laufmittel:Petrolether/Essigester 7:3).
- Ausbeute:: 3,9 g (94 % der Theorie).
- ¹H-NMR (CDCl₃): δ =: 0,0 (s, 6H); 0,87 (s, 9H); 0,8-1,0 (m, 2H); 1,22 (d, 6H); 1,10-1,30 (m, 2H) ; 2,31 (m, 1H) ; 3,12 (s, 3H) ; 3,30 (m, 1H); 3,98 (s, 2H); 4,47 (m, 2H); 7,0-7,3 (m, 4H) ppm.

### Beispiel 150

Methyl-erythro-(E)-7-[2-cyclopropyl-4-(4-fluorphenyl)-6-isopropyl-5-methoxymethyl-pyrid-3-yl]-3,5-dihydroxy-hept-6-enoat 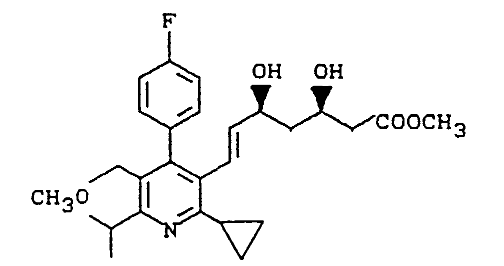 Aus der Verbindung aus Beispiel 149 wurde, in Analogie zu den Reaktionen aus den Beispielen 7, 8, 9, 10, das Beispiel 150 hergestellt.
- ¹H-NMR (CDCl₃): δ =: 0,89 (m, 2H); 1,18 (m, 2H); 1,26 (d, 6H); 1,40 (m, 2H); 2,24 (m, 1H); 2,44 (m, 2H); 3,17 (s, 3H); 3,30 (m, ¹H); 3,72 (s, 3H); 4,03 (s, 2H); 4,12 (m, 1H); 4,32 (m, 114); 5,51 (d,d, 114); 6,32 (d, 1H); 7,10 (m, 4H) ppm.

### Beispiel 151

3-Amino-3-cyclopropyl-acrylsäureethylester 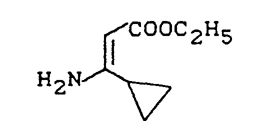 49,9 g (0,32 mol) Cyclopropylcarbonylessigsäureethylester in 200 ml trockenem Toluol werden mit 1,1 g p-Toluolsulfonsäure versetzt und bei Raumtemperatur unter Rühren mit Ammoniak-Gas gesättigt. Nach Stehenlassen über Nacht wird 8 h am Wasserabscheider unter Rückfluß gekocht, wobei kontinuierlich Ammoniak-Gas eingeleitet wird, Man läßt über Nacht abkühlen, filtriert, engt die Toluollösung im Vakuum ein und destilliert im Hochvakuum bis 65°C vom nicht umgesetzten Ausgangsmaterial ab. Die Substanz befindet sich anschließend im Rückstand. Ausbeute; 11,9 g (24 % der Theorie).

### Beispiel 152

1,4-Dihydro-2,6-dicyclopropyl-4-(4-fluorphenyl)-pyridin-3,5-dicarbonsäure-diethylester 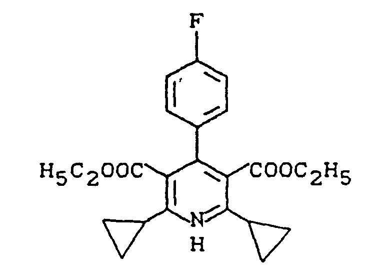 6,2 g (40 mmol) der Verbindung aus Beispiel 43 und 10,5 g (40 mmol) der Verbindung aus Beispiel 40 werden in 100 ml Ethylenglykol gelöst und über Nacht am Rück-fluß gekocht. Nach Abkühlen auf Raumtemperatur wird die Mischung mehrmals mit Ether extrahiert, die organische Phase je einmal mit 10 %iger Salzsäure, gesättigter Natriumbicarbonat-Lösung und Wasser gewaschen, über Magnesiumsulfat getrocknet und im Vakuum eingeengt.
- Ausbeute:: 10,4 g (65,1 % der Theorie).
- ¹H-NMR (CDCl₃): δ =: 0,60 (m, 4H); 0,95 (m, 4H); 1,23 (t, 6H); 2,72 (m, 2H); 4,12 (m, 4H); 5,02 (s, 1H); 5,40 (s, 1H); 6,88 (m, 2H); 7,20 (m, 2H) ppm.

### Beispiel 153

Methyl-erythro-(E)-7-(2,6-dicyclopropyl-4-(4-fluorphenyl)-5-methoxymethyl-pyrid-3-yl]-3,5-dihydroxy-hept-6-enoat 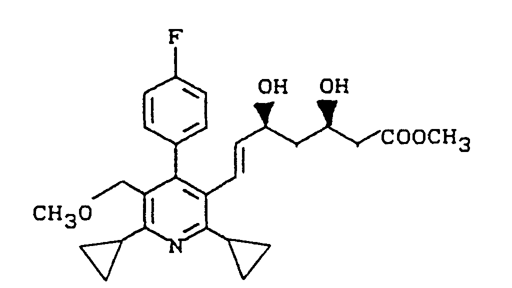 Aus der Verbindung aus Beispiel 150 wurde, in Analogie zu den Reaktionen aus den Beispielen 3, 4, 59, 60, 7, 8, 9, 10, das Beispiel 153 hergestellt.
- ¹H-NMR (CDCl₃): δ =: 0,89 (m, 4H); 1,08 (m, 4H); 1,40 (m, 2H); 2,21 (m, 2H); 2,43 (m, 2H); 3,21 (s, 3H); 3,72 (s, 3H); 4,11 (m, 1H); 4,15 (s, 2H); 4,30 (m, 1H); 5,47 (dd, 1H); 6,30 (d, 1H); 7,10 (m, 4H) ppm.

### Beispiel 154

2,6-Diisopropyl-5-ethoxymethyl-4-(4-fluorphenyl)-pyridin-3-carbonsäureethylester 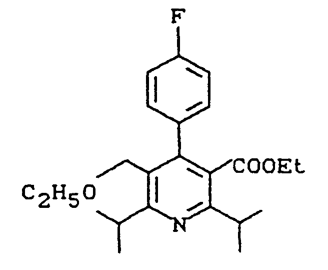 Zu einer Suspension von 2,29 g (76 mmol) Natriumhydrid (80 %ig) in 30 ml absol. Tetrahydrofuran tropft man bei Raumtemperatur 4,4 ml (76 mmol) absol. Ethanol und rührt 30 min. Dann tropft man eine Lösung von 2,7 g (7,6 mmol) der Verbindung aus Beispiel 86 in 20 ml absol. Tetrahydrofuran zu und erhitzt über Nacht unter Rückfluß. Anschließend wird der Reaktionsansatz auf Eiswasser gegossen und, nachdem der pH auf 8 eingestellt wurde, mehrmals mit Ether extrahiert. Die organische Phase wird mit Kochsalzlösung gewaschen, über Natriumsulfat getrocknet, eingeengt und über Kieselgel chromatographiert (Laufmittel Essigester/ Petrolether 5:95).
- Ausbeute:: 1,07 g (36,4 % der Theorie).
- ¹H-NMR (CDCl₃): δ =: 0,95 (t, 3H); 1,13 (t, 3H); 1,31 (m, 6H); 3,05 (m, 1H); 3,32 (q, 2H) ; 3,41 (m, 1H); 3,97 (q, 2H) ; 4,18 (d, 2H); 7,08 (m, 2H); 7,27 m, 2H) ppm.

### Beispiel 155

Methyl-erythro-(E)-7-[2,6-diisopropyl-5-ethoxymethyl-4-(4-fluorphenyl)-pyrid-3-yl]-3,5-dihydroxy-hept-6-enoat 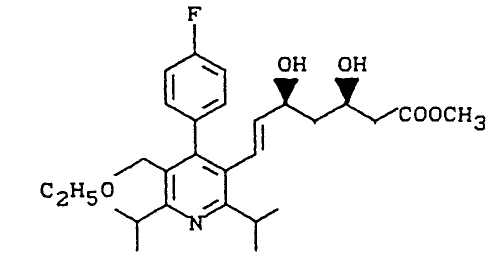 Aus der Verbindung aus Beispiel 154 wurde, in Analogie zu den Reaktionen aus den Beispielen 60, 7, 8, 9, 10, das Beispiel 155 erhalten.
- ¹H-NMR (CDCl₃): δ =: 1,13 (t, 3H); 1,20-1,50 (m, 8H); 2,43 (m, 2H); 3,30 (m, 3H); 3,72 (s, 3H); 4,08 (m, 1H); 4,12 (s, 2H); 4,29 (m, 1H); 5,25 (dd, 1H); 6,30 (d, 1H); 7,0-7,2 (m, 4H) ppm.
Analog Beispiel 155 wurden aus Beispiel 86 folgende Verbindungen erhalten:

### Beispiel 156

Methyl-erythro-(E)-(7-[2,6-diisopropyl-4-(4-fluorphenyl)-5-propyloxymethyl-pyrid-3-yl]-3,5-dihydroxy-hept-6-enoat.

### Beispiel 157

Methyl-erythro-(E)-(7-[2,6-diisopropyl-4-(4-fluorphenyl)-5-isopropoxymethyl-pyrid-3-yl]-3,5-dihydroxy-hept-6-enoat.

### Beispiel 158

Methyl-erythro-(E)-(7-[2,6-diisopropyl-4-(4-fluorphenyl)-5-butyloxymethyl-pyrid-3-yl]-3,5-dihydroxy-hept-6-enoat.

### Beispiel 159

Methyl-erythro-(E)-(7-[2,6-diisopropyl-4-(4-fluorphenyl)-5-pentyloxymethyl-pyrid-3-yl]-3,5-dihydroxy-hept-6-enoat.

### Beispiel 160

Methyl-erythro-(E)-(7-[2,6-diisopropyl-4-(4-fluorphenyl)-5-hexyloxymethyl-pyrid-3-yl]-3,5-dihydroxy-hept-6-enoat.

### Beispiel 161

Natrium-erythro-(E)-7-[2,6-diisopropyl-5-ethoxymethyl-4-(4-fluorphenyl)-pyrid-3-yl]-3,5-dihydroxy-hept-6-enoat 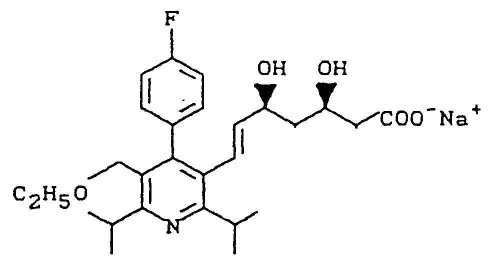 487 mg (1 mmol) der Verbindung aus Beispiel 155 werden in 10 ml Tetrahydrofuran gelöst und mit 10 ml 0,1 N Natronlauge versetzt. Nach 1 h wird das Tetrahydrofuran am Vakuum abgezogen und der wäßrige Rückstand wird gefriergetrocknet.
- Ausbeute:: 490 mg (99 % der Theorie).

### Beispiel 162

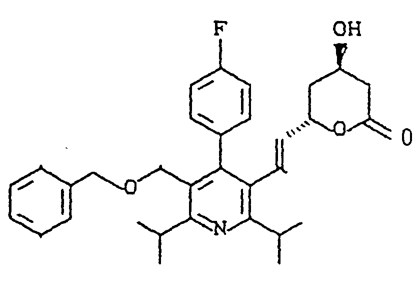

trans-(E)-6-[2-(3-benzyloxymethyl-2,6-diisopropyl-4-(4-fluorphenyl)-pyrid-5-yl)-ethenyl]-3,4,5,6-tetrahydro-4-hydroxy-2H-pyran-2-on

5,5 g (10 mmol) der Verbindung aus Beispiel 17 werden in 100 ml Tetrahydrofuran gelöst und nach Zugabe von 100 ml 0,1 N Natronlauge wird 1 h bei Raumtemperatur gerührt. Anschließend wird mit 100 ml Wasser verdünnt, mit 1 N HCl auf pH 4,4 gestellt und mit Methylenchlorid extrahiert. Die Methylenchloridphase wird mit Natriumsulfat getrocknet und im Vakuum eingeengt. Der Rückstand wird in 100 ml absol. Toluol gelöst, mit 40 g Molekularsieb 4 Å versetzt und über Nacht unter Rückfluß erhitzt. Anschließend wird vom Molekularsieb abfiltriert, im Vakuum eingeengt und der Rückstand wird mit Petrolether kristallisiert.
- Ausbeute:: 4,3 g (83,2 % der Theorie)
- ¹H-NMR (CDCl₃): δ =: 1,22 (d,6H); 1,32 (d,6H; 1,40-1,80 (m,2H); 2,45-2,70 (m,2H); 3,30 (m,2H); 4,12 (m,1H); 4,14 (s,2H); 4,45 (s,2H); 5,04 (m,1H); 5,28 (dd,1H); 6,39 (d,1H); 6,95-7,40 (m,9H)ppm.

### Beispiel 163

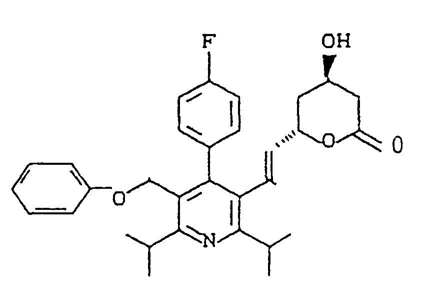

trans-(E)-6-[2-(2,6-diisopropyl-4-(4-fluorphenyl)-5-phenoxymethyl-pyrid-3-yl)-ethenyl]-3,4,5,6-tetrahydro-4-hydroxy-2H-pyran-2-one

Analog Beispiel 162 erhält man aus 540 mg (1 mmol) der Verbindung aus Beispiel 89 450 mg (89,4 % der Theorie).
- ¹H-NMR (CDCl₃): δ =: 1,25 (d,6H); 1,30 (d,6H); 1,40-1,70 (m,2H); 2,60 (m,2H); 3,30 (m,2H); 4,18 (m,1H); 4,65 (s,2H); 5,08 (m,1H): 5,30 (dd,1H); 6,42 (d,1H); 6,70-7,30 (m,9H)ppm.

### Beispiel 164

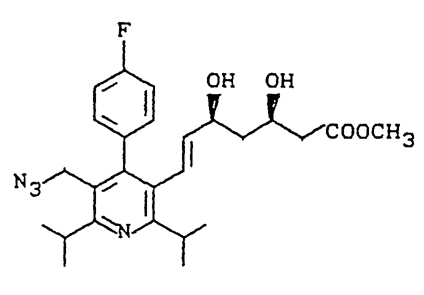

Methyl-erythro-(E)-7-[3-azidomethyl-2,6-diisopropyl-4-(4-fluorphenyl)-pyrid-5-yl)-3,5-dihydroxy-hept-6-enoat

459 mg (1 mmol) der Verbindung aus Beispiel 11 und 320 mg (1,1 mmol) Triphenylphosphin werden in 10 ml absol. Tetrahydrofuran gelöst. Nach Zugabe von 3,2 ml einer 0,48 molaren Lösung von HN₃ in Toluol wird mit einem Eisbad gekühlt und 173 ml (1,1 mmol) Azodicarbonsäurediethylester werden zugegeben. Anschließend wird über Nacht bei Raumtemperatur gerührt und dann im Vakuum eingeengt. Der Rückstand wird über Kieselgel chromatographiert (Laufmittel: Essigester/Petrolether 1:1).
- Ausbeute:: 260 mg (53,7 % der Theorie)
- ¹H-NMR (CDCl₃): δ =: 1,30 (m,6H); 1,39 (d,6H);1,25-1,60 (m,2H); 2,50 (m,2H); 3,37 (m,2H); 3,80 (s,3H); 4,15 (m,lH); 4,18 (s,2H); 4,36 (m,1H); 5,36 (dd,1H); 6,36 (d,1H); 7,15 (m,4H) ppm.

### Beispiel 165

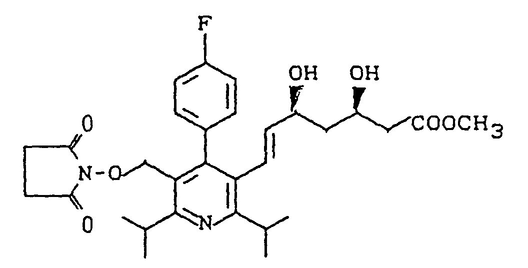

Methyl-erythro-(E)-7-[2,6-diisopropyl-4-(4-fluorphenyl)-5-succinimidooxymethyl-pyrid-3-yl)-3,5-dihydroxy-hept-6-enoat

Die Herstellung erfolgt analog Beispiel 164 aus der Verbindung aus Beispiel 11 und N-Hydroxysuccinimid.
- ¹H-NMR (CDCl₃): δ =: 1,28 (m,6H); 1,37 (d,6H);1,2-1,5 (m,2H); 2,43 (m,2H); 2,66 (s,4H); 3,32 (sept.,1H); 3,72 (s,3H); 3,78 (sept.,1H); 4,08 (m,1H); 4,31 (m,lH); 4,86 (s,2H); 5,28 (dd,lH); 6,33 (d,lH); 7,0-7,4 (m,4H) ppm.

### Beispiel 166

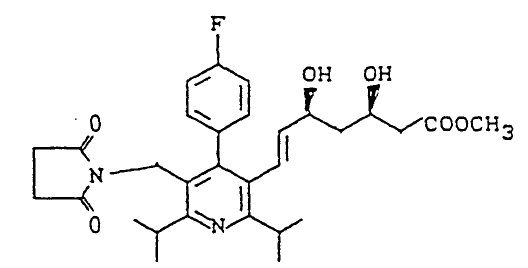

Methyl-erythro-(E)-7-[2,6-diisopropyl-4-(4-fluorphenyl)-5-succinimidomethyl-pyrid-3-yl)-3,5-dihydroxy-hept-6-enoat

Die Herstellung erfolgt analog Beispiel 164 aus der Verbindung aus Beispiel 11 und Succinimid.
- ¹H-NMR (CDCl₃): δ =: 1,23 (m,12H); 1,25-1,50 (m,2H); 2,43 (m,2H); 2,51 (s,4H); 3,16 (m,1H); 3,28 (m,1H); 3,73 (s,3H); 4,07 (m,lH); 4,26 (m,1H); 4,52 (s,2H); 5,25 (dd,1H); 6,20 (d,1H); 7,0-7,2 (m,4H) ppm.

### Beispiel 167

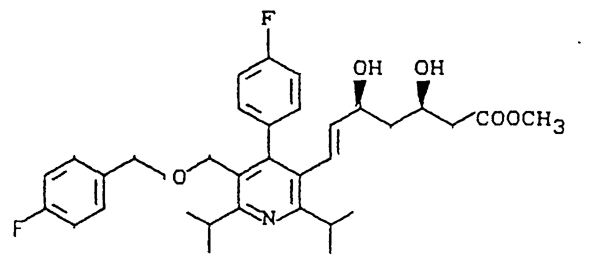

Methyl-erythro-(E)-7-[2,6-diisopropyl-3-(4-fluorbenzyloxymethyl)-4-(4-fluorphenyl)-pyrid-5-yl)-3,5-dihydroxy-hept-6-enoat

Aus der Verbindung des Beispiels 4 und 4-Fluorbenzylbromid wird analog der Beispiele 12-17 das Beispiel 166 erhalten.
- ¹H-NMR (CDCl₃): δ =: 1,25 (m,6H); 1,32 (d,6H); 1,2-1,5 (m,2H); 2,42 (m,2H); 3,30 (m,2H); 3,72 (s,3H); 4,07 (m,1H); 4,13 (s,2H); 4,28 (m,1H); 4,30 (s,2H); 5,22 (dd,lH); 6,30 (d,1H); 6,90-7,30 (m,8H) ppm.

### Beispiel 168

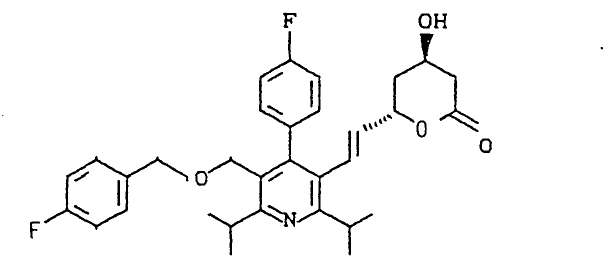

trans-(E)-6-[2-(2,6-diisopropyl-3-(4-fluorbenzyloxymethyl)-4-(4-fluorphenyl)-pyrid-5-yl-)-ethenyl]-3,4,5,6-tetrahydro-4-hydroxy-2H-pyran-2-on

Die Herstellung erfolgt analog Beispiel 164 aus der Verbindung von Beispiel 167.
- ¹H-NMR (CDCl₃): δ =: 1,23 (d,6H); 1,32 (d,6H); 1,40-1,80 (m,2H); 2,40 (m,2H); 3,30 (m,2H); 4,13 (s,2H); 4,16 (m,lH); 4,30 (s,2H); 5,05 (m,1H); 5,28 (dd,1H); 6,37 (d,1H); 6,9-7,3 (m,8H) ppm.

### Beispiel 169

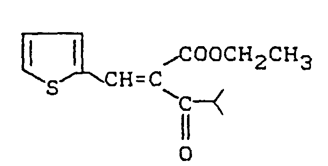

(E/Z)-2-Ethoxycarbonyl-4-methyl-1-(thiophen-2-yl)penten-3-on

Analog der Vorschrift für Beispiel 1 wird die Titelverbindung aus Isobutyrylessigsäurethylester und Thiophen-2-carbaldehyd hergestellt.
- Ausbeute:: 86 % gelbes Öl, Kp 145°C (1,5 mbar)

### Beispiel 170

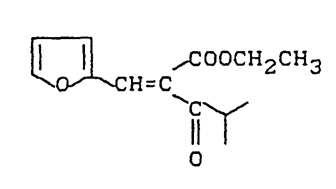

(E/Z)-2-Ethoxycarbonyl-1-(furan-2-yl)-4-methyl-penten-3-on

Analog der Vorschrift für Beispiel 1 wird die Titelverbindung aus Furan-2-carbaldehyd und Isobutyrylessigsäureethylester hergestellt.
- Ausbeute:: 93 % gelbes Öl, Kp.: 130°C (0,5 mbar)

### Beispiel 171

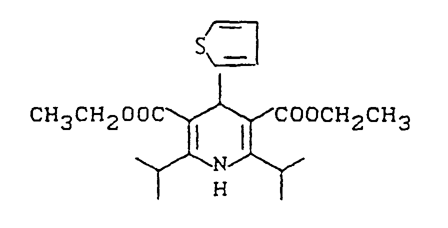

2,6-Diisopropyl-4-(thiopen-2-yl)-1,4-dihydropyridin-3,5-bis(carbonsäureethylester)

70 g (0,28 mol) der Verbindung aus Beispiel 169 und 44 g (0.28 mol) Ethyl-3-amino-4-methyl-pent-2-enoat wurden 24 h auf 160°C erhitzt. Man nimmt in Essigester auf, wäscht dreimal mit 6 N Salzsäure, zweimal mit Wasser und gesättiger Natriumhydrogencarbonatlösung und trocknet die org. Phase über Natriumsulfat. Man engt im Vakuum ein und chromatographiert den Rückstand an 400 g Kieselgel 230-400 mesh mit Petrolether/Dichlormethan 2:1.
- Ausbeute:: 50 g (46 %) farbl. Kristalle vom Schmp. 72°C (aus n-Hexan)

### Beispiel 172

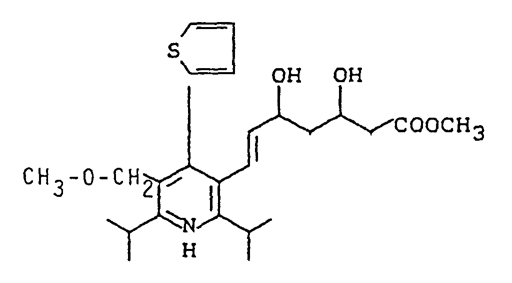

Methyl-erythro-(E)-7-[2,6-diisopropyl-5-methoxymethyl-4(thiophen-2-yl)-pyridin-3-yl]-3,5-dihydroxy-hept-6-enoat

Ausgehend von den Verbindungen aus Beispiel 171 wurde analog zu den Vorschriften für die Beispiele 3,4,59,6, 7,8,9 und 10 die Titelverbindung hergestellt.

Farblose Kristalle vom Schmp. 94°C

### Beispiel 173

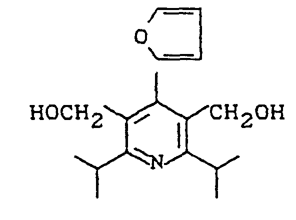

4-(Furan-2-yl)-3,5-bis(hydroxyethyl)-2,6-diisopropylpyridin

Ausgehend von der Verbindung aus Beispiel 170 und Ethyl-3-amino-4-methyl-pent-2-enoat wurde in Analogie zu den Verfahren aus Beispiel 171,3 und 122 die Titelverbindung hergestellt, Farbl. Kristalle vom Schmp. 212°C.

### Beispiel 174

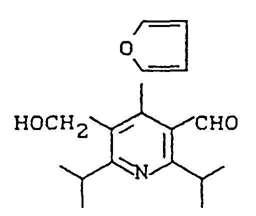

3-Formyl-4-(furan-2-yl)-5-hydroxymethyl-2,6-diisopropyl-pyridin

Die Synthese erfolgt aus 32 g (0.11 mol) der Verbindung aus Beispiel 173 und 28.6 g (0.13 mol) Pyridiumchlorochromat analog zur Vorschrift von Beispiel 7.
- Ausbeute:: 14,6 g (46 %)farbl. Kristalle vom Schmp. 113°C

### Beispiel 175

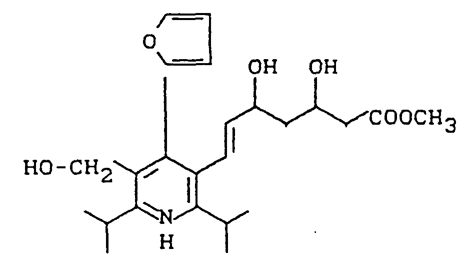

Methyl-erythro-(E)-7-[4-(furan-2-yl)-5-hydroxymethyl-2,6-diisopropyl-pyridin-3-yl]-3,5-dihydroxy-hept-6-enoat

Analog den Verfahren aus den Beispielen 123, 124 und 125 wurde ausgehend von der Verbindung aus Beispiel 174 die Titelverbindung hergestellt.

Farbl. Kristalle vom Schmp. 86°C

### Beispiel 176

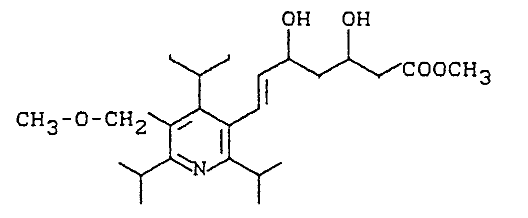

Methyl-erythro-(E)-7-[2,4,6-triisopropyl-5-methoxymethyl -pyridin-3-yl]-3,5-dihydroxy-hept-6-enoat

Ausgehend von den Verbindungen aus Beispiel 119 und Ethyl-3-amino-4-methyl-pent-2-enoat wurde in Analyse zu den Verfahren aus den Beispielen 171, 3, 4, 59, 6, 7, 8, 9 und 10 die Titelverbindung synthetisiert.
Farbl. Öl
- ¹H-NMR(CDCl₃): δ =: 1,1-1,35 (m,18H, isopropyl-H) 1,65-1,85 (m,2H, 4-H) 2,55 (d,2H,2-H), 3,2-3,45 (m,7H, isopropyl-H, OH, CH₃-O-CH₂) 3,7 (m,4H, OH, COOCH₃) 4,35 (m,1H:, HO-CH) 4,45(s,2H,CH₃-O-CH₂) 4,62 (m,1H, HO-CH) 5,55 (dd,1H,6-H) 6,73 (d, 1H, 7-H)

### Anwendungsbeispiel

### Beispiel 177

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat, 0,03 m Ethylendiamintetraessigsäure, 0,002 m Dithiothreit (SPE)-Puffer pH 7,2, homogenisiert. Anschließend wurde 15 Minuten bei 15 000^{*} g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol KₓH_{y}-Phosphat pH 7,2, 20 µl Enzympräparation und 56 nMol 3-Hydroxy-3-methyl-glutaryl Coenzym A (Glutaryl-3-14C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 100 gesetzt und mit dem simultan bestimmten IC₅₀-Wert der Testverbindung verglichen.

| Beispiel Nr. | relative Aktivität (Mevinolin = 1) |
|---|---|
| 17 | 20 |
| 70 | 30 |
| 72 | 17 |
| 85 | 20 |
| 89 | 10 |
| 99 | 20 |
| 100 | 30 |
| 105 | 4 |
| 107 | 3 |
| 139 | 20 |
| relative in vitro Aktivitäten, Mevinolin = 1 | |

### 2. Anwendungsbeispiel

### Beispiel 178

Die subchronische Wirkung der erfindungsgemäßen Verbindungen auf die Blutcholesterinwerte von Hunden wurde in mehrwöchigen Fütterungsexperimenten geprüft. Dazu wurde die zu untersuchende Substanz über einen mehrwöchigen Zeitraum einmal täglich in einer Kapsel gesunden Beagle-Hunden zusammen mit dem Futter p.o. gegeben. Dem Futter war außerdem während der gesamten Versuchsperiode, d.h. vor, während und nach der Applikationsperiode der zu untersuchenden Substanzen Colestyramin (4 g / 100 g Futter) als Gallensäuresequestrant beigemischt. Zweimal wöchentlich wurde den Hunden venöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

So ergab sich z.B. für die erfindungsgemäße Verbindung Beispiel Nr. 17 nach 2-wöchiger Applikation von täglich 8 mg/kg p.o. eine Senkung des Serumcholesterins um 66%.

## Patentansprüche

1. Verfahren zur Herstellung von substituierten Pyridinen der Formel 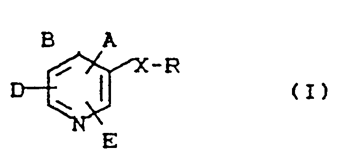 in welcher
A
- für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein kann,
worin
R¹, R²
- gleich oder verschieden sind und Alkyl, Aryl, Aralkyl, Acyl, Alkylsulfonyl oder Arylsulfonyl bedeuten,
oder
- für Aryl steht, das bis zu 5-fach gleich oder verschieden substituiert sein kann durch Alkyl, das gegebenenfalls durch Hydroxy oder Alkoxy substituiert sein kann, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
worin
R¹ und R² die oben angegebene Bedeutung haben,
- für geradkettiges oder verzweigtes Alkyl steht,
B
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
worin
R¹, R²
- gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
D,E gleich oder verschieden sind und
- für Wasserstoff stehen, oder
- für CN oder NO₂ stehen, oder
- für Cycloalkyl stehen, oder
- für geradkettiges oder verzweigtes Alkyl stehen, das substituiert sein kann durch Azido, Halogen, Hydroxy, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
worin
R¹, R² die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
oder
- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein können,
worin
R¹ und R² die oben angegebene Bedeutung haben,
oder
- für Aryl stehen, das bis zu 5-fach gleich oder verschieden substituiert sein kann durch Alkyl, das gegebenenfalls durch Hydroxy oder Alkoxy substituiert sein kann, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
worin
R¹ und R² die oben angegebene Bedeutung haben,
oder
- für eine Gruppe der Formel -NR3R4, -COR⁵ oder -CR¹¹R¹²-Y stehen,
worin
R³ und R⁴ gleich oder verschieden sind und
- Wasserstoff oder
- Alkyl, Aryl, Aralkyl, oder
- eine Gruppe der Formel -COR⁶ oder -SO₂R⁷ bedeuten, oder
R³ und R⁴ gemeinsam eine Alkylidenkette bilden, die durch N, O, S und/oder N-Alkyl, N-Aryl, N-Aryl, N-Carbamoyl oder N-Alkoxycarbonyl unterbrochen sein kann,
R⁶
- für Wasserstoff steht, oder
- für eine Gruppe -NHR⁸ steht, oder
- für Alkoxy steht, oder
- für Alkyl, Aryl, Aryloxy, Aralkyl, Aralkoxy oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein' können,
R⁷
- für Cycloalkyl steht, oder
- für Alkyl steht, das durch Cyano, Halogen, Trifluormethyl, Trifluormethoxy oder Alkoxycarbonyl substituiert sein kann, oder
- für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
und
R⁸
- für Wasserstoff steht, oder
- für Cycloalkyl steht, oder
- für gegebenenfalls durch Cyano, Halogen, Trifluormethyl oder Trifluormethoxy substituiertes Alkyl steht, oder
- für Aryl, Aralkyl oder Heteroaryl steht, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
R⁵
- Wasserstoff, Cycloalkyl, Hydroxy, Alkoxy, Trimethylsilylalkoxy, Aryloxy, Aralkoxy bedeutet, oder
- für eine Gruppe der Formel -NR⁹R¹⁰ steht,
worin
R⁹ und R¹⁰ gleich oder verschieden sind und Wasserrstoff, Alkyl, Aryl oder Aralkyl bedeuten,
oder
- ein über ein Stickstoffatom gebundenen, gegebenenfalls substituierten heterocyclischen Rest der Reihe Pyrrolidin, Piperidin, Morpholin, Thiomorpholin oder Piperazin bedeutet,
und
R¹¹ und R¹² gleich oder verschieden sein können und
- für Wasserstoff, oder
- für Alkyl stehen, das gegebenenfalls durch Hydroxy, Halogen, Alkoxy oder Alkoxycarbonyl substituiert sein kann, oder
- für Cycloalkyl stehen, oder
R¹¹ und R¹² gemeinsam einen gesättigten oder ungesättigten carbocyclischen oder heterocyclischen Ring mit bis zu 6 Kohlenstoffatomen bilden,
und
Y
- eine Gruppe der Formel -NR¹³R¹⁴, -COR¹⁵, - S-R¹⁶, -SO-R¹⁶, -SO₂-R¹⁶, -OR¹⁷ oder -N₃ bedeutet,
worin
R¹³ und R¹⁴ gleich oder verschieden sind und
- für Wasserstoff, Alkyl, Aryl oder Aralkyl stehen, wobei die Arylreste durch Halogen, Cyano, Alkyl, Alkoxy oder Trifluormethyl substituiert sein können, oder
- für eine Gruppe der Formel -COR¹⁵, -SO₂R¹⁶ stehen,
oder R¹³ und R¹⁴ gemeinsam eine Alkylenkette bilden, die durch N, O, S und/oder N-Alkyl, N-Aryl, N-Aralkyl, N-Carbamoyl oder N-Alkoxycarbonyl unterbrochen sein kann,
R¹⁵
- Wasserstoff bedeutet, oder
- eine Gruppe -NR¹⁸R¹⁹ bedeutet, oder
- Alkyl oder Alkoxy bedeutet, oder
- Aryl, Aryloxy, Aralkyl, Aralkoxy oder Heteroaryl bedeutet, wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können,
R¹⁶
- Cycloalkyl bedeutet, oder
- geradkettiges oder verzweigtes Alkyl bedeutet, das durch Cyano, Halogen, Trifluormethyl, Trifluormethoxy oder Alkoxycarbonyl substituiert sein kann, oder
- Aryl, Aralkyl oder Heteroaryl bedeutet,
wobei die genannten Reste bis zu 3-fach gleich oder verschieden durch Alkyl, Alkoxy, Alkylthio, Halogen, Cyano, Trifluormethyl, Trifluormethoxy, Trifluormethylthio, Amino, Alkylamino oder Dialkylamino substituiert sein können, oder
- Trimethylsilyl oder Dimethylethylsilyl bedeutet, oder
- eine Gruppe -NR⁹R¹⁰ bedeutet,
wobei
R⁹ und R¹⁰ die oben angegebene Bedeutung haben,
R¹⁷
- für Wasserstoff steht, oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -NR¹R²,
worin
R¹, R² die oben angegebehe Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
oder
- für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -NR¹R² substituiert sein kann,
worin
R¹ und R² die oben angegebene Bedeutung haben,
oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -NR¹R²,
worin
R¹ und R² die oben angegebene Bedeutung haben,
oder
- für 2,5-Dioxo-tetrahydropyrryl,
- für Tetrahydropyranyl, oder
- für Trialkylsilyl steht, oder eine Gruppe COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- Cycloalkyl bedeuten, oder
- gegebenenfalls durch Cyano, Halogen, Tri fluormethyl oder Trifluormethoxy substituiertes Alkyl bedeuten, oder
- Aryl, Aralkyl oder Heteroaryl bedeuten, wobei die genannten Reste bis zu 3-fach gleich oder verschieden durch Alkyl, Alkoxy, Alkylthio, Halogen, Cyano, Trifluormethyl, Trifluormethoxy, .Trifluormethylthio, Amino, Alkylamino oder Dialkylamino substituiert sein können,
oder,
D und E gemeinsam für den Rest der Formel 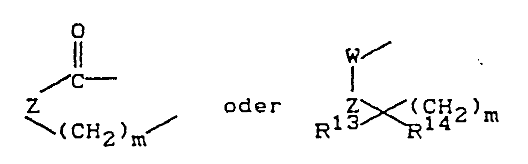 stehen, und einen Ring bilden, wobei
W - für eine Gruppe der Formel C=O oder -CHOH steht,
m - für eine Zahl 1, 2 oder 3 steht,
Z - für O, S, CH₂ oder für N-R²⁰ steht,
R¹³ und R¹⁴ die oben angegebene Bedeutung haben,
und
R²⁰ - für Wasseretoff, Alkyl, Aryl, Aralkyl, Carbamoyl oder Alkoxycarbonyl steht,
und wobei in diesem Fall D und E benachbart sind,
X - für eine Gruppe der Formel -CH₂-CH₂- oder-CH=CH-steht,
und
R - für eine Gruppe der Formel 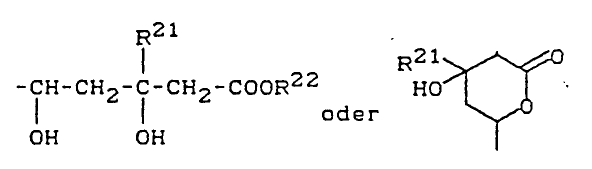 steht, worin
R²¹ - Wasserstoff oder Alkyl bedeutet
und
R²²
- Wasserstoff,
- Alkyl, Aryl oder Aralkyl, oder
- ein Kation bedeutet,
sowie deren Oxidationsprodukte,
dadurch gekennzeichnet, daß man Verbindungen der Formel (XXII) 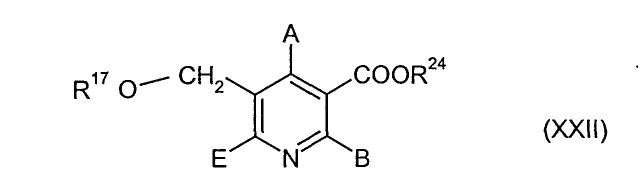
in denen R¹⁷ für Wasserstoff oder Methyl, Ethyl, Propyl, Isopropyl, Butyl, Isobutyl, tert. Butyl, Pentyl, Isopentyl, Hexyl oder Isohexyl steht und
R²⁴ für einen Alkylrest mit bis zu 4 Kohlenstoffatomen steht,
im ersten Schritt [1] in inerten Lösemitteln mit Metallhydriden als Reduktionsmittel in Temperaturbereichen von -70°C bis +100°C zu den Hydroxymethylverbindungen reduziert und diese im zweiten Schritt [2] nach üblichen Methoden zu den Aldehyden oxidiert, und die Aldehyde (XII) im dritten Schritt [3] mit Diethyl-2-(cyclohexylamino)-vinylphosphonat in Anwesenheit von Natriumhydrid in inerten Lösemitteln in einem Temperaturbereich von -20°C bis + 40°C zu den Aldehyden
der Formel 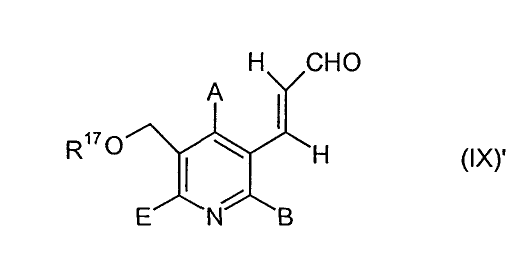 umsetzt, und diese in inerten Lösemitteln mit Acetessigester der allgemeinen Formel (X) 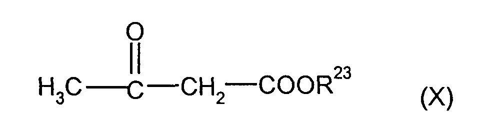 in welcher
R²³ für Alkyl steht,
in Anwesenheit von Basen umsetzt,
die resultierenden Ketone reduziert, und
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen
(X = -CH₂-CH₂-) die Ethenverbindungen (X = -CH=CH-) nach üblichen Methoden hydriert,
um gegebenenfalls Isomeren trennt.

2. Die nach dem Verfahren gemäß Anspruch 1 erhältlichen Verbindungen der Formel (I).
